# EUROPEAN PATENT SPECIFICATION

(11) **EP 3 221 461 B1**
(45) Date of publication and mention of the grant of the patent: **01.01.2025**
(21) Application number: 15861129.3
(22) Date of filing: 16.11.2015
(51) Int. Cl.: C12N 9/10, A61P 11/14, C07D 515/02, C07D 515/14, C12P 17/18, C07D 273/01, C07D 515/22

(54) **NOSCAPINOID-PRODUCING MICROBES AND METHODS OF MAKING AND USING THE SAME**
NOSCAPINOID-PRODUZIERENDE MIKROBEN SOWIE VERFAHREN ZUR HERSTELLUNG UND VERWENDUNG DAVON
MICROBES PRODUISANT DES NOSCAPINOÏDES ET LEURS MÉTHODES DE PRODUCTION ET D'UTILISATION

(30) Priority: 17.11.2014 US 201462080610 P; 23.01.2015 US 201562107238 P; 04.05.2015 US 201562156701 P; 08.05.2015 US 201562159122 P; 11.06.2015 US 201562174475 P
(43) Date of publication of application: 27.09.2017
(73) Proprietor: The Board of Trustees of the Leland Stanford Junior University, Stanford, CA 94305-2038 (US)
(72) Inventor: SMOLKE, Christina D., Menlo Park, California 94025 (US); LI, Yanran, Riverside, CA 92521 (US)
(74) Representative: Carpmaels & Ransford LLP
(86) International application number: PCT/US2015/060891
(87) International publication number: WO 2016/081371

(56) References cited:
- WO-A1-2015/021561
- WO-A1-2016/179296
- WO-A2-2013/136057
- WO-A2-2013/136057
- WO-A2-2014/143744
- US-A1- 2007 199 090
- US-A1- 2008 176 754
- US-A1- 2013 340 119
- T.-T. T. DANG ET AL: "CYP82Y1 Is N-Methylcanadine 1-Hydroxylase, a Key Noscapine Biosynthetic Enzyme in Opium Poppy", JOURNAL OF BIOLOGICAL CHEMISTRY, vol. 289, no. 4, 9 December 2013 (2013-12-09), pages 2013 - 2026, XP055319194, ISSN: 0021-9258, DOI: 10.1074/jbc.M113.505099
- ELENA FOSSATI ET AL: "Reconstitution of a 10-gene pathway for synthesis of the plant alkaloid dihydrosanguinarine in Saccharomyces cerevisiae", NATURE COMMUNICATIONS, vol. 5, 11 February 2014 (2014-02-11), XP055165144, DOI: 10.1038/ncomms4283
- KRISTY M HAWKINS ET AL: "Production of benzylisoquinoline alkaloids in Saccharomyces cerevisiae", NATURE CHEMICAL BIOLOGY, vol. 4, no. 9, 10 August 2008 (2008-08-10), pages 564 - 573, XP055138483, ISSN: 1552-4450, DOI: 10.1038/nchembio.105
- YANRAN LI ET AL: "Engineering biosynthesis of the anticancer alkaloid noscapine in yeast", NATURE COMMUNICATIONS, vol. 7, 5 July 2016 (2016-07-05), GB, pages 12137 - 1, XP055482763, ISSN: 2041-1723, DOI: 10.1038/ncomms12137
- ANDREAS GESELL ET AL: "Heterologous expression of two FAD-dependent oxidases with ()-tetrahydroprotoberberine oxidase activity fromandin insect cells", PLANTA ; AN INTERNATIONAL JOURNAL OF PLANT BIOLOGY, SPRINGER, BERLIN, DE, vol. 233, no. 6, 15 February 2011 (2011-02-15), pages 1185 - 1197, XP019908613, ISSN: 1432-2048, DOI: 10.1007/S00425-011-1357-4
- T. WINZER ET AL: "A Papaver somniferum 10-Gene Cluster for Synthesis of the Anticancer Alkaloid Noscapine", SCIENCE, vol. 336, no. 6089, 31 May 2012 (2012-05-31), pages 1704 - 1708, XP055032027, ISSN: 0036-8075, DOI: 10.1126/science.1220757

## Description

### SUMMARY

The present disclosure relates to methods for the production of diverse benzylisoquinoline alkaloids (BIAs), such as noscapinoids, in engineered non-plant host cells.

The invention provides a method for forming a product stream having a benzylisoquinoline alkaloid product that is downstream of canadine, the method comprising: culturing an engineered yeast cell (e.g. an engineered non-plant cell) that produces a benzylisoquinoline alkaloid product that is a derivative of canadine along a metabolic pathway that converts canadine to a noscapinoid product, wherein the benzylisoquinoline alkaloid product is selected from the group consisting of noscapine and narcotinehemiacetal, and wherein the engineered yeast cell comprises at least one heterologous sequence encoding a heterodimeric enzyme of *Papaver somniferum* O-methyltransferase MT2 and *Papaver somniferum* O-methyltransferase MT3, or a heterodimeric enzyme of *Papaver somniferum* O-methyltransferase MT2 and *Papaver somniferum* norcoclaurine 6-O-methyltransferase 6OMT; and separating the benzylisoquinoline alkaloid product from cellular material to provide a product stream having the benzylisoquinoline alkaloid product, and wherein the cell increases production of noscapine or narcotinehemiacetal relative to a control cell.

In examples, the engineered host cells may utilize canadine as a starting compound, which may be added to the engineered host cell. In other examples, the engineered host cells may utilize canadine that is produced by the cell from a precursor such as norlaudanosoline or tyrosine. In additional examples, the engineered host cells may produce noscapinoids, precursors of noscapinoids, metabolites of noscapinoids, analogs of noscapinoids, intermediates of noscapinoids, and/or derivatives of noscapinoids de novo from a yeast cell that has been engineered to produce noscapinoids from tyrosine, where tyrosine is produced naturally in the yeast cell. In further examples, the engineered host cells may produce noscapinoids, precursors of noscapinoids, metabolites of noscapinoids, analogs of noscapinoids, intermediates of noscapinoids, functionalized derivatives of noscapinoids, and/or derivatives of noscapinoids that are formed using analogs of precursor compounds, such as tyrosine.

The engineered non-plant cell comprises one or more heterologous coding sequences encoding at least one enzyme involved in the metabolic pathway that converts canadine, or an analog of canadine, to a noscapinoid product. In some embodiments, the engineered non-plant cell comprises heterologous coding sequences encoding more than one enzyme involved in the metabolic pathway that converts canadine to a noscapinoid product. Additionally, in some embodiments, the engineered non-plant cell comprises heterologous coding sequences encoding two distinct enzymes involved in the metabolic pathway that converts canadine to a noscapinoid product. In some embodiments, the engineered non-plant cell comprises heterologous coding sequences encoding more than three distinct enzymes involved in the metabolic pathway that converts canadine to a noscapinoid product. In some embodiments, the compound is produced within the engineered non-plant cell.

The benzylisoquinoline alkaloid product is a phthalideisoquinoline alkaloid. In some embodiments, the phthalideisoquinoline alkaloid product is narcotinehemiacetal and, in further embodiments, the at least one enzyme involved in the metabolic pathway comprises MT2 and MT3, and wherein the MT2 and MT3 convert narcotolinehemiacetal to narcotinehemiacetal. In some embodiments, the at least one enzyme involved in the metabolic pathway comprises MT2 and 6OMT, and wherein the MT2 and 6OMT convert narcotolinehemiacetal to narcotinehemiacetal.

In some embodiments, the phthalideisoquinoline alkaloid product is noscapine, and in further embodiments, the at least one enzyme involved in the metabolic pathway comprises MT2 and MT3, and wherein the MT2 and MT3 convert narcotoline to noscapine. In some embodiments, the at least one enzyme involved in the metabolic pathway comprises MT2 and 6OMT, and wherein the MT2 and 6OMT convert narcotoline to noscapine.

The engineered non-plant cell is a yeast cell. In some embodiments, the engineered non-plant cell is a reticuline-producing cell. In some embodiments, the reticuline-producing cell comprises coding sequences for producing PTPS, SepR, PCD, QDHPR, DHFR, TyrH, NCS, DODC, CYP80B1, CPR, 6OMT, 4'OMT, CNMT, ARO4, ARO7, ARO10, and TKL1, wherein each coding sequence is chromosomally integrated into the reticuline-producing cell. In some embodiments, at least one extra copy of a coding sequence that produces at least one of TyrH, 4'OMT, and NCS is chromosomally integrated into the reticuline-producing cell, thereby increasing production of reticuline within the reticuline-producing cell. In some embodiments, the reticuline-producing cell comprises at least one heterologous coding sequence for producing at least one of BBE, S90MT, MT1, CAS, TNMT, CYP82Y1, CYP82X2, AT1, CYP82X1, CXE1, SDR1, and MT3, wherein each coding sequence is chromosomally integrated into the reticuline-producing cell. In some embodiments, at least one extra copy of a coding sequence that produces at least one of TyrH, 4'OMT, and NCS is chromosomally integrated into the reticuline-producing cell, thereby increasing production of reticuline within the reticuline-producing cell. In some embodiments, at least one extra copy of a coding sequence that produces at least one of CYP82X2 and MT1 is chromosomally integrated into the reticuline-producing cell, thereby increasing production of noscapine within the reticuline-producing cell.

In some embodiments, the engineered non-plant cell comprises at least one heterologous sequence encoding at least one mutant enzyme. In some embodiments, the at least one mutant enzyme is selected from the group consisting of a CYP82Y1 N-terminus mutant, a CYP82X2 mutant, a CYP82X1 mutant. In some embodiments, the engineered non-plant cell comprises at least one heterologous sequence encoding at least one promoter. In some embodiments, the at least one promoter is selected from the group consisting of HXT7, ADH1, PGK1, TPI1, PYK1, TEF1, GAL1, CYC1, PUT1, CIT2, and GPD.

In some embodiments, the engineered non-plant cell comprises one or more plant chaperones selected from the group consisting of binding immunoglobulin protein (BiP), DnaJ protein, glucose regulated protein (GRP) 94, binding protein (BiP), protein disulphide isomerase (PDI), cyclophilin, and calnexin. In some embodiments, one or more of the enzymes is spatially localized to a compartment in the engineered non-plant cell, wherein the compartment is selected from the group consisting of mitochondrion, endoplasmic reticulum (ER), golgi, vacuole, nucleus, plasma membrane, peroxisome, and periplasm. In some embodiments, the one or more enzymes are spatially localized to the outside of the compartment in the engineered non-plant cell. In some embodiments, the one or more enzymes are spatially localized to the inside of the compartment in the engineered non-plant cell. In some embodiments, the engineered non-plant cell comprises at least one heterologous coding sequence for encoding at least one tailoring enzyme. In some embodiments, the at least one tailoring enzyme is selected from a group consisting of halogenase, prenaltransferase, glycosylase, methylase, demethylase, and oxidoreductase.

The invention provides a method for forming a product stream having a benzylisoquinoline alkaloid product that is downstream of canadine.

In some embodiments, the amount of the at least one enzyme in the engineered non-plant cell is more than the amount of the at least one enzyme in the non-engineered non-plant cell. The benzylisoquinoline alkaloid product is a phthalideisoquinoline alkaloid selected from the group consisting of narcotinehemiacetal , and noscapine. In some embodiments, the phthalideisoquinoline alkaloid product is narcotinehemiacetal, and wherein the engineered non-plant cell comprises at least one of MT2, MT3, 6OMT, and CXE1. In some embodiments, the phthalideisoquinoline alkaloid product is noscapine, and wherein the engineered non-plant cell comprises at least one of MT3, 6OMT, CXE1, and SDR1.

In some embodiments, the engineered non-plant cell is a reticuline-producing cell. In some embodiments, the reticuline-producing cell comprises coding sequences for producing PTPS, SepR, PCD, QDHPR, DHFR, TyrH, NCS, DODC, CYP80B1, CPR, 6OMT, 4'OMT, CNMT, ARO4, ARO7, ARO10, and TKL1, wherein each coding sequence is chromosomally integrated into the reticuline-producing cell. In some embodiments, at least one extra copy of a coding sequence that produces at least one of TyrH, 4'OMT, and NCS is chromosomally integrated into the reticuline-producing cell, thereby increasing production of reticuline within the reticuline-producing cell. In some embodiments, the reticuline-producing cell comprises at least one heterologous coding sequence for producing at least one of BBE, S90MT, MT1, CAS, TNMT, CYP82Y1, CYP82X2, AT, CYP82X1, CXE1, SDR1, and MT3, wherein each coding sequence is chromosomally integrated into the reticuline-producing cell. In some embodiments, at least one extra copy of a coding sequence that produces at least one of TyrH, 4'OMT, and NCS is chromosomally integrated into the reticuline-producing cell, thereby increasing production of reticuline within the reticuline-producing cell.

In some embodiments, the engineered non-plant cell comprises at least one heterologous sequence encoding at least one mutant enzyme. In some embodiments, the at least one mutant enzyme is selected from the group consisting of a CYP82Y1 N-terminus mutant, a CYP82X2 mutant, a CYP82X1 mutant. In some embodiments, the engineered non-plant cell comprises at least one heterologous sequence encoding at least one promoter. In some embodiments, the at least one promoter is selected from the group consisting of HXT7, ADH1, PGK1, TPI1, PYK1, TEF1, GAL1, CYC1, PUT1, CIT2, and GPD.

In some embodiments, the engineered non-plant cell comprises one or more plant chaperones selected from the group consisting of binding immunoglobulin protein (BiP), DnaJ protein, glucose regulated protein (GRP) 94, binding protein (BiP), protein disulphide isomerase (PDI), cyclophilin, and calnexin. In some embodiments, one or more of the enzymes is spatially localized to a compartment in the engineered non-plant cell, wherein the compartment is selected from the group consisting of mitochondrion, endoplasmic reticulum (ER), golgi, vacuole, nucleus, plasma membrane, peroxisome, and periplasm. In some embodiments, the one or more enzymes are spatially localized to the outside of the compartment in the engineered non-plant cell. In some embodiments, the one or more enzymes are spatially localized to the inside of the compartment in the engineered non-plant cell.

In some embodiments, the engineered non-plant cell comprises at least one heterologous coding sequence for encoding at least one tailoring enzyme. In some embodiments, the at least one tailoring enzyme is selected from a group consisting of halogenase, prenaltransferase, glycosylase, methylase, demethylase, and oxidoreductase. The engineered non-plant cell is a yeast cell. In some embodiments, the engineered non-plant cell is cultured under *in vitro* conditions. In some embodiments, the engineered non-plant cell is cultured under *in vivo* conditions.

In some embodiments, the product stream does not contain more than 5 ppm of a molecule selected from the group of lignin, flavonoids, phenanthreoids, latex, rubisco, meconic acid, pseudomorphine, narceine, thebaol, and pollen. In some embodiments, the product stream does not contain a detectable amount of pesticides. In some embodiments, the product stream contains at least one portion of a non-plant cell. In some embodiments, the non-plant cell is an engineered non-plant cell. In some embodiments, the at least one portion of the non-plant cell is present in the product stream in a detectable amount. In some embodiments, the at least one portion of the non-plant cell is detectable using liquid chromatography-mass spectrometry. In some embodiments, the at least one portion of the non-plant cell is detectable using mass spectrometry. In some embodiments, the at least one portion of the non-plant cell is detectable using spectroscopy. In some embodiments, the benzylisoquinoline alkaloid product is recovered from said product stream using at least liquid-liquid extraction. In some embodiments, the benzylisoquinoline alkaloid product is recovered immediately after a fermentation process has been completed.

The engineered host cells may include heterologous coding sequences for a variety of enzymes involved in synthetic pathways from starting compounds to noscapinoids of interest or precursors thereof. Also provided are methods of producing noscapinoids, derivatives thereof, and/or precursors thereof by culturing the engineered host cells under culture conditions that promote activity of enzymes encoded by the heterologous coding sequences of the host cells. Also disclosed are compositions, e.g., host cells, starting compounds and kits, etc., that find use in methods of the invention.

WO 2016/179296 discloses benzylisoquinoline alkaloid (BIA) precursor producing microbes, and methods of making and using the same. WO 2015/021561 discloses compositions and methods for making noscapine and synthesis intermediates thereof.

WO 2014/143744 discloses benzylisoquinoline alkaloids (BIA) producing microbes, and methods of making and using the same. Gesell et al., Planta, 2011, 233(6):1185-1197 teaches heterologous expression of two FAD-dependent oxidases with (S)-tetrahydroprotoberberine oxidase activity from *Argemone mexicana* and *Berberis wilsoniae* in insect cells. Winzer et al., Science, 2012, 29;336(6089):1704-8 discloses a *Papaver somniferum* 10-gene cluster for synthesis of the anticancer alkaloid noscapine. WO 2013/136057 discloses genes involved in noscapine production. Li et al., Nature Communications, 2016, 7:12137 teaches engineering the biosynthesis of the anticancer alkaloid noscapine in yeast.

### BRIEF DESCRIPTION OF THE DRAWINGS

The novel features of the invention are set forth with particularity in the appended claims. A better understanding of the features and advantages of the invention will be obtained by reference to the following detailed description that sets forth illustrative embodiments, in which the principles of the invention are utilized, and the accompanying drawings of which:
**FIG. 1** illustrates a biosynthetic scheme for conversion of canadine to noscapine based on biochemical identification in plants.
**FIG. 2A** illustrates a biosynthetic scheme for conversion of canadine to noscapine based on biochemical characterization in *Saccharomyces cerevisiae,* in accordance with embodiments of the invention.
**FIG. 2B** illustrates another biosynthetic scheme for conversion of canadine to noscapine and production of byproducts based on biochemical characterization in *Saccharomyces cerevisiae,* in accordance with embodiments of the invention.
**FIG. 3** illustrates EIC (extract ion chromatograph) traces from liquid chromatography-mass spectrometry (LC-MS) analysis of compounds secreted into the culture medium by engineered yeast strains, in accordance with embodiments of the invention.
**FIG. 4** illustrates positive ion electrospray ionization (ESI) mass spectra MS/MS fragmentation for compounds described in **FIG. 3**, in accordance with embodiments of the invention.
**FIG. 5** illustrates synthesis of N-methylcanadine in vivo from yeast expressing TNMT from either *Papaver somniferum* or *Eschscholzia californica* and grown in the presence of canadine, in accordance with embodiments of the invention.
**FIG. 6** illustrates results of assays in vivo from yeast grown in the presence of canadine with expression of different versions of CYP82Y1 downstream of various promoters, with or without the presence of PsTNMT, in accordance with embodiments of the invention.
**FIG. 7** illustrates results of assays in vivo from yeast grown in the presence of canadine with expression of CYP82X2 downstream of various promoters (GAL1, GPD, PGK1, HXT7), in the presence of PsTNMT and CYP82Y1A, in accordance with embodiments of the invention.
**FIG. 8** illustrates results of assays in vivo from yeast grown in the presence of canadine with expression of wild type (WT) downstream of various promoters (GAL1, GPD, PGK1, CYC1, ADH1, HXT7), in the presence of PsTNMT and CYP82Y1A, with and without CYP82X2 and PsAT1, in accordance with embodiments of the invention.
**FIG. 9** illustrates a biosynthetic scheme for conversion of norlaudanosoline to noscapine, in accordance with embodiments of the invention.
**FIG. 10** illustrates EIC (extract ion chromatograph) traces from LC-MS analysis of compounds secreted into the culture medium by an engineered noscapine producing yeast strain, in accordance with embodiments of the invention.
**FIG. 11** illustrates biosynthesis of noscapine from norlaudanosoline, in accordance with embodiments of the invention.
**FIG. 12** illustrates a biosynethic pathway of noscapine from tyrosine, in accordance with embodiments of the invention.
**FIG. 13** illustrates EIC (extract ion chromatograph) traces from LC-MS analysis of de novo production of noscapine in yeast, in accordance with embodiments of the invention.
**FIG. 14** illustrates an exemplary biosynthetic schematic of de novo production of noscapinoids in yeast, in accordance with embodiments of the invention.
**FIG. 15** illustrates another exemplary biosynthetic schematic of de novo production of noscapinoids in yeast, in accordance with embodiments of the invention.
**FIG. 16** illustrates a biosynthetic scheme for conversion of glucose to 4-HPA, dopamine, and 3,4-DHPA, in accordance with embodiments of the invention.
**FIG. 17** illustrates a biosynthetic scheme for conversion of L-tyrosine to reticuline via norcoclaurine, in accordance with embodiments of the invention.
**FIG. 18** illustrates a biosynthetic scheme for conversion of L-tyrosine to reticuline via norlaudanosoline, in accordance with embodiments of the invention.
**FIG. 19** illustrates examples of synthesis, recycling, and salvage pathways of tetrahydrobiopterin, in accordance with embodiments of the invention.
**FIG. 20** illustrates a biosynthetic scheme for conversion of L-tyrosine to protoberberine, phthalideisoquinoline, and berberine alkaloid products, in accordance with embodiments of the invention.
**FIG. 21** illustrates a biosynthetic scheme for conversion of L-tyrosine to noscapine, noscapinoid, and phthalideisoquinoline alkaloid products, in accordance with embodiments of the invention.
**FIG. 22** illustrates a biosynthetic scheme for conversion of L-tyrosine to sanguinarine and benzophenanthridine alkaloids, in accordance with embodiments of the invention.

### DETAILED DESCRIPTION

Noscapinoids are an emerging class of microtubule-modulating anticancer agents with high water solubility that are of interest as chemotherapeutic agents for the treatment of human cancers. Noscapinoids are in the class of opium alkaloids that include compounds characterized as phthalideisoquinoline alkaloids. A number of noscapinoids have been synthesized that show enhanced tumor specificity and tumor regression, and little or no toxicity to normal tissues. Based on successive synthetic modifications at different points in the scaffold structure of the noscapinoid, aided by computational design and structure-activity relationship studies, the noscapinoids have been classified into different "generations" based on modifications. Noscapine is a noscapinoid compound that has been used worldwide as a cough suppressant for over fifty years and is made naturally in plants. Noscapine is a parent compound from which many noscapinoid compounds have been derived. The ability of noscapine to treat cancer was demonstrated in 1998. Although the dose required for treating cancer is much higher than when it serves as cough suppressant (1,000-2,250 mg/day for cancer treatment vs. 45-200 mg/day for cough treatment), the side effects of noscapine are fewer than traditional chemotherapy drugs. Noscpaine binds to tubulin with a stoichiometry of one noscapine molecule per tubulin dimer. However, unlike other tubulin binding chemotherapeutic agents that either depolymerize (nocodazole, colchicines, and vinca alkaloids) or over polymerize tubulin (paclitaxel), the anticancer activity of noscapine may be derived from its kinetic stabilization of the microtubules through a unique microbubule binding mode that causes apoptosis. From a pharmacological perspective, noscapine has many advantages as microtubule binding drugs: it is effective against multidrug resistant cancer cell lines, affects cancer cells differently from the normal dividing cells, and has a better pharmacokinetics profile with no significant side effects.

Before aspects of the invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the invention, representative illustrative methods and materials are now described.

The term "pathway derivative" as used herein refers to a compound having a chemical structure derived from the structure of a parent or reference compound (e.g., a compound disclosed herein) that differs by at least one structural difference, e.g., by having one or more substituents added, removed, modified, and/or further substituted. A structural difference may alter the core of the parent or reference compound, e.g., through a cyclization or ring-opening transformation. For example, pathway derivatives of reticuline include salutaridine and thebaine. A structural difference may arise by any method, e.g., by induced chemical transformation, by enzymatic catalysis, and/or by spontaneous reaction. Unless clearly indicated to the contrary, a pathway derivative need not be synthesized using the parent or reference compound as a starting material or as an intermediate, although in some cases, a derivative may be synthesized from the parent or reference compound.

The terms "analog" and "derivative" as used herein refer to a compound having a chemical structure that differs from the structure of a parent or reference compound (e.g., a compound disclosed herein) by having at least one structural difference, e.g., by having one or more substituents added, removed, modified, and/or further substituted from the parent or reference compound. A structural difference may alter the core of the parent or reference compound, e.g., through a cyclization or ring-opening transformation. For example, *N*-methylcanadine, 9-methyl-*N*-methylcanadine, and 6-chloro-canadine are analogs of canadine. Analogs of tyrosine include, for example, α-chloro-tyrosine, α-methyl tyrosine, and 3-nitro-tyrosine. A structural difference may arise by any method, e.g., by induced chemical transformation, by enzymatic catalysis, and/or by spontaneous reaction. Unless clearly indicated to the contrary, an analog need not be synthesized using the parent or reference compound as a starting material or as an intermediate, although in some cases, an analog may be synthesized from the parent or reference compound.

The term "functionalized derivative" as used herein refers to a compound having a structural feature that distinguishes it from a reference compound, wherein the distinguishing feature was present in the starting material used in the synthesis of the compound. For example, 3-chloro-reticuline can be produced from α-chloro-L-tyrosine and is considered herein to be a functionalized derivative of reticuline, as the chloride distinguishing the two compounds was a feature of the starting material used and not introduced by a step in the synthesis. Further examples include 6-hydroxy-canadine, a functionalized derivative of canadine that can be produced by a host cell from α-hydroxy-L-tyrosine, and 7-nitro-narcotoline, a functionalized derivative of narcotoline produced from 3-nitro-L-tyrosine.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Any recited method can be carried out in the order of events recited or in any other order which is logically possible.

### NOSCAPINOIDS

Host cells which produce noscapinoid products are provided. Noscapinoid products include noscapinoids, precursors of noscapinoids, metabolites of noscapinoids, analogs of noscapinoids, intermediates of noscapinoids, and/or derivatives of noscapinoids. Engineered strains of host cells may provide a platform for producing noscapinoid products and modifications thereof across several structural classes including, but not limited to, benzylisoquinoline alkaloids, protoberberines, secoberbines, phthalideisoquinolines, and others. Each of these classes is meant to include biosynthetic precursors, intermediates, and metabolites thereof, of any convenient member of an engineered host cell biosynthetic pathway that may lead to a member of the class. Non-limiting examples of compounds are given below for each of these structural classes. In some cases, the structure of a given example may or may not be characterized itself as a benzylisoquinoline alkaloid. The present chemical entities are meant to include all possible isomers, including single enantiomers, racemic mixtures, optically pure forms, mixtures of diastereomers, and intermediate mixtures.

Protoberberines may include, but are not limited to, scoulerine, cheilanthifoline, stylopine, nandinine, jatrorrhizine, stepholidine, discretamine, cis-*N*-methylstylopine, tetrahydrocolumbamine, palmatine, tetrahydropalmatine, columbamine, canadine, *N*-methylcanadine, 1-hydroxycanadine, berberine, *N*-methyl-ophiocarpine, 1,13-dihydroxy-*N*-methylcanadine, and 1-hydroxy-10-*O*-acetyl-*N*-methylcanadine.

Secoberbines may include, but are not limited to, 4'-*O*-desmethylmacrantaldehyde, 4'-*O*-desmethylpapaveroxine, 4'-*O*-desmethyl-3-*O*-acetylpapaveroxine, papaveroxine, and 3-*O*-aceteylpapaveroxine.

Phthalideisoquinolines may include, but are not limited to, narcotolinehemiacetal, narcotinehemiacetal, narcotoline, noscapine, adlumidine, adlumine, (+) or (-)-bicuculline, capnoidine, carlumine, corledine, corlumidine, decumbenine, 5'-*O*-demethylnarcotine, (+) or (-)-α or β-hydrastine, and hypecoumine.

A noscapinoid may be a derivative or an analog of noscapine and as such may also be characterized as a phthalideisoquinoline alkaloid. Any convenient noscapinoids may be targeted for production in the subject host cells. Noscapine analogues of interest include, but are not limited to, noscapine compounds that include modifications at the 6', 9', 1 and 7-positions, replacement of the naturally occurring N-methyl group in the 6'-position, e.g., with an N-ethylaminocarbonyl, substitution in the 9'-position, e.g., with a halo, aryl, alkyl, azido, or nitro, regioselective O-demethylation to reveal a free phenol in the 7-position, and reduction of the lactone to the corresponding cyclic ether in the 1-position; such as the compounds 9-bromonoscapine, 9-nitronoscapine, and 9-azidonoscapine and those compounds described by Naik et al., Journal of Molecular Modeling, 2012, 18(1):307-318, and Lopus and Naik, "Taking aim at a dynamic target: Noscapinoids as microtubule-targeted cancer therapeutics", Pharmacological Reports, 67 (1) 2015, 56-62 (published online 2014).

The term "noscapinoid product" is meant to include noscapinoids, precursors of noscapinoids, metabolites of noscapinoids, analogs of noscapinoids, intermediates of noscapinoids, and/or derivatives of noscapinoids. In some examples, noscapinoid derivatives and/or derivatives of noscapinoids may comprise pathway derivatives. In some examples, noscapinoid derivatives and/or derivatives of noscapinoids may comprise functionalized derivatives. In examples, a noscapinoid product may refer to any convenient protoberberine, secoberberine, or phthalideisoquinoline alkaloids that may be a precursor to noscapine or a noscapinoid of interest in a synthetic pathway of a cell, including, but not limited to, 1-hydroxy-N-methylcanadine, noscapine, narcotoline, narcotinehemiacetal, 4'-O-desmethyl-3-O-acetylpapaveroxine, narcotolinehemiacetal, N-methylophiocarpine, 1, 13-dihydroxy-N-methylcanadine, 1-hydroxy-13-Oacetyl-N-methylcanadine, narcotolinogendial (i.e., 4'-O-desmethylpapaveroxine), narcotolinal (i.e., 4'-O-desmethylmacrantaldehyde), and 1-hydroxycanadine. The noscapinoid product is downstream from canadine in the synthetic pathway of interest.

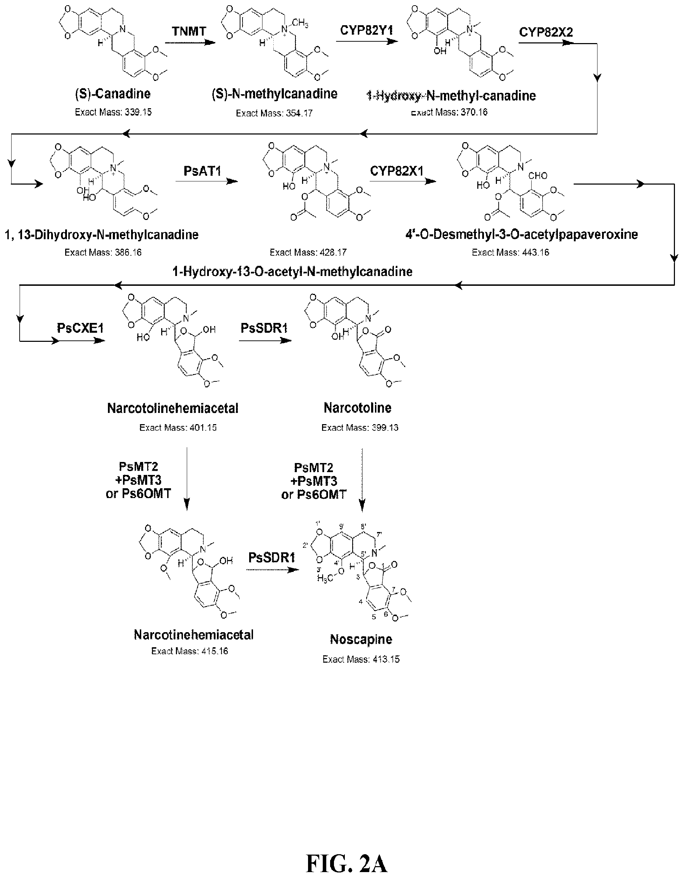

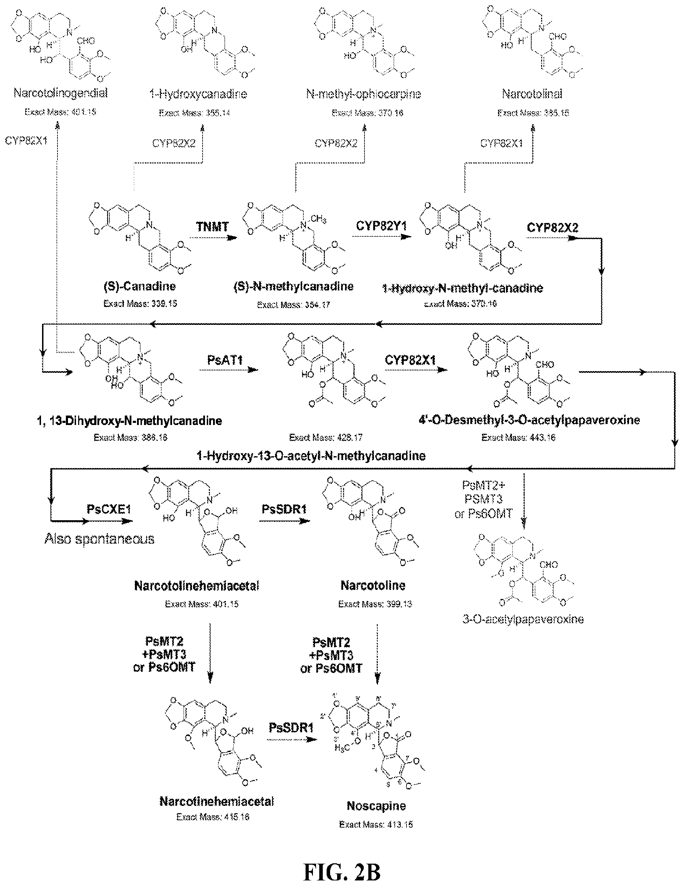

In some embodiments, the cell produces noscapine. In certain instances, the cell produces a derivative of noscapine. In some cases, the cell produces a noscapine precursor that is described in **FIGs. 2A** **and** **2B**. In some instances, the cell produces 1-hydroxy-N-methylcanadine. In certain embodiments, the cell produces 1-hydroxycanadine. In some cases, the cell produces 1, 13-dihydroxy-N-methylcanadine. In some instances, the cell produces N-methyl-ophiocarpine. In certain cases, the cell produces 4'-desmethyl-3-O-acetylpapaveroxine. In certain embodiments, the cell produces narcotolinal and narcotolinogendial.

Synthetic pathways to noscapinoids or a precursor thereof may be generated in the host cells, and may start with any convenient starting compound(s) or materials. The synthetic pathways generated in the host cells may start with any convenient compound that is a precursor of the noscapinoid of interest. The synthetic pathway of the subject host cells may be connected to any convenient upstream or downstream synthetic pathway to provide for the production of a target alkaloid from a desirable starting compound. The starting compound may be a precursor that is one or more biosynthetic steps removed from the target (such as two or more, three or more, or several biosynthetic steps removed). In some cases, the starting compound is itself produced by the host cell. The cell may produce noscapine or a noscapinoid of interest from canadine via a synthetic pathway that includes one or more noscapine precursors selected from the group consisting of (S)-N-methylcanadine, 1-hydroxy-N-methylcanadine, 1, 13-dihydroxy-N-methylcanadine, 1-hydroxy-13-Oacetyl-N-methylcanadine, 4'-desmethyl-3-O-acetylpapaveroxine, narcotolinehemiacetal, and narcotoline.

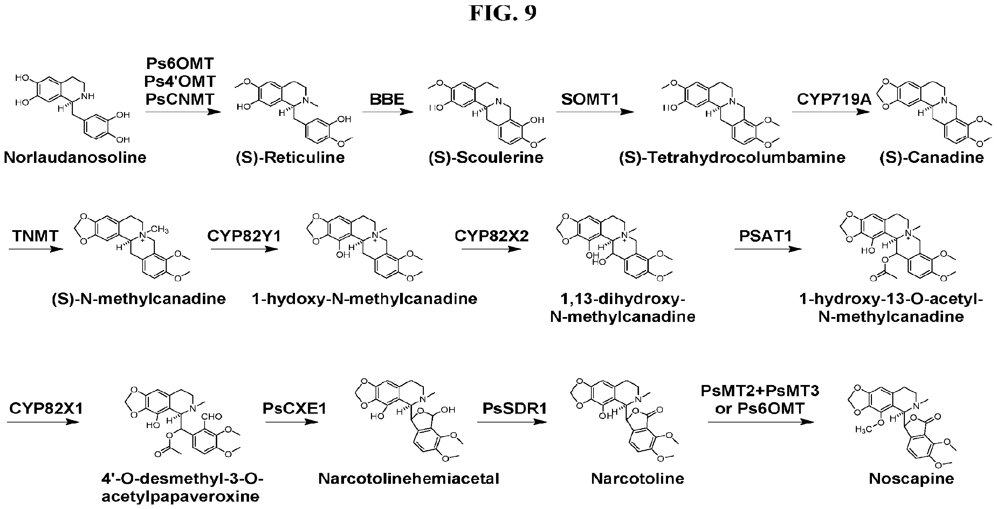

Any convenient host cells which produce a starting compound of interest may be adapted for use in the subject cells to produce noscapinoid or a precursor thereof. Starting compounds of interest include, but are not limited to, norlaudanosoline, canadine, scoulerine, tetrahydroberberrubine, stylopine, cheilanthifoline, tetrahydropalmatine, and any other convenient precursor of the target molecule that may be present in the endogenous noscapinoid pathway. In some cases, the starting compound is added to the host cell which converts the compound to a noscapinoid of interest, or precursor thereof. In some embodiments, the starting compound is canadine. **FIGs. 2A** **and** **2B** illustrate a synthetic pathway to noscapine and precursors thereof starting from (S)-canadine. In certain embodiments, the starting compound is norlaudanosoline. **FIG. 9** illustrates a synthetic pathway to noscapine and precursors thereof starting from norlaudanosoline. In certain instances, the starting compound may be produced by a norlaudanosoline-producing cell or a canadine-producing cell that is adapted to include a biosynthetic pathway that provides for production of a noscapinoid of interest, or a precursor thereof. In certain embodiments, the starting compound is tyrosine.

Thus, the starting material may be non-naturally occurring or the starting material may be naturally occurring. Other compounds may also be used as the starting material in the desired synthetic pathway, based upon the synthetic pathway present in the host cell. The source of the starting material may be from the host cell itself, e.g., tyrosine, or the starting material may be added or supplemented to the host cell from an outside source. For example, if the host cells are growing in liquid culture (an *in vivo* environment), the cell media may be supplemented with the starting material, e.g., tyrosine or norlaudanosoline, which is transported into the cells and converted into the desired products.

### HOST CELLS

As summarized above, one aspect of the invention is a host cell that produces a noscapinoid product. As used herein, the term "noscapinoid-producing cell" is meant to include cells that are engineered to produce a noscapinoid product from a starting compound via a synthetic pathway. The subject host cells may produce any convenient noscapinoid products, including but not limited to, 1-hydroxy-N-methylcanadine, N-methylcanadine, noscapine, narcotoline, narcotinehemiacetal, 4'-O-desmethyl-3-O-acetylpapaveroxine, narcotolinehemiacetal, N-methylophiocarpine, 1-hydroxy-13-Oacetyl-N-methylcanadine, 1,13-dihydroxy-N-methylcanadine, narcotolinogendial, 3-O-acetyl-papaveroxine, narcotolinal, and 1-hydroxylcanadine.

The host cells are yeast cells. Any convenient type of host cell may be utilized in producing the subject cells producing the subject noscapinoid-producing cells, see, e.g., US2008/0176754, WO/2012/039438, WO2013136057, US20140273109, and PCT application serial number US2014/063738 filed November 3, 2014.

Host cells of interest include, but are not limited to, bacterial cells, such as *Bacillus subtilis, Escherichia coli, Streptomyces,* and *Salmonella typhimuium* cells, insect cells such as *Drosophila melanogaster* S2 and *Spodoptera frugiperda* Sf9 cells, and yeast cells such as *Saccharomyces cerevisiae, Schizosaccharomyces pombe,* and *Pichia pastoris* cells. The host cell is a yeast cell. In certain instances, the yeast cells are ones that find use in industrial processes, such as yeast cells from the geus Pichia, including but not limited to, *P. farinose, P. anomala, P. heedii, P. guilliermondii, P. kluyveri, P. membranifaciens, P. norvegensis, P. ohmeri, P. pastoris, Pichia methanolica,* and *P*. *subpelliculosa.*

Any of the host cells described in US2008/0176754, WO/2012/039438, WO2013136057, US20140273109, and PCT application serial number US2014/063738 by Smolke et al. may be adapted for use in the subject cells and methods. In certain embodiments, the yeast cells may be of the species *Saccharomyces cerevisiae* (*S. cerevisiae*)*.* In certain embodiments, the yeast cells may be of the species *Schizosaccharomyces pombe.* In certain embodiments, the yeast cells may be of the species *Pichia pastoris.* Yeast is of interest as a host cell because cytochrome P450 proteins are able to fold properly into the endoplasmic reticulum membrane so that their activity is maintained. In examples, cytochrome P450 proteins are involved in some biosynthetic pathways of interest. In additional examples, cytochrome P450 proteins are involved in the production of noscapinoid products.

Yeast strains of interest that find use in the invention include, but are not limited to, CEN.PK (Genotype: *MATa*/α *ura3-52*/*ura3-52 trp1-289*/*trp1-289 leu2-3_112*/*leu2-3_112 his3* Δ*1*/*his3* Δ*1 MAL2-8C*/*MAL2-8C SUC2*/*SUC2*)*,* S288C, W303, D273-10B, X2180, A364A, Σ1278B, AB972, SK1, and FL100. In certain cases, the yeast strain is any of S288C (MATα; SUC2 mal mel gal2 CUP1 flo1 flo8-1 hap1), BY4741 (MATα; his3Δ1; leu2Δ0; met15Δ0; ura3Δ0), BY4742 (MATα; his3Δ1; leu2Δ0; lys2Δ0; ura3Δ0), BY4743 (MATa/MATα; his3Δ1/his3Δ1; leu2Δ0/leu2Δ0; met15Δ0/MET15; LYS2/lys2Δ0; ura3Δ0/ura3Δ0), and WAT11 or W(R), derivatives of the W303-B strain (MATa; ade2-1; his3-11, -15; leu2-3,-112; ura3-1; canR; cyr+) which express the *Arabidopsis thaliana* NADPH-P450 reductase ATR1 and the yeast NADPH-P450 reductase CPR1, respectively. In another embodiment, the yeast cell is W303alpha (MATα; his3-11,15 trp1-1 leu2-3 ura3-1 ade2-1). The identity and genotype of additional yeast strains of interest may be found at EUROSCARF (web.uni-frankfurt.de/fb15/mikro/euroscarf/col_index.html).

### Genetic Modifications to Host Cells

The host cells may be engineered to include one or more modifications (such as two or more, three or more, four or more, five or more, or even more modifications) that provide for the production of noscapinoid products. In some cases, a modification is a genetic modification, such as a mutation, addition, or deletion of a gene or fragment thereof, or transcription regulation of a gene or fragment thereof. As used herein, the term "mutation" refers to a deletion, insertion, or substitution of an amino acid(s) residue or nucleotide(s) residue relative to a reference sequence or motif. The mutation may be incorporated as a directed mutation to the native gene at the original locus. In some cases, the mutation may be incorporated as an additional copy of the gene introduced as a genetic integration at a separate locus, or as an additional copy on an episomal vector such as a 2µ or centromeric plasmid. In certain instances, the substrate inhibited copy of the enzyme is under the native cell transcriptional regulation. In some instances, the substrate inhibited copy of the enzyme is introduced with engineered constitutive or dynamic regulation of protein expression by placing it under the control of a synthetic promoter. In some examples, the object of one or more modifications may be a native gene. In some examples, the object of one or more modifications may be a non-native gene. In some examples, a non-native gene may be inserted into a host cell. In further examples, a non-native gene may be altered by one or more modifications prior to being inserted into a host cell.

An engineered host cell may overproduce one or more noscapinoid products. By overproduce is meant that the cell has an improved or increased production of a noscapinoid product relative to a control cell (e.g., an unmodified cell). By improved or increased production is meant both the production of some amount of the noscapinoid product where the control has no noscapinoid product production, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some noscapinoid product production.

In some cases, the one or more (such as two or more, three or more, or four or more) modifications may be selected from: a substrate inhibition alleviating mutation in a biosynthetic enzyme gene; a product inhibition alleviating mutation in a biosynthetic enzyme gene; a cofactor recovery promoting mechanism; a feedback inhibition alleviating mutation in a biosynthetic enzyme gene; and a transcriptional modulation modification of a biosynthetic enzyme gene; an inactivating mutation in an enzyme gene.

### Substrate Inhibition Alleviating Mutations

In some instances, the engineered host cells are cells that include one or more substrate inhibition alleviating mutations (such as two or more, three or more, four or more, five or more, or even more) in one or more biosynthetic enzyme genes of the cell. In some examples, the one or more biosynthetic enzyme genes are native to the cell (e.g., is present in an unmodified cell). In some examples, the one or more biosynthetic enzyme genes are non-native to the cell. As used herein, the term "substrate inhibition alleviating mutation" refers to a mutation that alleviates a substrate inhibition control mechanism of the cell.

A mutation that alleviates substrate inhibition reduces the inhibition of a regulated enzyme in the cell of interest relative to a control cell and provides for an increased level of the regulated compound or a downstream biosynthetic product thereof. In some cases, by alleviating inhibition of the regulated enzyme is meant that the IC₅₀ of inhibition is increased by 2-fold or more, such as by 3-fold or more, 5-fold or more, 10-fold or more, 30-fold or more, 100-fold or more, 300-fold or more, 1000-fold or more, or even more. By increased level is meant a level that is 110% or more of that of the regulated compound in a control cell or a downstream product thereof, such as 120% or more, 130% or more, 140% or more, 150% or more, 160% or more, 170% or more, 180% or more, 190% or more, or 200% or more, such as at least 3-fold or more, at least 5-fold or more, at least 10-fold or more or even more of the regulated compound in the engineered host cell or a downstream product thereof.

A variety of substrate inhibition control mechanisms and biosynthetic enzymes in the engineered host cell that are directed to regulation of levels of noscapine products may be targeted for substrate inhibition alleviation. The engineered host cell may include one or more substrate inhibition alleviating mutations in one or more biosynthetic enzyme genes. The one or more mutations may be located in any convenient biosynthetic enzyme genes where the biosynthetic enzyme is subject to regulatory control. In some embodiments, the engineered host cell may include one or more substrate inhibition alleviating mutations in one or more biosynthetic enzyme genes such as one of those genes described in **Table 1**.

Any convenient numbers and types of mutations may be utilized to alleviate a substrate inhibition control mechanism. In certain embodiments, the engineered host cells of the invention may include 1 or more, 2 or more, 3 or more, 4 or more, 5 or more, 6 or more, 7 or more, 8 or more, 9 or more, 10 or more, 11 or more, 12 or more, 13 or more, 14 or more, or even 15 or more substrate inhibition alleviating mutations, such as 1, 2, 3, 4, 5, 6, 7, 8, 9, 10, 11, 12, 13, 14, or 15 substrate inhibition alleviating mutations in one or more biosynthetic enzyme genes within the engineered host cell.

### Cofactor Recovery Promoting Mechanisms

In some instances, the engineered host cells are cells that include one or more cofactor recovery promoting mechanisms (such as two or more, three or more, four or more, five or more, or even more) in one or more biosynthetic enzyme genes of the cell. In some examples, the one or more biosynthetic enzyme genes are native to the cell (e.g., is present in an unmodified cell). In some examples, the one or more biosynthetic enzyme genes are non-native to the cell. As used herein, the term "cofactor recovery promoting mechanism" refers to a mechanism that promotes a cofactor recovery control mechanism of the cell. In some examples, the one or more cofactors of interest for recovery include but are not limited to S-adenosyl methionine, nicotinamide adenine dinucleotide phosphate, nicotinamide adenine dinucleotide, tetrahydrobiopterin, and flavin adenine dinucleotide.

A variety of cofactor recovery control mechanisms and biosynthetic enzymes in the engineered host cell that are directed to regulation of levels of noscapinoid products may be targeted for cofactor recovery promotion. The engineered host cell may include one or more cofactor recovery promoting mechanism in one or more biosynthetic enzyme genes. In some examples, the engineered host cell may include one or more cofactor recovery promoting mechanisms in one or more biosynthetic enzyme genes such as one of those genes described in **Table 1**.

Any convenient numbers and types of mechanisms may be utilized to promote a cofactor recovery control mechanism. In certain embodiments, the engineered host cells of the invention may include 1 or more, 2 or more, 3 or more, 4 or more, 5 or more, 6 or more, 7 or more, 8 or more, 9 or more, 10 or more, 11 or more, 12 or more, 13 or more, 14 or more, or even 15 or more cofactor recovery promoting mechanisms such as 1, 2, 3, 4, 5, 6, 7, 8, 9, 10, 11, 12, 13, 14, or 15 cofactor recovery promoting mechanisms in one or more biosynthetic enzyme genes within the engineered host cell.

### Product Inhibition Alleviating Mutations

In some instances, the engineered host cells are cells that include one or more product inhibition alleviating mutations (such as two or more, three or more, four or more, five or more, or even more) in one or more biosynthetic enzyme genes of the cell. In some examples, the one or more biosynthetic enzyme genes are native to the cell (e.g., is present in an unmodified cell). In some examples, the one or more biosynthetic enzyme genes are non-native to the cell. As used herein, the term "product inhibition alleviating mutation" refers to a mutation that alleviates a short term and/or long term product inhibition control mechanism of an engineered host cell. Short term product inhibition is a control mechanism of the cell in which there is competitive binding at a cosubstrate binding site. Long term product inhibition is a control mechanism of the cell in which there is irreversible binding of a compound away from a desired pathway.

A mutation that alleviates product inhibition reduces the inhibition of a regulated enzyme in the cell of interest relative to a control cell and provides for an increased level of the regulated compound or a downstream biosynthetic product thereof. In some cases, by alleviating inhibition of the regulated enzyme is meant that the IC₅₀ of inhibition is increased by 2-fold or more, such as by 3-fold or more, 5-fold or more, 10-fold or more, 30-fold or more, 100-fold or more, 300-fold or more, 1000-fold or more, or even more. By increased level is meant a level that is 110% or more of that of the regulated compound in a control cell or a downstream product thereof, such as 120% or more, 130% or more, 140% or more, 150% or more, 160% or more, 170% or more, 180% or more, 190% or more, or 200% or more, such as at least 3-fold or more, at least 5-fold or more, at least 10-fold or more or even more of the regulated compound in the engineered host cell or a downstream product thereof.

A variety of product inhibition control mechanisms and biosynthetic enzymes in the engineered host cell that are directed to regulation of levels of noscapinoid products may be targeted for product inhibition alleviation. The engineered host cell may include one or more product inhibition alleviating mutations in one or more biosynthetic enzyme genes. The mutation may be located in any convenient biosynthetic enzyme genes where the biosynthetic enzyme is subject to regulatory control. In some embodiments, the engineered host cell includes one or more product inhibition alleviating mutations in one or more biosynthetic enzyme genes such as one of those genes described in **Table 1**.

Any convenient numbers and types of mutations may be utilized to alleviate a product inhibition control mechanism. In certain embodiments, the engineered host cells of the invention may include 1 or more, 2 or more, 3 or more, 4 or more, 5 or more, 6 or more, 7 or more, 8 or more, 9 or more, 10 or more, 11 or more, 12 or more, 13 or more, 14 or more, or even 15 or more product inhibition alleviating mutations, such as 1, 2, 3, 4, 5, 6, 7, 8, 9, 10, 11, 12, 13, 14, or 15 product inhibition alleviating mutations in one or more biosynthetic enzyme genes within the engineered host cell.

### Feedback Inhibition Alleviating Mutations

In some instances, the engineered host cells are cells that include one or more feedback inhibition alleviating mutations (such as two or more, three or more, four or more, five or more, or even more) in one or more biosynthetic enzyme genes of the cell. In some cases, the one or more biosynthetic enzyme genes are native to the cell (e.g., is present in an unmodified cell). Additionally or alternatively, in some examples the one or more biosynthetic enzyme genes are non-native to the cell. As used herein, the term "feedback inhibition alleviating mutation" refers to a mutation that alleviates a feedback inhibition control mechanism of an engineered host cell. Feedback inhibition is a control mechanism of the cell in which an enzyme in the synthetic pathway of a regulated compound is inhibited when that compound has accumulated to a certain level, thereby balancing the amount of the compound in the cell. A mutation that alleviates feedback inhibition reduces the inhibition of a regulated enzyme in the engineered host cell relative to a control cell. In this way, the engineered host cell provides for an increased level of the regulated compound or a downstream biosynthetic product thereof. In some cases, by alleviating inhibition of the regulated enzyme is meant that the IC₅₀ of inhibition is increased by 2-fold or more, such as by 3-fold or more, 5-fold or more, 10-fold or more, 30-fold or more, 100-fold or more, 300-fold or more, 1000-fold or more, or even more. By increased level is meant a level that is 110% or more of that of the regulated compound in a control cell or a downstream product thereof, such as 120% or more, 130% or more, 140% or more, 150% or more, 160% or more, 170% or more, 180% or more, 190% or more, or 200% or more, such as at least 3-fold or more, at least 5-fold or more, at least 10-fold or more or even more of the regulated compound in the host cell or a downstream product thereof.

A variety of feedback inhibition control mechanisms and biosynthetic enzymes that are directed to regulation of levels of BIAs of interest may be targeted for alleviation in the host cell. The host cell may include one or more feedback inhibition alleviating mutations in one or more biosynthetic enzyme genes native to the cell. The one or more mutations may be located in any convenient biosynthetic enzyme genes where the biosynthetic enzyme is subject to regulatory control. In some embodiments, the engineered host cell may include one or more feedback inhibition alleviating mutations in one or more biosynthetic enzyme genes such as one of those genes described in **Table 1**.

Any convenient numbers and types of mutations may be utilized to alleviate a feedback inhibition control mechanism. As used herein, the term "mutation" refers to a deletion, insertion, or substitution of an amino acid(s) residue or nucleotide(s) residue relative to a reference sequence or motif. The mutation may be incorporated as a directed mutation to the native gene at the original locus. In some cases, the mutation may be incorporated as an additional copy of the gene introduced as a genetic integration at a separate locus, or as an additional copy on an episomal vector such as a 2µ or centromeric plasmid. In certain instances, the feedback inhibited copy of the enzyme is under the native cell transcriptional regulation. In some instances, the feedback inhibited copy of the enzyme is introduced with engineered constitutive or dynamic regulation of protein expression by placing it under the control of a synthetic promoter.

In certain embodiments, the engineered host cells of the invention may include 1 or more, 2 or more, 3 or more, 4 or more, 5 or more, 6 or more, 7 or more, 8 or more, 9 or more, 10 or more, 11 or more, 12 or more, 13 or more, 14 or more, or even 15 or more feedback inhibition alleviating mutations, such as 1, 2, 3, 4, 5, 6, 7, 8, 9, 10, 11, 12, 13, 14, or 15 feedback inhibition alleviating mutations in one or more biosynthetic enzyme genes within the engineered host cell.

### Transcriptional Modulation Modifications

The host cells may include one or more transcriptional modulation modifications (such as two or more, three or more, four or more, five or more, or even more modifications) of one or more biosynthetic enzyme genes of the cell. In some examples, the one or more biosynthetic enzyme genes are native to the cell. In some examples, the one or more biosynthetic enzyme genes are non-native to the cell. Any convenient biosynthetic enzyme genes of the cell may be targeted for transcription modulation. By transcription modulation is meant that the expression of a gene of interest in a modified cell is modulated, e.g., increased or decreased, enhanced or repressed, relative to a control cell (e.g., an unmodified cell). In some cases, transcriptional modulation of the gene of interest includes increasing or enhancing expression. By increasing or enhancing expression is meant that the expression level of the gene of interest is increased by 2-fold or more, such as by 5-fold or more and sometimes by 25-, 50-, or 100-fold or more and in certain embodiments 300-fold or more or higher, as compared to a control, i.e., expression in the same cell not modified (e.g., by using any convenient gene expression assay). Alternatively, in cases where expression of the gene of interest in a cell is so low that it is undetectable, the expression level of the gene of interest is considered to be increased if expression is increased to a level that is easily detectable. In certain instances, transcriptional modulation of the gene of interest includes decreasing or repressing expression. By decreasing or repressing expression is meant that the expression level of the gene of interest is decreased by 2-fold or more, such as by 5-fold or more and sometimes by 25-, 50-, or 100-fold or more and in certain embodiments 300-fold or more or higher, as compared to a control. In some cases, expression is decreased to a level that is undetectable. Modifications of host cell processes of interest that may be adapted for use in the subject host cells are described in U.S. Publication No. 20140273109 (14/211,611) by Smolke et al.

In some embodiments, the transcriptional modulation modification may include a substitution of a strong promoter for a native promoter of the one or more biosynthetic enzyme genes or the expression of an additional copy(ies) of the gene or genes under the control of a strong promoter. The promoters driving expression of the genes of interest may be constitutive promoters or inducible promoters, provided that the promoters may be active in the host cells. The genes of interest may be expressed from their native promoters. Additionally or alternatively, the genes of interest may be expressed from non-native promoters. Although not a requirement, such promoters may be medium to high strength in the host in which they are used. Promoters may be regulated or constitutive. In some embodiments, promoters that are not glucose repressed, or repressed only mildly by the presence of glucose in the culture medium, may be used. There are numerous suitable promoters, examples of which include promoters of glycolytic genes such as the promoter of the *B*. *subtilis* tsr gene (encoding fructose biphosphate aldolase) or GAPDH promoter from yeast *S*. *cerevisiae* (coding for glyceraldehyde-phosphate dehydrogenase) (Bitter G. A., Meth. Enzymol. 152:673 684 (1987)). Other strong promoters of interest include, but are not limited to, the ADHI promoter of baker's yeast (Ruohonen L., et al, J. Biotechnol. 39:193 203 (1995)), the phosphate-starvation induced promoters such as the PHO5 promoter of yeast (Hinnen, A., et al, in Yeast Genetic Engineering, Barr, P. J., et al. eds, Butterworths (1989), the alkaline phosphatase promoter from *B. licheniformis* (Lee. J. W. K., et al., J. Gen. Microbiol. 137:1127 1133 (1991)), GPD1, and TEF1. Yeast promoters of interest include, but are not limited to, inducible promoters such as Gal1-10, Gal1, GalL, GalS, repressible promoter Met25, tetO, and constitutive promoters such as glyceraldehyde 3-phosphate dehydrogenase promoter (GPD), alcohol dehydrogenase promoter (ADH), translation-elongation factor-1-alpha promoter (TEF), cytochrome c-oxidase promoter (CYC1), MRP7 promoter, etc. In some instances, the strong promoter is GPD1. In certain instances, the strong promoter is TEF1. Autonomously replicating yeast expression vectors containing promoters inducible by hormones such as glucocorticoids, steroids, and thyroid hormones are also known and include, but are not limited to, the glucorticoid responsive element (GRE) and thyroid hormone responsive element (TRE), see e.g., those promoters described in U.S. Pat. No. 7,045,290. Vectors containing constitutive or inducible promoters such as alpha factor, alcohol oxidase, and PGH may be used. Additionally any promoter/enhancer combination (as per the Eukaryotic Promoter Data Base EPDB) could also be used to drive expression of genes of interest. It is understood that any convenient promoters specific to the host cell may be selected, e.g., *E. coli.* In some cases, promoter selection may be used to optimize transcription, and hence, enzyme levels to maximize production while minimizing energy resources.

### Inactivating Mutations

The engineered host cells may include one or more inactivating mutations to an enzyme of the cell (such as two or more, three or more, four or more, five or more, or even more). The inclusion of one or more inactivating mutations may modify the flux of a synthetic pathway of an engineered host cell to increase the levels of a BIA of interest or a desirable enzyme or precursor leading to the same. In some examples, the one or more inactivating mutations are to an enzyme native to the cell. Additionally or alternatively, the one or more inactivating mutations are to an enzyme non-native to the cell. As used herein, by "inactivating mutation" is meant one or more mutations to a gene or regulatory DNA sequence of the cell, where the mutation(s) inactivates a biological activity of the protein expressed by that gene of interest. In some cases, the gene is native to the cell. In some instances, the gene encodes an enzyme that is inactivated and is part of or connected to the synthetic pathway of a BIA of interest produced by the host cell. In some instances, an inactivating mutation is located in a regulatory DNA sequence that controls a gene of interest. In certain cases, the inactivating mutation is to a promoter of a gene. Any convenient mutations (e.g., as described herein) may be utilized to inactivate a gene or regulatory DNA sequence of interest. By "inactivated" or "inactivates" is meant that a biological activity of the protein expressed by the mutated gene is reduced by 10% or more, such as by 20% or more, 30% or more, 40% or more, 50% or more, 60% or more, 70% or more, 80% or more, 90% or more, 95% or more, 97% or more, or 99% or more, relative to a control protein expressed by a non-mutated control gene. In some cases, the protein is an enzyme and the inactivating mutation reduces the activity of the enzyme.

In some examples, the engineered host cell includes an inactivating mutation in an enzyme native to the cell. Any convenient enzymes may be targeted for inactivation. Enzymes of interest may include, but are not limited to those enzymes, described in **Table 1** whose action in the synthetic pathway of the engineered host cell tends to reduce the levels of noscapinoid products.

### Heterologous coding sequences

The engineered host cells harbor one or more heterologous coding sequences (such as two or more, three or more, four or more, five or more) which encode activity(ies) that enable the engineered host cells to produce desired noscapinoid products, e.g., as described herein. As used herein, the term "heterologous coding sequence" is used to indicate any polynucleotide that codes for, or ultimately codes for, a peptide or protein or its equivalent amino acid sequence, e.g., an enzyme, that is not normally present in the host organism and may be expressed in the host cell under proper conditions. As such, "heterologous coding sequences" includes multiple copies of coding sequences that are normally present in the host cell, such that the cell is expressing additional copies of a coding sequence that are not normally present in the cells. The heterologous coding sequences may be RNA or any type thereof, e.g., mRNA, DNA or any type thereof, e.g., cDNA, or a hybrid of RNA/DNA. Coding sequences of interest include, but are not limited to, full-length transcription units that include such features as the coding sequence, introns, promoter regions, 3'-UTRs, and enhancer regions.

The engineered host cells may also be modified to possess one or more genetic alterations to accommodate the heterologous coding sequences. Alterations of the native host genome include, but are not limited to, modifying the genome to reduce or ablate expression of a specific protein that may interfere with the desired pathway. The presence of such native proteins may rapidly convert one of the intermediates or final products of the pathway into a metabolite or other compound that is not usable in the desired pathway. Thus, if the activity of the native enzyme were reduced or altogether absent, the produced intermediates would be more readily available for incorporation into the desired product. In some instances, where ablation of expression of a protein may be of interest, as in proteins involved in the pleiotropic drug response, including, but not limited to, ATP-binding cassette (ABC) transporters, multidrug resistance (MDR) pumps, and associated transcription factors.

The host cells may be modified to include a variety of plant proteins that provide for a desirable activity or property. Any convenient plant proteins related to the synthesis of a noscapinoid of interest or precursor thereof may be utilized in the engineered host cells, such as enzymes, chaperones, co-factors, and the like. In some cases, the host cell includes a plant chaperone protein. The plant chaperone may facilitate the action of an enzyme of interest in the host cell, thereby providing for an improved production of the noscapinoid of interest or precursor thereof. Plant chaperones of interest include, but are not limited to, binding immunoglobulin protein (BiP), DnaJ protein, glucose regulated protein (GRP) 94, binding protein (BiP), protein disulphide isomerase (PDI), cyclophilin, and calnexin.

Heterologous coding sequences include but are not limited to sequences that encode enzymes, either wild-type or equivalent sequences, that are normally responsible for the production of noscapinoid products in plants. In some cases, the enzymes for which the heterologous sequences code may be any of the enzymes in the 1-BIA pathway, and may be from any convenient source. The choice and number of enzymes encoded by the heterologous coding sequences for the particular synthetic pathway may be selected based upon the desired product. In certain embodiments, the host cells of the invention may include 2 or more, 3 or more, 4 or more, 5 or more, 6 or more, 7 or more, 8 or more, 9 or more, 10 or more, 11 or more, 12 or more, 13 or more, 14 or more, or even 15 or more heterologous coding sequences, such as 2, 3, 4, 5, 6, 7, 8, 9, 10, 11, 12, 13, 14, or 15 heterologous coding sequences.

As used herein, the term "heterologous coding sequences" also includes the coding portion of the peptide or enzyme, i.e., the cDNA or mRNA sequence, of the peptide or enzyme, as well as the coding portion of the full-length transcriptional unit, i.e., the gene including introns and exons, as well as "codon optimized" sequences, truncated sequences or other forms of altered sequences that code for the enzyme or code for its equivalent amino acid sequence, provided that the equivalent amino acid sequence produces a functional protein. Such equivalent amino acid sequences may have a deletion of one or more amino acids, with the deletion being N-terminal, C-terminal, or internal. Truncated forms are envisioned as long as they have the catalytic capability indicated herein. Fusions of two or more enzymes are also envisioned to facilitate the transfer of metabolites in the pathway, provided that catalytic activities are maintained.

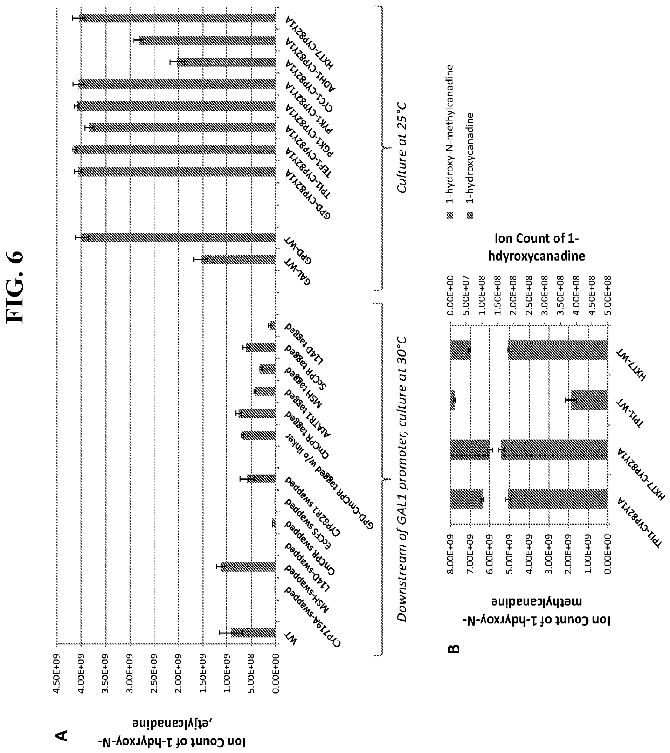

Operable fragments, mutants, or truncated forms may be identified by modeling and/or screening. In some cases, this is achieved by deletion of, for example, N-terminal, C-terminal, or internal regions of the protein in a step-wise fashion, followed by analysis of the resulting derivative with regard to its activity for the desired reaction compared to the original sequence. If the derivative in question operates in this capacity, it is considered to constitute an equivalent derivative of the enzyme proper. Any convenient enzyme of interest may be mutated or engineered to provide for a desirable biological activity in the engineered host cell. In some cases, the mutant enzyme is engineered to facilitate the correct folding of the enzyme. In certain instances, the mutant enzyme is engineered to increase a desirable activity or property of the enzyme relative to a non-mutated enzyme. In certain instances, the mutant enzyme is engineered to decrease an undesirable activity or property of the enzyme relative to a non-mutated enzyme. In some embodiments, the cell includes one or more heterologous coding sequences that encode one or more mutant enzymes. In some instances, the mutant enzyme is a CYP82Y1 N-terminus mutant. The N-terminus of CYP82Y1 may be engineered to facilitate the correct folding of the enzyme. **FIG. 6** illustrates an increase in noscapinoid precursor production using a variety of CYP82Y1 enzyme mutants. In some case, the activity of a first enzyme is improved through swapping the N-terminus tag with that of a second enzyme.

Aspects of the invention also relate to heterologous coding sequences that code for amino acid sequences that are equivalent to the native amino acid sequences for the various enzymes. An amino acid sequence that is "equivalent" is defined as an amino acid sequence that is not identical to the specific amino acid sequence, but rather contains at least some amino acid changes (deletions, substitutions, inversions, insertions, etc.) that do not essentially affect the biological activity of the protein as compared to a similar activity of the specific amino acid sequence, when used for a desired purpose. Equivalent sequences are also meant to include those which have been engineered and/or evolved to have properties different from the original amino acid sequence. Mutable properties of interest include catalytic activity, substrate specificity, selectivity, stability, solubility, localization, etc. In certain embodiments, an "equivalent" amino acid sequence contains at least 80%-99% identity at the amino acid level to the specific amino acid sequence, in some cases at least about 85%, 86%, 87%, 88%, 89%, 90%, 91%, 92%, 93%, 94% and more in certain cases, at least 95%, 96%, 97%, 98%, and 99% identity, at the amino acid level. In some cases, the amino acid sequence may be identical but the DNA sequence is altered such as to optimize codon usage for the host organism, for example.

In some instances, the expression of each type of enzyme is increased through additional gene copies (i.e., multiple copies), which increases intermediate accumulation and/or production of noscapinoid products. Embodiments of the invention include increased production of noscapinoid products in a host cell through simultaneous expression of multiple species variants of a single or multiple enzymes. In some cases, additional gene copies of a single or multiple enzymes are included in the host cell. Any convenient methods may be utilized including multiple copies of a heterologous coding sequence for an enzyme in the host cell.

In some examples, the engineered host cell includes multiple copies of a heterologous coding sequence for an enzyme, such as 2 or more, 3 or more, 4 or more, 5 or more, or even 10 or more copies. In certain embodiments, the engineered host cell includes multiple copies of heterologous coding sequences for one or more enzymes, such as multiple copies of two or more, three or more, four or more, etc. In some cases, the multiple copies of the heterologous coding sequence for an enzyme are derived from two or more different source organisms as compared to the host cell. For example, the engineered host cell may include multiple copies of one heterologous coding sequence, where each of the copies is derived from a different source organism. As such, each copy may include some variations in explicit sequences based on inter-species differences of the enzyme of interest that is encoded by the heterologous coding sequence.

Unless otherwise noted, the heterologous coding sequences are as reported in GENBANK. A list of enzymes of interest is shown in **Table 1**. The host cells of the invention may include any combination of the listed enzymes, from any source. Unless otherwise indicated, Accession numbers disclosed herein refer to GenBank. Some accession numbers refer to the *Saccharomyces* genome database (SGD), which is available on the world-wide web at www.yeastgenome.org.

The cell includes one or more heterologous coding sequences that encode one or more enzymes of interest (e.g., as described herein). Enzymes of interest include, but are not limited to, those enzymes described in **Table 1.** In certain embodiments, the cell includes a heterologous coding sequence that encodes a tetrahydroprotoberberine N-methyltransferase (TNMT). In some instances, the cell includes a heterologous coding sequence that encodes a N-methylcanadine 1-hydroxylase (CYP82Y1). In some cases, the cell includes a heterologous coding sequence that encodes a 1-hydroxy-N-methylcanadine 13-hydroxylase (CYP82X2). In certain instances, the cell includes a heterologous coding sequence that encodes a 1,13-dihydroxy-N-methylcanadine 3-O acetyl transferase (PsAT1). In some embodiments, the cell includes a heterologous coding sequence that encodes a 4'-O-desmethyl-3-O-acetylpapaveroxine synthase (CYP82X1). In some instances, the cell includes a heterologous coding sequence that encodes a narcotinehemiacetal synthase (PsCXE1). In certain instances, the cell includes a heterologous coding sequence that encodes a noscapine synthase (PsSDR1). In some cases, the cell includes one or more heterologous coding sequences (e.g., two or more) that encode one or more enzymes selected from PsMT3 and Ps6OMT. In certain cases, the cell includes heterologous coding sequences that encode the enzymes PsMT3 and PsMT2. In certain cases, the cell includes heterologous coding sequences that encode the enzymes Ps6OMT and PsMT2. In certain embodiments, the cell includes heterologous coding sequences that encode the enzymes CYP82Y1, CYP82X1, and CYP82X2. The heterologous coding sequences may be expressed in the host cell using any convenient methods. In some instances, the heterologous coding sequences are expressed from a low-copy construct. In some cases, the cell includes heterologous coding sequences that are derived from a source organism selected from *P*. *somniferum* and *E. californica.* In some embodiments, the cell lacks an enzyme of interest (e.g., as described herein). In certain embodiments, the cell lacks one or more enzymes selected from PsMT3, Ps6OMT, CYP82X1, CYP82X2, PsTNMT or EcTNMT, PsCXE1, PsSDR1, PsAT1, and CYP82Y1. In certain embodiments, the cell lacks two or more (such as three or more, four or more, five or more, six or more, seven or more, eight or more or nine or more) enzymes selected from PsMT3, Ps6OMT, CYP82X1, CYP82X2, TNMT, PsCXE1, PsSDR1, PsAT1, and CYP82Y1. Any combination of two or more of the enzymes described herein may be selected to be excluded from the subject cells.

In some embodiments, the cell includes a PsMT3 enzyme. In some embodiments, the cell includes a Ps6OMT enzyme. In some instances, the cell includes an enzyme that is a homodimer. The cell includes an enzyme that is a heterodimer (e.g., a dimer of two enzymes). In certain instances, the cell includes a heterodimeric enzyme of PsMT2+MT3. In certain cases, the cell includes a heterodimeric enzyme of PsMT2+6OMT.

In some instances, the host cell includes a heterologous coding sequence for a CPR enzyme. Any convenient CPR enzymes may be utilized in the subject host cells. In certain instances, the CPR enzyme is an *Arabidopsis thaliana P450* Reductase (ATR), e.g., ATR1, or CPR enzyme from *P*. *somniferum* (PsCPR). The cell may produce a noscapinoid of interest using one or more enzymes that provide for derivatization of noscapine in the cell. In certain embodiments, the cell includes one or more heterologous coding sequences for one or more enzymes selected from a P450, a halogenase, a glycosylase, a methyltransferase, an acetyltransferase, a short-chain dehydrogenase, a carboxylesterase, and a prenyltransferase.

The cell may produce the noscapinoid or precursor thereof from norlaudanosoline. **FIG. 9** illustrates a synthetic pathway to noscapine and precursors thereof starting from norlaudanosoline. In some embodiments, the cell includes one or more heterologous coding sequences that encode one or more enzymes selected from TNMT, PsAT1, CYP82X1, PsCXE1, PsSDR1, PsMT3, CYP82Y1, CYP82X1, S9OMT, Ps6OMT, Ps4'OMT, PsCNMT, PsBBE, AtATR1, and CYP719A. The heterologous coding sequences may be expressed in the cell using any convenient methods. Expression methods of interest vary depending on a variety of factors such as the enzyme of interest and the cell product of interest and include, but are not limited to, chromosomal integration, expression from a YAC, expression from a low-copy plasmid and expression from a high-copy plasmid. In certain embodiments, the cell expresses TNMT, PsAT1, CYP82X1, PsCXE1, PsSDR1, PsMT2 from a YAC. In certain embodiments, the cell expresses CYP82Y1, CYP82X1, PsCXE1, PsSDR1, PsMT3 from a YAC. In certain embodiments, the cell expresses CYP82X2, CYP82X1, PsCXE1, PsSDR1, PsMT2, PsAT1, TNMT from a YAC. In certain instances, the cell expresses CYP82Y1 and CYP82X1 from a low-copy plasmid. In some instances, the cell expresses S9OMT from a high-copy plasmid. In some case, Ps6OMT, Ps4'OMT, PsCNMT, PsBBE, AtATR1, and CYP719A are chromosomally integrated in the cell. In some cases, TNMT, PsAT1, CYP82X1, PsCXE1, PsSDR1, PsMT3, PsMT2, CYP82Y1, CYP82X1, S9OMT, Ps6OMT, Ps4'OMT, PsCNMT, PsBBE, AtATR1, and CYP719A are chromosomally integrated in the cell. In some cases, RnPTPS, RnSepR, RnPCD, RnQDHPR, RnDHFR, RnTyrH, CjNCS, PpDODC, EcCYP80B1, PsCPR, Ps6OMT, Ps4'OMT, PsCNMT, ARO4(Q166K), ARO7(T226I), ARO10, TKL1, TNMT, PsAT1, CYP82X1, PsCXE1, PsSDR1, PsMT3, PsMT2, CYP82Y1, CYP82X1, S9OMT, PsBBE, and CYP719A are chromosomally integrated in the cell.

In some embodiments, the host cell (i.e., a yeast strain) is engineered for selective production of a noscapinoid of interest, or a precursor thereof by localizing one or more enzymes to a compartment in the cell. Any convenient compartments or structures of a cell may be targeted for localization of an enzyme of interest. In some embodiments, the cell includes an enzyme that is spatially localized to a compartment in the yeast cell, wherein the compartment is selected from mitochondrion, endoplasmic reticulum (ER), golgi, vacuole, nucleus, plasma membrane, peroxisome, and periplasm. In some instances, an enzyme is localized to the yeast endoplasmic reticulum by fusing an ER targeting sequence to the N-terminus of the protein. In certain cases, an enzyme of interest is spatially localized to the outside of the compartment in the yeast cell. In some instances, an enzyme of interest is spatially localized to the inside of the compartment in the yeast cell.

In some cases, an enzyme may be located in the host cell such that the compound produced by this enzyme spontaneously rearranges, or is converted by another enzyme to a desirable metabolite before reaching a localized enzyme that may convert the compound into an undesirable metabolite. The spatial distance between two enzymes may be selected to prevent one of the enzymes from acting directly on a compound to make an undesirable metabolite, and restrict production of undesirable end products (e.g., an undesirable opioid by-product). In certain embodiments, any of the enzymes described herein, either singularly or together with a second enzyme, may be localized to any convenient compartment in the host cell, including but not limited to, an organelle, endoplasmic reticulum, golgi, vacuole, nucleus, plasma membrane, or the periplasm.

In some embodiments, the host cell includes one or more of the enzymes that include a localization tag. Any convenient localization tags may be utilized. In some cases, the localization tag is a peptidic sequence that is attached at the N-terminal and or C-terminal of the enzyme. Any convenient methods may be utilized for attaching a tag to the enzyme. In some cases, the localization tag is derived from an endogenous yeast protein. Such tags may provide routing to a variety of yeast organelles, including but not limited to, the endoplasmic reticulum (ER), mitochondria (MT), plasma membrane (PM), and vacuole (V). In certain instances, the tag includes or is derived from, a transmembrane domain from within the tail-anchored class of proteins. In some embodiments, the localization tag locates the enzyme on the outside of an organelle. In certain embodiments, the localization tag locates the enzyme on the inside of an organelle.

In some instances, the expression of each type of enzyme is increased through additional gene copies (i.e., multiple copies), which increases intermediate accumulation and ultimately noscapinoid and/or noscapinoid precursor production. Embodiments of the invention include increased noscapinoid production in a host cell through simultaneous expression of multiple species variants of a single or multiple enzymes. In some cases, additional gene copies of a single or multiple enzymes are included in the host cell. Any convenient methods may be utilized including multiple copies of a heterologous coding sequence for an enzyme in the host cell.

In some embodiments, the host cell includes multiple copies of a heterologous coding sequence for an enzyme, such as 2 or more, 3 or more, 4 or more, 5 or more, or even 10 or more copies. In certain embodiments, the host cell include multiple copies of heterologous coding sequences for one or more enzymes, such as multiple copies of two or more, three or more, four or more, etc. In some instances, the host cell includes multiple copies of a heterologous coding sequence that encodes an enzyme selected from CYP82Y1, CYP82X1, CYP82X2, PsCXE1, PsSDR1, PsMT2, and PsMT3. In certain cases, multiple copies of each of the heterologous coding sequences that encode the enzymes CYP82Y1, CYP82X1, CYP82X2, PsCXE1, PsSDR1, and PsMT3 are included in the host cell.

In some cases, the multiple copies of the heterologous coding sequence for an enzyme are derived from two or more different source organisms as compared to the host cell. For example, the host cell may include multiple copies of one heterologous coding sequence, where each of the copies is derived from a different source organism. In certain cases, the copies are derived from *P*. *somniferum* and *E. californica* source organisms. As such, each copy may include some variations in explicit sequences based on inter-species differences of the enzyme of interest that is encoded by the heterologous coding sequence. In some instances, the host cell includes multiple heterologous coding sequences that each encode an enzyme and are each derived from a different source organisms as compared to the host cell. In some embodiments, the host cell includes copies of an enzyme derived from two or more different source organisms as compared to the host cell. In certain embodiments, the cell includes two or more heterologous coding sequences that each encode a TNMT enzyme. In some instances, the host cell includes two or more heterologous coding sequences that each encode a TNMT and are derived from different source organisms. In certain cases, the source organisms for the TNMT enzyme are selected from *P*. *somniferum* and *E. californica.*

The engineered host cell medium may be sampled and monitored for the production of noscapinoid products. The noscapinoid products may be observed and measured using any convenient methods. Methods of interest include, but are not limited to, LC-MS methods (e.g., as described herein) where a sample of interest is analyzed by comparison with a known amount of a standard compound. Additionally, there are other ways that noscapinoid products may be observed and/or measured. Examples of alternative ways of observing and/or measuring BIAs include GC-MS, UV-vis spectroscopy, NMR, LC-NMR, LC-UV, TLC, capillary electrophoresis, among others. Identity may be confirmed, e.g., by m/z and MS/MS fragmentation patterns, and quantitation or measurement of the compound may be achieved via LC trace peaks of know retention time and/or EIC MS peak analysis by reference to corresponding LC-MS analysis of a known amount of a standard of the compound.

### METHODS

### Process Steps

As summarized above, aspects of the invention include methods of preparing noscapinoid products. As such, aspects of the invention include culturing an engineered host cell under conditions in which the one or more host cell modifications (e.g., as described herein) are functionally expressed such that the cell converts starting compounds of interest into noscapinoid products. Also provided are methods that include culturing an engineered host cell under conditions suitable for protein production such that one or more heterologous coding sequences are functionally expressed and convert starting compounds of interest into noscapinoid products. In examples, the method is a method of preparing a noscapinoid product that includes culturing an engineered host cell (e.g., as described herein); adding a starting compound to the cell culture; and recovering the noscapinoid product from the cell culture.

In certain cases, the noscapinoid that is produced in the cell is utilized in the cell as an intermediate or starting material that is itself subsequently converted into a downstream benzylisoquinoline alkaloid (BIA) of interest. Any convenient BIAs may be produced via the noscapinoid-producing cells described herein. BIA-producing cells of interest which may be adapted for use in the subject methods include, but are not limited to, those described by Smolke et al in US20140273109. As such, in some embodiments, the method further includes culturing the host cell under conditions sufficient to produce a BIA from the noscapinoid and recovering the BIA from the cell culture.

Fermentation media may contain suitable carbon substrates. The source of carbon suitable to perform the methods of this disclosure may encompass a wide variety of carbon containing substrates. Suitable substrates may include, without limitation, monosaccharides (e.g., glucose, fructose, galactose, xylose), oligosaccharides (e.g., lactose, sucrose, raffinose), polysaccharides (e.g., starch, cellulose), or a combination thereof. In some cases, unpurified mixtures from renewable feedstocks may be used (e.g., cornsteep liquor, sugar beet molasses, barley malt). In some cases, the carbon substrate may be a one-carbon substrate (e.g., methanol, carbon dioxide) or a two-carbon substrate (e.g., ethanol). In other cases, other carbon containing compounds may be utilized, for example, methylamine, glucosamine, and amino acids.

Any convenient methods of culturing engineered host cells may be employed for producing the noscapinoid products. The particular protocol that is employed may vary, e.g., depending on the engineered host cell, the heterologous coding sequences, the enzymes of interest, the BIAs of interest, etc. The cells may be present in any convenient environment, such as an environment in which the cells are capable of expressing one or more functional heterologous enzymes. In some embodiments, the cells are cultured under conditions that are conducive to enzyme expression and with appropriate substrates available to allow production of noscapinoid products *in vivo.* In some embodiments, the functional enzymes are extracted from the engineered host for production of noscapinoid products under *in vitro* conditions. In some instances, the engineered host cells are placed back into a multicellular host organism. The engineered host cells are in any phase of growth, including, but not limited to, stationary phase and log-growth phase, etc. In addition, the cultures themselves may be continuous cultures or they may be batch cultures.

Cells may be grown in an appropriate fermentation medium at a temperature between 14-40°C. Cells may be grown with shaking at any convenient speed (e.g., 200 rpm). Cells may be grown at a suitable pH. Suitable pH ranges for the fermentation may be between pH 5-9. Fermentations may be performed under aerobic, anaerobic, or microaerobic conditions. Any suitable growth medium may be used. Suitable growth media may include, without limitation, common commercially prepared media such as synthetic defined (SD) minimal media or yeast extract peptone dextrose (YEPD) rich media. Any other rich, defined, or synthetic growth media appropriate to the microorganism may be used.

Cells may be cultured in a vessel of essentially any size and shape. Examples of vessels suitable to perform the methods of this disclosure may include, without limitation, multi-well shake plates, test tubes, flasks (baffled and non-baffled), and bioreactors. The volume of the culture may range from 10 microliters to greater than 10,000 liters.

The addition of agents to the growth media that are known to modulate metabolism in a manner desirable for the production of alkaloids may be included. In a non-limiting example, cyclic adenosine 2'3'-monophosphate may be added to the growth media to modulate catabolite repression.

Any convenient cell culture conditions for a particular cell type may be utilized. In certain embodiments, the host cells that include one or more modifications are cultured under standard or readily optimized conditions, with standard cell culture media and supplements. As one example, standard growth media when selective pressure for plasmid maintenance is not required may contain 20 g/L yeast extract, 10 g/L peptone, and 20 g/L dextrose (YPD). Host cells containing plasmids are grown in synthetic complete (SC) media containing 1.7 g/L yeast nitrogen base, 5 g/L ammonium sulfate, and 20 g/L dextrose supplemented with the appropriate amino acids required for growth and selection. Alternative carbon sources which may be useful for inducible enzyme expression include, but are not limited to, sucrose, raffinose, and galactose. Cells are grown at any convenient temperature (e.g., 30°C) with shaking at any convenient rate (e.g., 200 rpm) in a vessel, e.g., in test tubes or flasks in volumes ranging from 1-1000 mL, or larger, in the laboratory.

Culture volumes may be scaled up for growth in larger fermentation vessels, for example, as part of an industrial process. The industrial fermentation process may be carried out under closed-batch, fed-batch, or continuous chemostat conditions, or any suitable mode of fermentation. In some cases, the cells may be immobilized on a substrate as whole cell catalysts and subjected to fermentation conditions for alkaloid production.

A batch fermentation is a closed system, in which the composition of the medium is set at the beginning of the fermentation and not altered during the fermentation process. The desired organism(s) are inoculated into the medium at the beginning of the fermentation. In some instances, the batch fermentation is run with alterations made to the system to control factors such as pH and oxygen concentration (but not carbon). In this type of fermentation system, the biomass and metabolite compositions of the system change continuously over the course of the fermentation. Cells typically proceed through a lag phase, then to a log phase (high growth rate), then to a stationary phase (growth rate reduced or halted), and eventually to a death phase (if left untreated).

A continuous fermentation is an open system, in which a defined fermentation medium is added continuously to the bioreactor and an equal amount of fermentation media is continuously removed from the vessel for processing. Continuous fermentation systems are generally operated to maintain steady state growth conditions, such that cell loss due to medium being removed must be balanced by the growth rate in the fermentation. Continuous fermentations are generally operated at conditions where cells are at a constant high cell density. Continuous fermentations allow for the modulation of one or more factors that affect target product concentration and/or cell growth.

The liquid medium may include, but is not limited to, a rich or synthetic defined medium having an additive component described above. Media components may be dissolved in water and sterilized by heat, pressure, filtration, radiation, chemicals, or any combination thereof. Several media components may be prepared separately and sterilized, and then combined in the fermentation vessel. The culture medium may be buffered to aid in maintaining a constant pH throughout the fermentation.

Process parameters including temperature, dissolved oxygen, pH, stirring, aeration rate, and cell density may be monitored or controlled over the course of the fermentation. For example, temperature of a fermentation process may be monitored by a temperature probe immersed in the culture medium. The culture temperature may be controlled at the set point by regulating the jacket temperature. Water may be cooled in an external chiller and then flowed into the bioreactor control tower and circulated to the jacket at the temperature required to maintain the set point temperature in the vessel.

Additionally, a gas flow parameter may be monitored in a fermentation process. For example, gases may be flowed into the medium through a sparger. Gases suitable for the methods of this disclosure may include compressed air, oxygen, and nitrogen. Gas flow may be at a fixed rate or regulated to maintain a dissolved oxygen set point.

The pH of a culture medium may also be monitored. In examples, the pH may be monitored by a pH probe that is immersed in the culture medium inside the vessel. If pH control is in effect, the pH may be adjusted by acid and base pumps which add each solution to the medium at the required rate. The acid solutions used to control pH may be sulfuric acid or hydrochloric acid. The base solutions used to control pH may be sodium hydroxide, potassium hydroxide, or ammonium hydroxide.

Further, dissolved oxygen may be monitored in a culture medium by a dissolved oxygen probe immersed in the culture medium. If dissolved oxygen regulation is in effect, the oxygen level may be adjusted by increasing or decreasing the stirring speed. The dissolved oxygen level may also be adjusted by increasing or decreasing the gas flow rate. The gas may be compressed air, oxygen, or nitrogen.

Stir speed may also be monitored in a fermentation process. In examples, the stirrer motor may drive an agitator. The stirrer speed may be set at a consistent rpm throughout the fermentation or may be regulated dynamically to maintain a set dissolved oxygen level.

Additionally, turbidity may be monitored in a fermentation process. In examples, cell density may be measured using a turbidity probe. Alternatively, cell density may be measured by taking samples from the bioreactor and analyzing them in a spectrophotometer. Further, samples may be removed from the bioreactor at time intervals through a sterile sampling apparatus. The samples may be analyzed for alkaloids produced by the host cells. The samples may also be analyzed for other metabolites and sugars, the depletion of culture medium components, or the density of cells.

In another example, a feed stock parameter may be monitored during a fermentation process. In particular, feed stocks including sugars and other carbon sources, nutrients, and cofactors that may be added into the fermentation using an external pump. Other components may also be added during the fermentation including, without limitation, anti-foam, salts, chelating agents, surfactants, and organic liquids.

Any convenient codon optimization techniques for optimizing the expression of heterologous polynucleotides in host cells may be adapted for use in the subject host cells and methods, see e.g., Gustafsson, C. et al. (2004) Trends Biotechnol, 22, 346-353.

The subject method may also include adding a starting compound to the cell culture. Any convenient methods of addition may be adapted for use in the subject methods. The cell culture may be supplemented with a sufficient amount of the starting materials of interest (e.g., as described herein), e.g., a mM to µM amount such as between about 1-5 mM of a starting compound. It is understood that the amount of starting material added, the timing and rate of addition, the form of material added, etc., may vary according to a variety of factors. The starting material may be added neat or pre-dissolved in a suitable solvent (e.g., cell culture media, water, or an organic solvent). The starting material may be added in concentrated form (e.g., 10x over desired concentration) to minimize dilution of the cell culture medium upon addition. The starting material may be added in one or more batches, or by continuous addition over an extended period of time (e.g., hours or days).

### Methods for Isolating Products from the Fermentation Medium

The subject methods may also include recovering the noscapinoid products from the cell culture. Any convenient methods of separation and isolation (e.g., chromatography methods or precipitation methods) may be adapted for use in the subject methods to recover the noscapinoid products from the cell culture. Filtration methods may be used to separate soluble from insoluble fractions of the cell culture. In some cases, liquid chromatography methods (e.g., reverse phase HPLC, size exclusion, normal phase chromatography) may be used to separate the noscapinoid products of interest from other soluble components of the cell culture. In some cases, extraction methods (e.g., liquid extraction, pH based purification, solid phase extraction, affinity chromatography, ion exchange, etc.) may be used to separate the noscapinoid products from other components of the cell culture.

The produced alkaloids may be isolated from the fermentation medium using methods known in the art. A number of recovery steps may be performed immediately after (or in some instances, during) the fermentation for initial recovery of the desired product. Through these steps, the alkaloids (e.g., noscapinoid products) may be separated from the cells, cellular debris and waste, and other nutrients, sugars, and organic molecules may remain in the spent culture medium. This process may be used to yield a product enriched with noscapinoid products.

In an example, a product stream having a noscapinoid product is formed by providing engineered yeast cells and a feedstock including nutrients and water to a batch reactor. In particular, the engineered yeast cells may be subjected to fermentation by incubating the engineered yeast cells for a time period of at least about 5 minutes to produce a solution comprising the noscapinoid product and cellular material. Once the engineered yeast cells have been subjected to fermentation, at least one separation unit may be used to separate the noscapinoid product from the cellular material to provide the product stream comprising the noscapinoid product. In particular, the product stream may include the noscapinoid product as well as additional components, such as a clarified yeast culture medium. Additionally, a noscapinoid product may comprise one or more noscapinoid products, such as one or more noscapinoid compounds.

Different methods may be used to remove cells from a bioreactor medium that include noscapinoid products. In examples, cells may be removed by sedimentation over time. This process of sedimentation may be accelerated by chilling or by the addition of fining agents such as silica. The spent culture medium may then be siphoned from the top of the reactor or the cells may be decanted from the base of the reactor. Alternatively, cells may be removed by filtration through a filter, a membrane, or other porous material. Cells may also be removed by centrifugation, for example, by continuous flow centrifugation or by using a continuous extractor.

If some valuable noscapinoid products are present inside the cells, the cells may be permeabilized or lysed and the cell debris may be removed by any of the methods described above. Agents used to permeabilize the cells may include, without limitation, organic solvents (e.g., DMSO) or salts (e.g., lithium acetate). Methods to lyse the cells may include the addition of surfactants such as sodium dodecyl sulfate, or mechanical disruption by bead milling or sonication.

Noscapinoid products may be extracted from the clarified spent culture medium through liquid-liquid extraction by the addition of an organic liquid that is immiscible with the aqueous culture medium. In examples, the use of liquid-liquid extraction may be used in addition to other processing steps. Examples of suitable organic liquids include, but are not limited to, isopropyl myristate, ethyl acetate, chloroform, butyl acetate, methylisobutyl ketone, methyl oleate, toluene, oleyl alcohol, ethyl butyrate. The organic liquid may be added to as little as 10% or as much as 100% of the volume of aqueous medium.

In some cases, the organic liquid may be added at the start of the fermentation or at any time during the fermentation. This process of extractive fermentation may increase the yield of noscapinoid products from the host cells by continuously removing noscapinoid products to the organic phase.

Agitation may cause the organic phase to form an emulsion with the aqueous culture medium. Methods to encourage the separation of the two phases into distinct layers may include, without limitation, the addition of a demulsifier or a nucleating agent, or an adjustment of the pH. The emulsion may also be centrifuged to separate the two phases, for example, by continuous conical plate centrifugation.

Alternatively, the organic phase may be isolated from the aqueous culture medium so that it may be physically removed after extraction. For example, the solvent may be encapsulated in a membrane.

In examples, noscapinoid products may be extracted from a fermentation medium using adsorption methods. In examples, noscapinoid products may be extracted from clarified spent culture medium by the addition of a resin such as Amberlite^{®} XAD4 or another agent that removes noscapinoid products by adsorption. The noscapinoid products may then be released from the resin using an organic solvent. Examples of suitable organic solvents include, but are not limited to, methanol, ethanol, ethyl acetate, or acetone.

Noscapinoid products may also be extracted from a fermentation medium using filtration. At high pH, the noscapinoid products may form a crystalline-like precipitate in the bioreactor. This precipitate may be removed directly by filtration through a filter, membrane, or other porous material. The precipitate may also be collected by centrifugation and/or decantation.

The extraction methods described above may be carried out either *in situ* (in the bioreactor) or *ex situ* (e.g., in an external loop through which media flows out of the bioreactor and contacts the extraction agent, then is recirculated back into the vessel). Alternatively, the extraction methods may be performed after the fermentation is terminated using the clarified medium removed from the bioreactor vessel.

The subject methods may also include recovering the noscapinoid or precursor thereof from the cell culture. Any convenient methods of separation and isolation (e.g., organic solvent extraction under basic condition, solid phase extraction, chromatography methods, or precipitation methods) may be adapted for use in the subject methods to recover the noscapinoids of interest or precursors thereof from the cell culture. Filtration methods may be used to separate soluble from insoluble fractions of the cell culture. In some cases, liquid chromatography methods (e.g., reverse phase HPLC, size exclusion, normal phase chromatography) are used to separate the noscapinoid or precursor from other soluble components of the cell culture. In certain cases, the noscapinoid-producing cell (e.g., as described herein) coverts the noscapinoid into a downstream BIA of interest. As such, any of the methods described above may also be applied to recovery of any BIAs of interest that are produced.

Also included are methods of engineering host cells for the purpose of producing noscapinoids of interest or precursors thereof. Inserting DNA into host cells may be achieved using any convenient methods. The methods are used to insert the heterologous coding sequences into the host cells such that the host cells functionally express the enzymes and convert starting compounds of interest into product noscapinoids of interest or precursors thereof.

In some embodiments, the cell includes one or more promoters for the one or more of the heterologous coding sequences (e.g., as described herein). Any convenient promoters may be utilized in the subject host cells and methods. The promoters driving expression of the heterologous coding sequences may be constitutive promoters or inducible promoters, provided that the promoters can be active in the host cells. The heterologous coding sequences may be expressed from their native promoters, or non-native promoters may be used. Such promoters may be low to high strength in the host in which they are used. In some embodiments, the cell includes one or more strong promoters. Promoters may be regulated or constitutive. In certain embodiments, promoters that are not glucose repressed, or repressed only mildly by the presence of glucose in the culture medium, are used. Promoters of interest include but are not limited to, promoters of glycolytic genes such as the promoter of the *B*. *subtilis* tsr gene (encoding fructose bisphosphate aldolase) or GAPDH promoter from yeast *S*. *cerevisiae* (coding for glyceraldehyde-phosphate dehydrogenase), the ADH1 promoter of baker's yeast, the phosphate-starvation induced promoters such as the PHO5 promoter of yeast, the alkaline phosphatase promoter from *B*. *licheniformis,* yeast inducible promoters such as Gal1-10, Gal1, GalL, GalS, repressible promoter Met25, tetO, and constitutive promoters such as glyceraldehyde 3-phosphate dehydrogenase promoter (GPD), alcohol dehydrogenase promoter (ADH1), translation-elongation factor-1-α promoter (TEF), cytochrome c-oxidase promoter (CYC1), MRP7 promoter, GAL1, HXT7, PGK1, TPI1, PYK1, TEF1, etc. Autonomously replicating yeast expression vectors containing promoters inducible by hormones such as glucocorticoids, steroids, and thyroid hormones may also be used and include, but are not limited to, the glucorticoid responsive element (GRE) and thyroid hormone responsive element (TRE). These and other examples are described U.S. Pat. No. 7,045,290, including the references cited therein. Additional vectors containing constitutive or inducible promoters such as α factor, alcohol oxidase, and PGH may be used. Additionally any promoter/enhancer combination (as per the Eukaryotic Promoter Data Base EPDB) could also be used to drive expression of genes. Any convenient appropriate promoters may be selected for the host cell, e.g., *E. coli.* One can also use promoter selection to optimize transcript, and hence, enzyme levels to maximize production while minimizing energy resources.

In some instances, the cell includes one or more strong promoters selected from HXT7, ADH1, PGK1, TPI1, PYK1, and TEF1. In certain embodiments, the cell includes one or more heterologous coding sequences that encode CYP82Y1 or a CYP82Y1 mutant and includes a HXT7 promoter. In certain cases, the cell produces 1-hydroxy-N-methylcanadine. In certain instances, the cell produces 1-hydroxycanadine. In some embodiments, the cell includes one or more heterologous coding sequences that encode CYP82X2 or a CYP82X2 mutant and includes a HXT7 promoter. In certain instances, the cell produces 1,13-dihydroxy-N-methylcanadine. In some instances, the cell includes one or more heterologous coding sequences that encode CYP82X2 or a CYP82X2 mutant and includes one or more promoters selected from PGK1 and GPD. In certain embodiments, the cell produces N-methyl-ophiocarpine. In some instances, the cell includes one or more heterologous coding sequences that encode CYP82X1 or a CYP82X1 mutant and includes a HXT7 promoter. In certain embodiments, the cell produces 4'-O-desmethyl-3-O-acetylpapaveroxine.

Any convenient vectors may be utilized in the subject host cells and methods. Vectors of interest include vectors for use in yeast and other cells. Yeast vectors can be broken up into 4 general categories: integrative vectors (Ylp), autonomously replicating high copy-number vectors (YEp), autonomously replicating low copy-number vectors (YCp) and vectors for cloning large fragments (YACs). Vector DNA can be introduced into prokaryotic or eukaryotic cells via any convenient transformation or transfection techniques.

### Methods for Purifying Products from Alkaloid-Enriched Solutions

Subsequent purification steps may involve treating the post-fermentation solution enriched with noscapinoid products using methods known in the art to recover individual product species of interest to high purity.

In one example, noscapinoid products extracted in an organic phase may be transferred to an aqueous solution. In some cases, the organic solvent may be evaporated by heat and/or vacuum, and the resulting powder may be dissolved in an aqueous solution of suitable pH. In a further example, the noscapinoid products may be extracted from the organic phase by addition of an aqueous solution at a suitable pH that promotes extraction of the noscapinoid products into the aqueous phase. The aqueous phase may then be removed by decantation, centrifugation, or another method.

The noscapinoid product-containing solution may be further treated to remove metals, for example, by treating with a suitable chelating agent. The noscapinoid product-containing solution may be further treated to remove other impurities, such as proteins and DNA, by precipitation. In one example, the noscapinoid product-containing solution is treated with an appropriate precipitation agent such as ethanol, methanol, acetone, or isopropanol. In an alternative example, DNA and protein may be removed by dialysis or by other methods of size exclusion that separate the smaller alkaloids from contaminating biological macromolecules.

In further examples, the solution containing noscapinoid products may be extracted to high purity by continuous cross-flow filtration using methods known in the art.

If the solution contains a mixture of noscapinoid products, it may be subjected to acid-base treatment to yield individual noscapinoid product species using methods known in the art. In this process, the pH of the aqueous solution is adjusted to precipitate individual noscapinoid products.

For high purity, small-scale preparations, the noscapinoid products may be purified in a single step by liquid chromatography.

### Yeast-Derived Alkaloid APIs Versus Plant-Derived APIs

The clarified yeast culture medium (CYCM) may contain a plurality of impurities. The clarified yeast culture medium may be dehydrated by vacuum and/or heat to yield an alkaloid-rich powder. This product is analogous to the concentrate of poppy straw (CPS), which is exported from poppy-growing countries and purchased by API manufacturers. For the purposes of this invention, CPS is a representative example of any type of purified plant extract from which the desired alkaloids product(s) may ultimately be further purified. **Table 2** and **Table 3** highlight the impurities in these two products that may be specific to either CYCM or CPS or may be present in both. While some noscapinoid products may have a pigment as an impurity, other noscapinoid products may be categorized as pigments themselves. Accordingly, these noscapinoid products may be assessed for impurities based on non-pigment impurities. Additionally, in some examples, a noscapinoid product that is produced using a fermentation process may contain portions of one or more cells that are used in a fermentation process to produce the noscapinoid product. For example, when yeast are used to product the noscapinoid product, portions of the yeast cell may be within the noscapinoid product. By analyzing a product of unknown origin for a subset of these impurities, a person of skill in the art could determine whether the product originated from a yeast or plant production host.

API-grade pharmaceutical ingredients are highly purified molecules. As such, impurities that could indicate the plant- or yeast-origin of an API (such as those listed in **Table 2** and **Table 3**) may not be present at the API stage of the product. Indeed, many of the API products derived from yeast strains of the invention may be largely indistinguishable from the traditional plant-derived APIs. In some cases, however, conventional alkaloid compounds may be subjected to chemical modification using chemical synthesis approaches, which may show up as chemical impurities in plant-based products that require such chemical modifications. For example, chemical derivatization may often result in a set of impurities related to the chemical synthesis processes. In certain situations, these modifications may be performed biologically in the yeast production platform, thereby avoiding some of the impurities associated with chemical derivation from being present in the yeast-derived product. In particular, these impurities from the chemical derivation product may be present in an API product that is produced using chemical synthesis processes but may be absent from an API product that is produced using a yeast-derived product. Alternatively, if a yeast-derived product is mixed with a chemically-derived product, the resulting impurities may be present but in a lesser amount than would be expected in an API that only or primarily contains chemically-derived products. In this example, by analyzing the API product for a subset of these impurities, a person of skill in the art could determine whether the product originated from a yeast production host or the traditional chemical derivatization route.

Non-limiting examples of impurities that may be present in biosynthesized APIs but not in chemically-derivatized APIs may include portions of non-plant cells that are utilized to produce noscapinoid products. In examples, biosynthesized APIs may include portions of yeast cells that are utilized to ferment noscapinoid products within the yeast cells. Additionally, in the case where the yeast-derived compound and the plant-derived compound are both subjected to chemical modification through chemical synthesis approaches, the same impurities associated with the chemical synthesis process may be expected in the products. In such a situation, the starting material (e.g., CYCM or CPS) may be analyzed as described above.

### Methods of Engineering Host Cells

Also disclosed are methods of engineering host cells for the purpose of producing noscapinoid products. Inserting DNA into host cells may be achieved using any convenient methods. The methods are used to insert the heterologous coding sequences into the engineered host cells such that the host cells functionally express the enzymes and convert starting compounds of interest into noscapinoid products.

Any convenient promoters may be utilized in the subject engineered host cells and methods. The promoters driving expression of the heterologous coding sequences may be constitutive promoters or inducible promoters, provided that the promoters are active in the engineered host cells. The heterologous coding sequences may be expressed from their native promoters, or non-native promoters may be used. Such promoters may be low to high strength in the host in which they are used. Promoters may be regulated or constitutive. In certain embodiments, promoters that are not glucose repressed, or repressed only mildly by the presence of glucose in the culture medium, are used. Promoters of interest include but are not limited to, promoters of glycolytic genes such as the promoter of the *B*. *subtilis* tsr gene (encoding the promoter region of the fructose bisphosphate aldolase gene) or the promoter from yeast *S*. *cerevisiae* gene coding for glyceraldehyde 3-phosphate dehydrogenase (GPD, GAPDH, or TDH3), the ADH1 promoter of baker's yeast, the phosphate-starvation induced promoters such as the PHO5 promoter of yeast, the alkaline phosphatase promoter from *B. licheniformis,* yeast inducible promoters such as Gal1-10, Gal1, GalL, GalS, repressible promoter Met25, tetO, and constitutive promoters such as glyceraldehyde 3-phosphate dehydrogenase promoter (GPD), alcohol dehydrogenase promoter (ADH), translation-elongation factor-1-α promoter (TEF), cytochrome c-oxidase promoter (CYC1), MRP7 promoter, etc. Autonomously replicating yeast expression vectors containing promoters inducible by hormones such as glucocorticoids, steroids, and thyroid hormones may also be used and include, but are not limited to, the glucorticoid responsive element (GRE) and thyroid hormone responsive element (TRE). These and other examples are described U.S. Pat. No. 7,045,290, including the references cited therein. Additional vectors containing constitutive or inducible promoters such as α factor, alcohol oxidase, and PGH may be used. Additionally any promoter/enhancer combination (as per the Eukaryotic Promoter Data Base EPDB) could also be used to drive expression of genes. Any convenient appropriate promoters may be selected for the host cell, e.g., *E. coli.* One may also use promoter selection to optimize transcript, and hence, enzyme levels to maximize production while minimizing energy resources.

Any convenient vectors may be utilized in the subject engineered host cells and methods. Vectors of interest include vectors for use in yeast and other cells. The types of yeast vectors may be broken up into 4 general categories: integrative vectors (Ylp), autonomously replicating high copy-number vectors (YEp or 2µ plasmids), autonomously replicating low copy-number vectors (YCp or centromeric plasmids) and vectors for cloning large fragments (YACs). Vector DNA is introduced into prokaryotic or eukaryotic cells via any convenient transformation or transfection techniques. DNA of another source (e.g. PCR-generated double stranded DNA product, or synthesized double stranded or single stranded oligonucleotides) may be used to engineer the yeast by integration into the genome. Any single transformation event may include one or several nucleic acids (vectors, double stranded or single stranded DNA fragments) to genetically modify the host cell.

### UTILITY

The engineered host cells and methods of the invention, e.g., as described above, find use in a variety of applications. Applications of interest include, but are not limited to: research applications and therapeutic applications. Methods of the invention find use in a variety of different applications including any convenient application where the production of noscapinoid products.

The subject engineered host cells and methods find use in a variety of therapeutic applications. Therapeutic applications of interest include those applications in which the preparation of pharmaceutical products that include noscapinoid products. The engineered host cells described herein produce noscapinoid products. The subject host cells may be utilized to produce noscapinoid products of interest from simple and inexpensive starting materials that may find use in the production of BIAs of interest, including canadine, and noscapinoid end products. As such, the subject host cells find use in the supply of therapeutically active noscapinoid products.

In some instances, the engineered host cells and methods find use in the production of commercial scale amounts of noscapinoid products thereof where chemical synthesis of these compounds is low yielding and not a viable means for large-scale production. In certain cases, the host cells and methods are utilized in a fermentation facility that would include bioreactors (fermenters) of e.g., 5,000-200,000 liter capacity allowing for rapid production of noscapinoid products for therapeutic products. Such applications may include the industrial-scale production of noscapinoid products from fermentable carbon sources such as cellulose, starch, and free sugars.

The subject engineered host cells and methods find use in a variety of research applications. The subject host cells and methods may be used to analyze the effects of a variety of enzymes on the biosynthetic pathways of a variety of noscapinoid products. In addition, the engineered host cells may be engineered to produce noscapinoid products that find use in testing for bioactivity of interest in as yet unproven therapeutic functions. In some cases, the engineering of host cells to include a variety of heterologous coding sequences that encode for a variety of enzymes elucidates the high yielding biosynthetic pathways towards noscapinoid products. In certain cases, research applications include the production of noscapinoid products for therapeutic molecules of interest that may then be further chemically modified or derivatized to desired products or for screening for increased therapeutic activities of interest. In some instances, host cell strains are used to screen for enzyme activities that are of interest in such pathways, which may lead to enzyme discovery via conversion of metabolites produced in these strains. The subject host cells and methods may be used to as a production platform for plant specialized metabolites.

The subject engineered host cells and methods may be used as a production platform for plant specialized metabolites. The subject host cells and methods may be used as a platform for drug library development as well as plant enzyme discovery. For example, the subject engineered host cells and methods may find use in the development of natural product based drug libraries by taking yeast strains producing interesting scaffold molecules, such as protopine, and further functionalizing the compound structure through combinatorial biosynthesis or by chemical means. By producing drug libraries in this way, any potential drug hits are already associated with a production host that is amenable to large-scale culture and production. As another example, these subject engineered host cells and methods may find use in plant enzyme discovery. The subject host cells provide a clean background of defined metabolites to express plant EST libraries to identify new enzyme activities. The subject host cells and methods provide expression methods and culture conditions for the functional expression and increased activity of plant enzymes in yeast.

### KITS AND SYSTEMS

Also disclosed are kits and systems, where the kits and systems may include one or more components employed in methods of the invention, e.g., engineered host cells, starting compounds, heterologous coding sequences, vectors, culture medium, etc., as described herein. The subject kit may include an engineered host cell (e.g., as described herein), and one or more components selected from the following: starting compounds, a heterologous coding sequence and/or a vector including the same, vectors, growth feedstock, components suitable for use in expression systems (e.g., cells, cloning vectors, multiple cloning sites (MCS), bi-directional promoters, an internal ribosome entry site (IRES), etc.), and a culture medium.

Any of the components described herein may be provided in the kits, e.g., host cells including one or more modifications, starting compounds, culture medium, etc. A variety of components suitable for use in making and using heterologous coding sequences, cloning vectors and expression systems may find use in the subject kits. Kits may also include tubes, buffers, etc., and instructions for use. The various reagent components of the kits may be present in separate containers, or some or all of them may be pre-combined into a reagent mixture in a single container, as desired.

Also disclosed are systems for producing noscapinoid products, where the systems may include engineered host cells including one or more modifications (e.g., as described herein), starting compounds, culture medium, a fermenter and fermentation equipment, e.g., an apparatus suitable for maintaining growth conditions for the host cells, sampling and monitoring equipment and components, and the like. A variety of components suitable for use in large scale fermentation of yeast cells may find use in the subject systems.

In some cases, the system includes components for the large scale fermentation of engineered host cells, and the monitoring and purification of noscapinoid products produced by the fermented host cells. In certain embodiments, one or more starting compounds (e.g., as described herein) are added to the system, under conditions by which the engineered host cells in the fermenter produce one or more desired noscapinoid products. In some instances, the host cells produce a noscapinoid product (e.g., as described herein).

In some cases, the system includes processes for monitoring and or analyzing one or more noscapinoid product compounds produced by the subject host cells. For example, a LC-MS analysis system as described herein, a chromatography system, or any convenient system where the sample may be analyzed and compared to a standard, e.g., as described herein. The fermentation medium may be monitored at any convenient times before and during fermentation by sampling and analysis. When the conversion of starting compounds to noscapinoid products is complete, the fermentation may be halted and purification of the noscapinoid products may be done. As such, in some cases, the subject system includes a purification component suitable for purifying the noscapinoid products from the host cell medium into which it is produced. The purification component may include any convenient means that may be used to purify the noscapinoid products produced by fermentation, including but not limited to, silica chromatography, reverse-phase chromatography, ion exchange chromatography, HIC chromatography, size exclusion chromatography, liquid extraction, and pH extraction methods. In some cases, the subject system provides for the production and isolation of noscapinoid fermentation products of interest following the input of one or more starting compounds to the system.

The following examples are put forth so as to provide those of ordinary skill in the art with a complete disclosure and description of how to make and use the invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.), but some experimental errors and deviations should be accounted for. Unless indicated otherwise, parts are parts by weight, molecular weight is weight average molecular weight, temperature is in degrees Centigrade, and pressure is at or near atmospheric.

### EXPERIMENTAL

### Engineered yeast Saccharomyces cerevisiae for the production of noscapine and synthetic intermediates, and various noscapinoids.

The yeast strains are capable of performing transformations to produce noscapine and derivatives. Examples of the compounds these strains can produce are N-methylcanadine, 1-hydroxy-N-methylcanadine, noscapine, narcotoline, narcotinehemiacetal, 4'-O-desmethyl-3-O-acetylpapaveroxine, 3-O-acetylpapaveroxine, narcotolinehemiacetal, N-methylophiocarpine, 1, 13-dihydroxy-N-methylcanadine, 1-hydroxy-13-O-acetyl-N-methylcanadine, narcotolinogendial, 1-hydroxycanadine, and narcotolinal, among which 1-hydroxylcanadine, 4'-O-desmethyl-3-O-acetylpapaveroxine, narcotolinehemiacetal, narcotolinehemiacetal, 1, 13-dihydroxy-N-methylcanadine, 1-hydroxy-13-O-acetyl-N-methylcanadine, narcotolinogendial, narcotolinal have never been identified before. In addition, the yeast cells are engineered by correcting the biosynthetic pathway of noscapine, characterizing enzymatic function, and demonstrating heterologous pathway reconstitution to produce an efficient and accurate approach towards plant natural product biosynthesis.

### Example 1: Yeast strains synthesizing noscapine, synthetic intermediates and derivatives from canadine

Yeast strains were developed that produce protoberberine and phthalideisoquinoline alkaloids, such as 1-hydroxy-N-methylcanadine, noscapine, narcotoline, narcotinehemiacetal, 4'-O-desmethyl-3-O-acetylpapaveroxine, 3-O-acetylpapaveroxine, narcotolinehemiacetal, N-methylophiocarpine, 1, 13-dihydroxy-N-methylcanadine, 1-hydroxy-13-O-acetyl-N-methylcanadine, narcotolinogendial, narcotolinal, 1-hydroxycanadine.

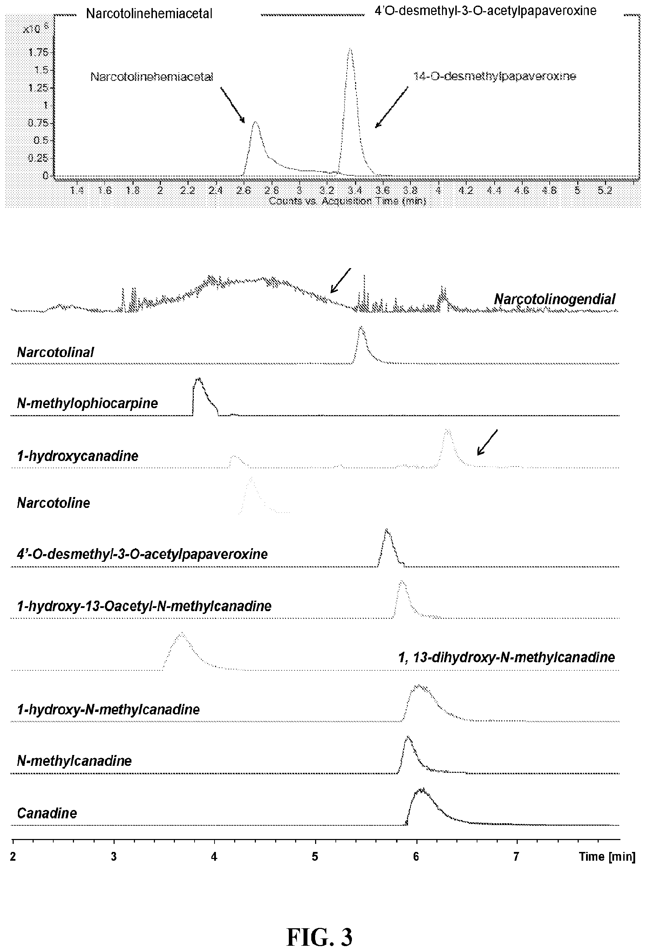

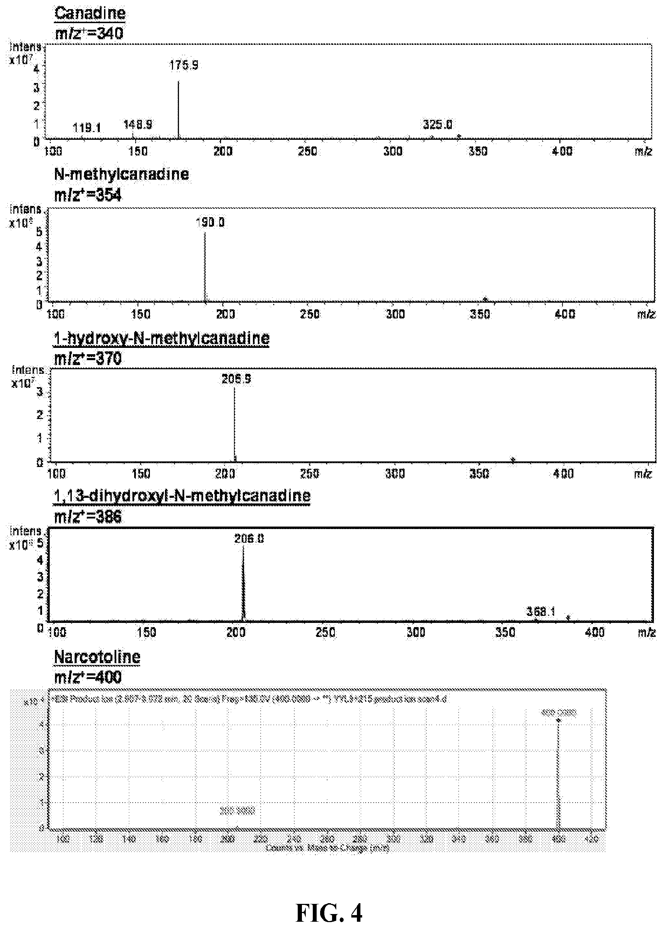

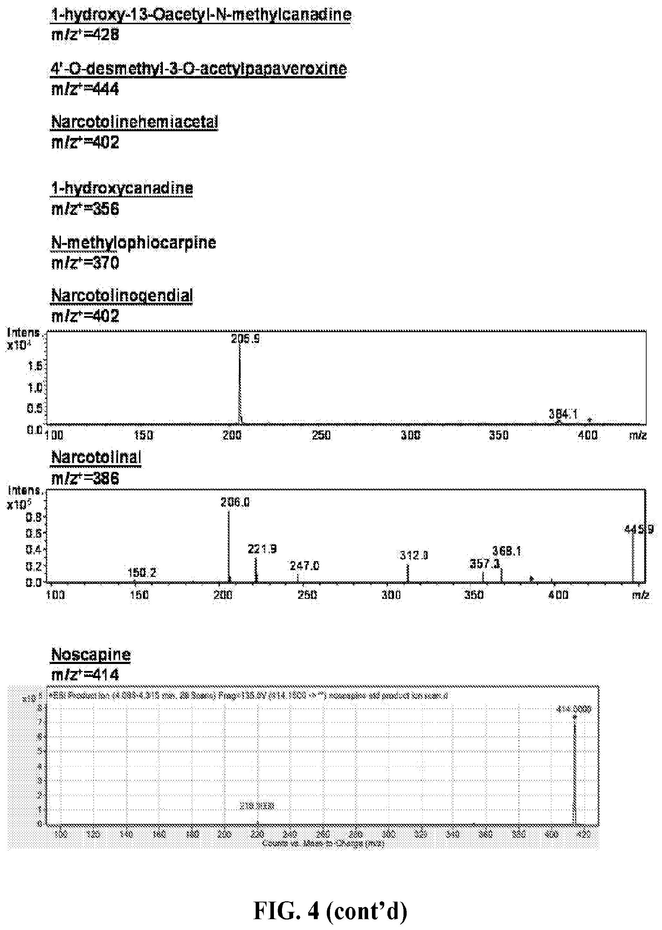

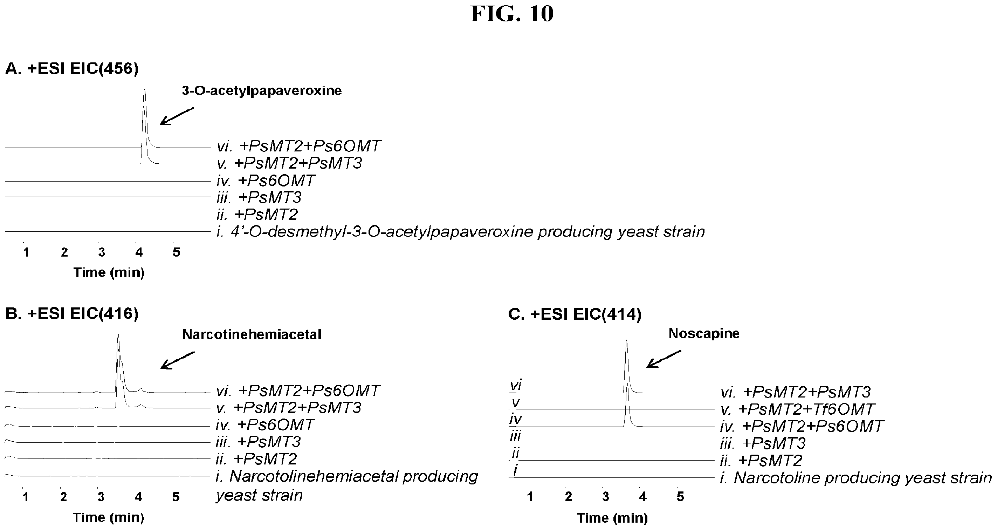

**FIGs. 2A** **and** **2B** depict the synthetic pathway from canadine to noscapine. This pathway includes the enzyme activity of CYP82X1, CYP82X2, PsAT1, CYP82Y1, PsCXE1, PsSDR1, N4'OMT. The noscapine biosynthetic pathway was engineered in yeast to produce various noscapine synthetic intermediates and derivatives.
1.1) To produce N-methylcanadine from canadine, tetrahydroprotoberberine N-methyltransferase (TNMT) from *P*. *somniferum* (PsTNMT) or *E. californica* (EcTNMT) is expressed from a low-copy construct (e.g., low-copy plasmid or chromosomally integrated) in a yeast strain with ATR1 chromosomally integrated **(****FIGs. 3** and **4****).** Other possible variants of each of the enzymes are included in **Table 1.**
1.2) To produce 1-hydroxy-N-methylcanadine from canadine, CYP82Y1 from *P*. *somniferum* is expressed on a low-copy plasmid in an N-methylcanadine producing strain described previously **(****FIGs. 3** and **4****).** Other possible variants of each of the enzymes are included in **Table 1.**
1.3) To produce 1, 13-dihydroxy-N-methylcanadine, CYP82X2 from *P*. *somniferum* is expressed from a low-copy construct (e.g., low-copy plasmid or chromosomally integrated) in a 1-hydroxy-N-methylcanadine producing strain described previously **(****FIGs. 3** and **4****).** Other possible variants of each of the enzymes are included in **Table 1.**
1.4) To produce 1-hydroxy-13-O-acetyl-N-methylcanadine, PsAT1 from P. *somniferum* is expressed from a low-copy construct (e.g., low-copy plasmid, YAC or chromosomally integrated) in a 1, 13-dihydroxy-N-methylcanadine producing strain described previously **(****FIGs. 3** and **4****).** Other possible variants of each of the enzymes are included in **Table 1.**
1.5) To produce 4'-O-desmethyl-3-O-acetylpapaveroxine, CYP82X1 from P. *somniferum* is expressed from a low-copy construct (e.g., low-copy plasmid or YAC) in a 1-hydroxy-13-O-acetyl-N-methylcanadine producing strain described previously (**FIGs. 3** and **4**). Other possible variants of each of the enzymes are included in **Table 1.**
1.6) To produce narcotolinehemiacetal, PsCXE1 from *P*. *somniferum* is expressed from a low-copy construct (e.g., low-copy plasmid or YAC) in a 4'-O-desmethyl-3-O-acetylpapaveroxine producing strain described previously **(****FIGs. 3** and **4****).** Other possible variants of each of the enzymes are included in **Table 1.**
1.7) To produce narcotoline, PsSDR1 from *P*. *somniferum* is expressed from a low-copy construct (e.g., low-copy plasmid or YAC) in a narcotolinehemiacetal producing strain described previously **(****FIGs. 3** and **4****).** Other possible variants of each of the enzymes are included in **Table 1.**
1.8) To produce noscapine, PsMT3 or Ps6OMT and PsMT2 from *P*. *somniferum* are co-expressed from low-copy constructs (e.g., low-copy plasmid or YAC or chromosomal integration) in a narcotoline producing strain described previously **(****FIG. 10****)**. Other possible variants of each of the enzymes are included in **Table 1.** Tf6OMT does not convert narcotoline to noscapine.
1.9) To produce 1-hydroxycanadine, CYP82Y1 from *P*. *somniferum* is expressed from a low-copy plasmid in a yeast strain with ATR1 chromosomally integrated **(****FIGs. 3** and **4**). Other possible variants of each of the enzymes are included in **Table 1.**
1.10) To produce N-methyl-ophiocarpine, CYP82X2 from P. *somniferum* is expressed from a low-copy construct (e.g., low-copy plasmid or YAC) in an N-methylcanadine producing strain described previously **(****FIGs. 3** and **4****).** Other possible variants of each of the enzymes are included in **Table 1.**
1.11) To produce narcotolinal, CYP82X1 from *P*. *somniferum* is expressed from a low-copy construct (e.g., low-copy plasmid or YAC) in a 1-hydroxyl-N-methylcanadine producing strain described previously **(****FIGs. 3** and **4****).** Other possible variants of each of the enzymes are included in **Table 1.**
1.12) To produce narcotolinogendial, CYP82X1 from *P*. *somniferum* is expressed from a low-copy construct (e.g., low-copy plasmid or YAC) in a 1, 13-dihydroxy-N-methylcanadine producing strain described previously **(****FIGs. 3** and **4****).** Other possible variants of each of the enzymes are included in **Table 1.**
1.13) To produce 3-O-acetylpapaveroxine, PsMT3 or Ps6OMTand PsMT2 from P. *somniferum* are co-expressed from low-copy constructs (e.g., low-copy plasmid or YAC or chromosomal integration) in a 4'-O-desmethyl-3-O-acetylpapaveroxine producing strain described previously (**FIG. 10**). Other possible variants of each of the enzymes are included in **Table I.**
1.14) To produce narcotinehemiacetal, PsMT3 or Ps6OMT and PsMT2 from P. *somniferum* are co-expressed from low-copy constructs (e.g., low-copy plasmid or YAC or chromosomal integration) in a narcotolinehemiacetal producing strain described previously (**FIG. 10**). Other possible variants of each of the enzymes are included in **Table 1.**
1.15) To produce more noscapine analogues, a certain enzyme or certain set of enzymes are removed from the noscapine producing strain. One example is that to synthesize hydrastine, PsMT2, PsMT3 or Ps6OMT and CYP82Y1 are excluded from the noscapine producing strain described previously. In addition, different substrates other than canadine are fed to the noscapine producing strain described previously to afford more noscapine analogues. The substrates include, but are not limited to, (S)-scoulerine, (S)-tetrahydroberberrubine, (S)-stylopine, (S)-cheilanthifoline, and (S)-tetrahydropalmatine.
1.16) To produce more noscapinoids, tailoring enzymes are co-expressed with the previously described strains. Examples of enzymes introduced to the noscapine (or intermediate(s))-producing strains include, but not limited to, P450s, halogenase, glycosylase, methyltransferase, prenyltransferase. One example is that the mammalian liver P450 CYP2D6 can break the methylenedioxyl bridge of canadine to afford 9,10-dimethoxy-6,8,13,13a-tetrahydro-5H-isoquinolino[3,2-a]isoquinoline-2,3-diol.

### Example 2: Strategies for optimizing biosynthesis of noscapine, synthetic intermediates and derivatives in yeast

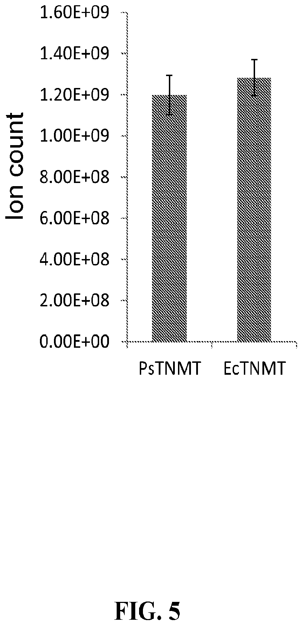

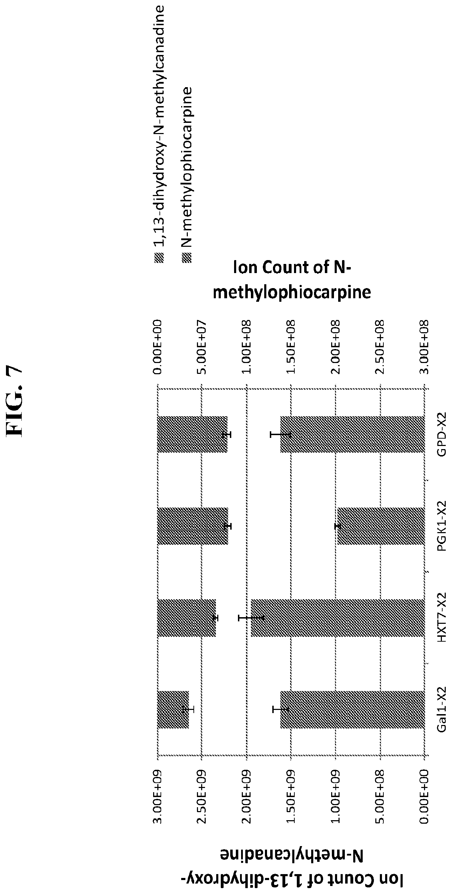

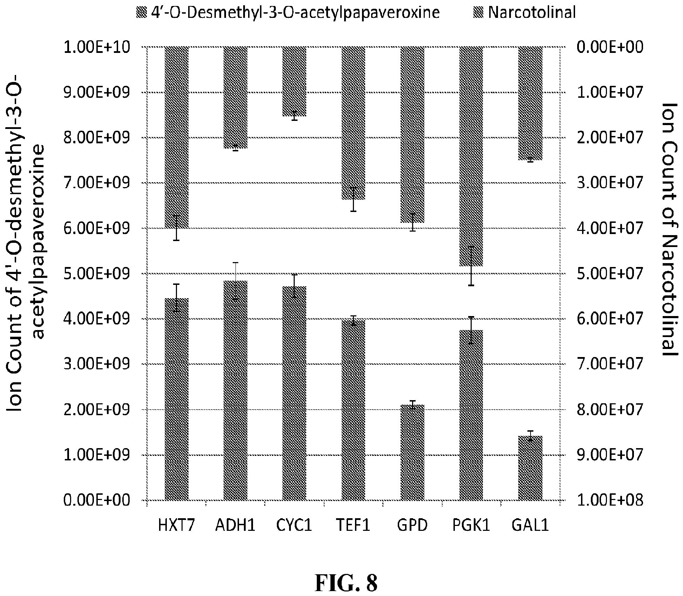

Tools and methods to optimize the production of protoberberine and phthalideisoquinoline alkaloids within the context of the engineered yeast strain were developed. The enzymatic activity of the three P450s, i.e., CYP82Y1, CYP82X1, and CYP82X2, were optimized towards both noscapine and intermediates. The organelle routing toolkit is utilized to relocate and optimize the interactions between the following types of enzymes, including P450s, methyltransferases, acetyltransferase, short-chain dehydrogenase, and carboxylesterase, in order to get optimal production of the target noscapine or noscapinoids in the engineered yeast strains.
2.1) To produce more N-methylcanadine, variants of TNMT enzymes from different species are expressed in yeast. **FIG. 5** depicts the measurement of N-methylcanadine production from different TNMT variants expressed in *S*. *cerevisiae.* The data demonstrate that certain TNMT enzyme variants produce higher levels of N-methylcanadine than others.
2.2) To produce more 1-hydroxy-N-methylcanadine from canadine, the N-terminus of CYP82Y1 was engineered to facilitate the correct folding of the enzyme. **FIG. 6** depicts the measurement of 1-hydroxy-N-methycanadine production using different CYP82Y1 N-terminus mutants, under different temperatures (25°C and 30°C). When cultured at 30°C and regulated by the same promoter (GAL1 promoter), the activity of CYP82Y1 is improved through swapping the N-terminus tag with that of MSH (CYP82Y1A). When cultured at 25°C and regulated by the GPD promoter, the activities of CYP82Y1 and CYP82Y1A are similar. The data implies that the N-terminus tag of MSH facilitates the formation of soluble CYP82Y1 in *S*. *cerevisiae.*
2.3) To produce more 1-hydroxy-N-methylcanadine from canadine, optimal transcriptional regulation of CYP82Y1 is determined through testing different types of promoters upstream of the CYP82Y1 gene. **FIG. 6** depicts the measurement of 1-hydroxy-N-methycanadine production using CYP82Y1 regulated by different promoters under different temperatures (25°C and 30°C). Under higher culture temperature, the activity of CYP82Y1 is highest when regulated by the late stage HXT7 (strong) and ADH1 (relatively weaker than HXT7p) promoter. The strong promoters activated in the early growth stage, such as the PGK1, TPI1, PYK1, TEF1 promoters, are less effective when cultured at 30°C. In comparison, when the engineered yeast strain is cultured at 25°C, the activity of CYP82Y1 regulated by the HXT7 and strong promoters are approximately the same level. And in comparison, the production of 14-hydroxyl-N-methylcanadine is lower when CYP82Y1 is regulated by ADH1 and the weak CYC1 promoter.
2.4) To produce more 1-hydroxycanadine, all the CYP82Y1 mutants and various promoter-CYP82Y1 combinations are examined in *S*. *cerevisiae* at 25°C. The HXT7 promoter upstream of CYP82Y1A is combined for the synthesis of 1-hydroxycanadine. For the biosynthesis of 1-hydroxy-N-methylcanadine, CYP82Y1 and CYP82Y1A exhibit similar efficiency at 25°C when regulated by HXT7 promoter; in contrast, CYP82Y1A is twice as efficient as CYP82Y1 towards the biosynthesis of 1-hydroxycanadine (**FIG. 6**).
2.5) To produce more 1, 13-dihydroxy-N-methylcanadine, optimal transcriptional regulation of CYP82X2 is determined through testing different types of promoters upstream of the CYP82X2 gene. **FIG. 7** depicts the measurement of 1, 13-dihydroxy-N-methylcanadine production using CYP82X2 regulated by different promoters at 25°C. Similar to CYP82Y1, the HXT7 promoter results in a high level of 1, 13-dihydroxy-N-methylcanadineproduction. Furthermore, additional copies of CYP82X2 are expressed in the engineered yeast strain. Since CYP82X2 is efficient in converting 1-hydroxy-N-methylcanadine to 1, 13-dihydroxy-N-methylcanadine when the amount of 1-hydroxy-N-methylcanadine is limited, it is proposed that the conversion rate of CYP82X2 is not as fast as the export rate of 1-hydroxy-N-methylcandine out of the yeast cells. According, TNMT is relocated in the engineered yeast strain to delay the synthesis of 1-hydroxy-N-methylcanadine. Also, CYP82X2 and CYP82Y1 are further engineered to be located on the same side of endoplasmic reticulum, to increase the access of CYP82X2 to 1-hydroxy-N-methylcanadine.
2.6) To produce more N-methyl-ophiocarpine, various promoter-CYP82X2 combinations are also tested in the narcotolinogendial producing strain. **FIG. 7** depicts the measurement of N-methyl-ophiocarpine production using CYP82X2 regulated by different promoters at 25°C. Different from when this enzyme is functioning on its natural substrate 1-hydroxy-N-methylcanadine, CYP82X2 exhibits higher activity towards the synthesis of N-methyl-ophiocarpine when expressed from the PGK1 and GPD promoters.
2.7) To produce more 4'-O-desmethyl-3-O-acetylpapaveroxine, optimal transcriptional regulation of CYP82X1 is determined through testing different types of promoters upstream of the CYP82X1 gene. The production of 4'-O-desmethyl-3-O-acetylpapaveroxine is measured in different yeast strains expressing CYP82X1 downstream of different promoters at 25°C. The HXT7, ADH1, and CYC1 promoters result in a high synthesis level of 4'-O-desmethyl-3-O-acetylpapaveroxine (**FIG. 8**).
2.8) To produce more narcotolinal and narcotolinogendial, optimal transcriptional regulation of CYP82X1 is determined through testing different types of promoters upstream of the CYP82X1 gene. The narcotolinal or narcotolinogendial production is measured and compared among each pair. For the synthesis of narcotolinal, the activity of CYP82X1 is better when CYP82X1 is regulated by PGK1 (**FIG. 8**).
2.9) To decrease the expression stress of yeast for the expression of three heterologous plant P450s in the noscapine producing strain, selective plant chaperones are expressed for better yeast growth, higher level of soluble plant P450s and higher production of noscapine. The plant chaperones utilized include, binding immunoglobulin protein (BiP), DnaJ protein, glucose regulated protein (GRP) 94, binding protein (BiP), protein disulphide isomerase (PDI), cyclophilin, and calnexin.
2.10) To produce more noscapine, additional copies of downstream enzymes (including CYP82X2, CYP82Y1, CYP82X1, PsCXE1, PsSDR1, PsMT2, and PsMT3 or Ps6OMT) are expressed in the engineered yeast strains. Also, the noscapine production level in the engineered yeast strain is enhanced by optimizing the localization of the noscapine biosynthetic enzymes in yeast. The optimal localization mode of the plant biosynthetic enzymes mimics their natural context. The rate-limiting steps are re-localized to a different organelle, or in vicinity of enzymes of the previous steps. And co-localization of various combinations and numbers of biosynthetic enzymes are examined.
2.11) To produce more noscapine intermediates, the efficiency of variants of downstream biosynthetic enzymes from other plant species, such as *Hydrastis canadensis, Dicentra cucullaria,* and *Adlumia fungosa,* are compared in the engineered yeast strain with that from *P*. *somniferum.* In addition, some downstream enzymes are also engineered to exhibit more flexible substrate specificity so as to be able to efficiently convert unnatural substrates into the final noscapine analogues.

### Example 3: Yeast strains synthesizing noscapine from norlaudanosoline

The yeast are engineered to include six additional enzymatic steps, specifically those catalyzed by the enzymes Ps6OMT, Ps4'OMT, PsCNMT, PsBBE, S9OMT, and CYP719A, within an engineered yeast strain to produce noscapine from canadine. The engineered yeast strain for the biosynthesis of plant natural products includes up to 15 heterologous enzymatic steps engineered and introduced into a yeast strain (**FIG. 9**). In examples, an additional 8 enzymes in the yeast may be engineered to produce reticuline from norlaudanosoline. In additional examples, an additional 6 enzymes in the yeast may be engineered to produce noscapnine from canadine.

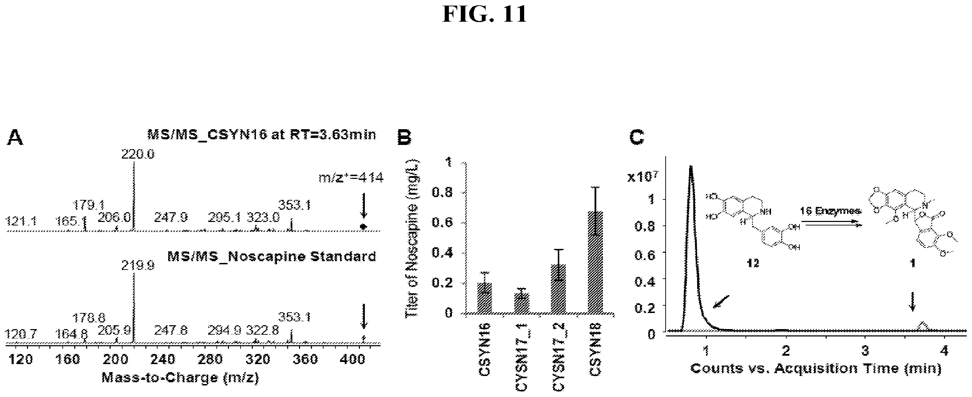

To produce noscapine from norlaudanosoline, TNMT, PsAT1, CYP82X1, PsCXE1, PsSDR1, PsMT2, PsMT3 are expressed from a YAC, CYP82Y1 and CYP82X1 are expressed from a low-copy plasmid, S9OMT is expressed from a high-copy plasmid in the canadine producing strain with Ps6OMT, Ps4'OMT, PsCNMT, PsBBE, AtATR1, and CYP719A chromosomally integrated. In addition, Ps6OMT, Ps4'OMT, PsCNMT, PsBBE, AtATR1, CYP719A, TNMT, S9OMT, CYP82Y1, CYP82X1, CYP82X2, PsCXE1, PsSDR1, PsMT2, and PsAT1 are chromosomally integrated. Other possible variants of each of the enzymes are included in **Table I**. Extra copies of CYP82X1 and/or S9OMT are expressed from a low-copy plasmid for higher production of noscapine (**FIG. 11**).

### Example 4: Yeast strains synthesizing noscapine de novo

The yeast are engineered to include thirteen additional enzymatic steps, specifically those catalyzed by the enzymes RnPTPS, RnSepR, RnPCD, RnQDHPR, RnDHFR, RnTyrH, CjNCS, PpDODC, EcCYP80B1, ARO4(Q166K), ARO7(T226I), ARO10, and TKL1, within an engineered yeast strain to produce noscapine from norlaudanosoline. The engineered yeast strain for the biosynthesis of plant natural products includes up to 28 heterologous enzymatic steps engineered and introduced into a yeast strain (**FIG. 9**).

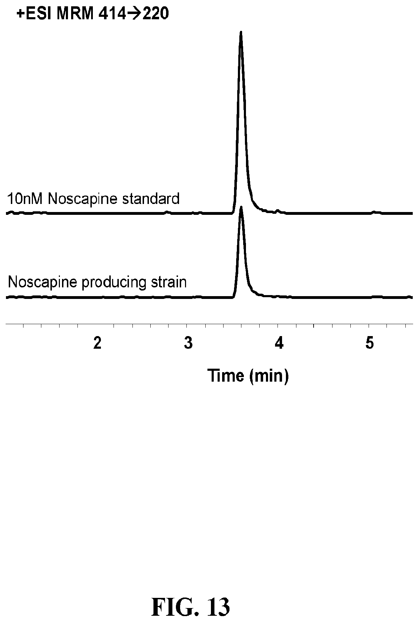

To produce noscapine *de novo,* PsBBE, PsMT1 (PsS9OMT), CjCAS, TNMT, CYP82Y1, CYP82X2, PSAT1, CYP82X1, PsCXE1, PsSDR1, PsMT2, and PsMT3 are chromosomally integrated into three different locus (*leu2, trp1,* and *his3*) of reticuline producing yeast strain with or without zwf1 deletion. And RnPTPS, RnSepR, RnPCD, RnQDHPR, RnDHFR, RnTyrH, CjNCS, PpDODC, EcCYP80B1, PsCPR, Ps6OMT, Ps4'OMT, PsCNMT, ARO4(Q166K), ARO7(T226I), ARO10, and TKL1 are chromosomally integrated into the yeast strain for the de novo synthesis of reticuline, with extra copy of RnTyrH, Ps4'OMT, CjNCS chromosomally integrated into either zwf1 locus or YPL250C locus to enhance the production of reticuline. Other possible variants of each of the enzymes are included in Table I. **(****FIG. 13****)**.

### Example 5: Yeast strains synthesizing noscapinoids de novo

The noscapine producing yeast are cultured in defined medium with tyrosine replaced with one or one set of tyrosine analogs so as to synthesize functionalized derivatives of benzylisoquinoline, protoberberine, secoberberine, and phthalideisoquinoline alkaloids. A functionalized derivative as used herein refers to a compound having a structural feature that distinguishes it from a reference compound, wherein the distinguishing feature was present in the starting material used in the synthesis of the compound. For example, 3-chloro-reticuline can be produced from α-chloro-L-tyrosine and is considered herein to be a functionalized derivative of reticuline, as the chloride distinguishing the two compounds was a feature of the starting material used and not introduced by a step in the synthesis. Further examples include 6-hydroxy-canadine, a functionalized derivative of canadine that can be produced by a host cell from α-hydroxy-L-tyrosine, and 7-nitro-narcotoline, a functionalized derivative of narcotoline produced from 3-nitro-L-tyrosine. Similarly, functionalized derivatives of benzylisoquinoline, protoberberine, secoberberine, and phthalideisoquinoline alkaloids may be produced from starting materials such as analogs of tyrosine. In additional examples, one or more set of genes are taken out to accumulate certain groups of functionalized derivatives.

To produce noscapinoids *de novo,* a yeast strain is engineered to express PsBBE, PsMT1 (PsS9OMT), CjCAS, TNMT, CYP82Y1, CYP82X2, PSAT1, CYP82X1, PsCXE1, PsSDR1, PsMT2, PsMT3, RnPTPS, RnSepR, RnPCD, RnQDHPR, RnDHFR, RnTyrH, CjNCS, PpDODC, EcCYP80B1, PsCPR, Ps6OMT, Ps4'OMT, PsCNMT, ARO4(Q166K), ARO7(T226I), ARO10, and TKL1, with an extra copy of RnTyrH, Ps4'OMT, and CjNCS chromosomally integrated into either zwf1 locus or YPL250C locus, and an extra copy of GAPDH, CYP82X2, TRP1, and PsMT1 chromosomally integrated into *ura3* locus. The engineered noscapine producing strain was cultured in defined medium without tyrosine, but with one or a set of tyrosine analogs, which include but are not limited to α-substituted L-tyrosine (e.g. α-methyl L-tyrosine, scheme 1) and 3-substituted L-tyrosine (e.g. 3-Nitro-L-tyrosine, scheme 2). Functionalized derivatives of benzylisoquinoline, protoberberine, secoberberine, and phthalideisoquinoline alkaloids are accumulated along the noscapine biosynthetic pathway. In some cases, to accumulate more functionalized derivatives of benzylisoquinoline, protoberberine, or secoberberine alkaloids, certain or certain sets of downstream enzymes are removed from the noscapine producing strain.

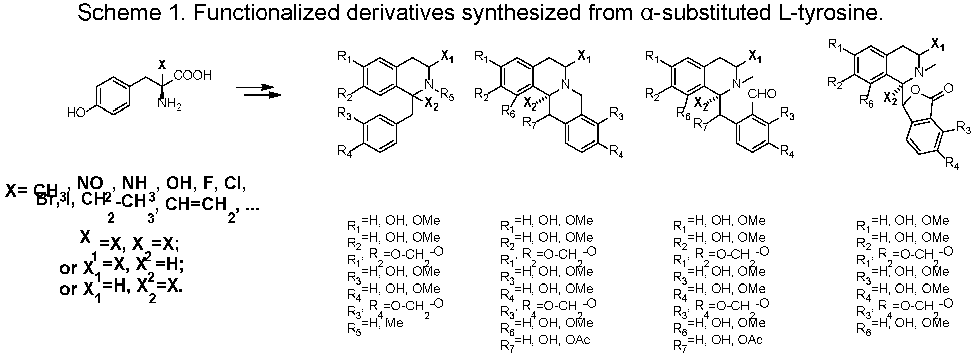

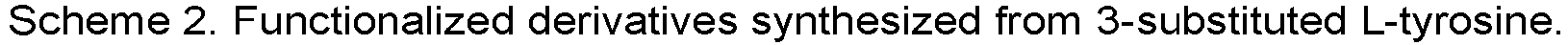

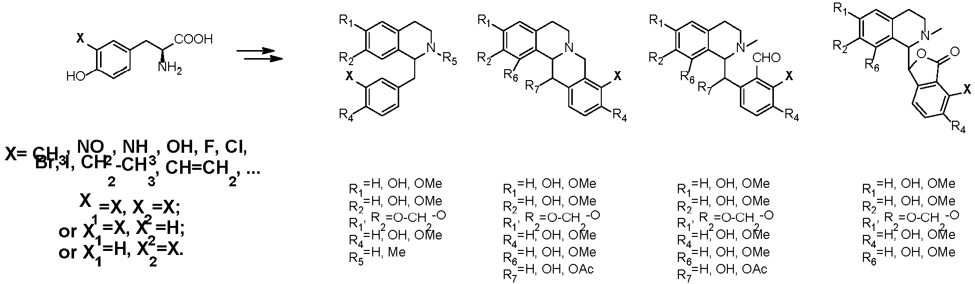

### Description of the figures

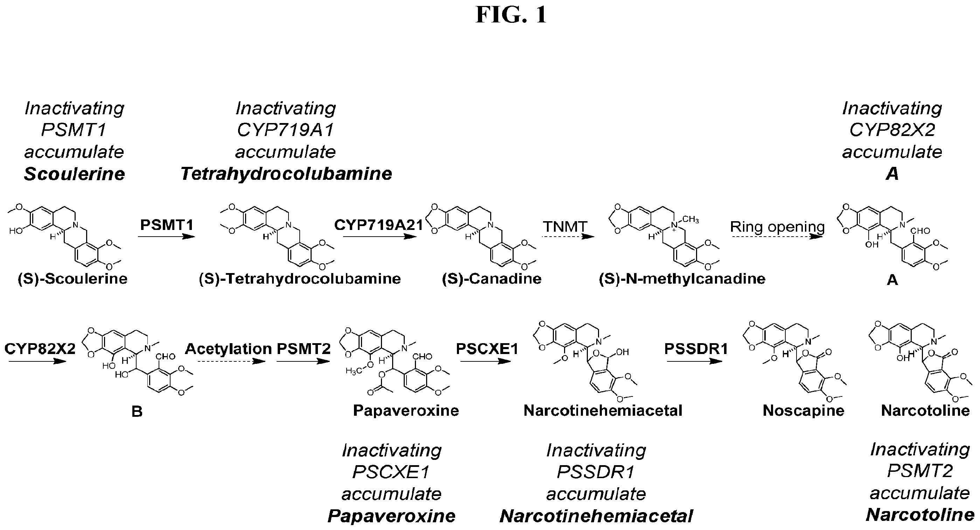

**FIG. 1** illustrates a biosynthetic scheme for conversion of canadine to noscapine based on biochemical identification in plants, in accordance with embodiments of the invention. The dashed arrows indicate the enzymatic steps not verified. The solid arrows indicate enzymatic steps that are verified either through heterologous expression in yeast (PsMT1, CYP82Y1, PsSDR1), or through virus induced gene silencing (VIGS) in *P*. *somniferum* (PsMT1, CYP719A21, CYP82X2, PsMT2, PsCXE1, PsSDR1). When PsMT1, CYP719A21, CYP82X2, PsCXE1, PsSDR1, and PsMT2 are inactivated in P. *somniferum,* (S)-canadine, (S)-N-methylcanadine, narcotolinal, papaveroxine, narcotinehemiacetal, and narcotoline are accumulated accordingly.
**FIG. 2A** illustrates a biosynthetic scheme for conversion of canadine to noscapine based on biochemical characterization in *Saccharomyces cerevisiae,* in accordance with embodiments of the invention.
**FIGs. 2A** and **2B** depict the updated biosynthetic pathway for the synthesis of noscapine from canadine. **FIG. 2A** depicts the major pathway for the biosynthesis of noscapine, and **FIG. 2B** illustrates the major pathway as well as the pathway for the production of side products. As seen in **FIG. 2A**, and according to the biochemical characterization in yeast, CYP82X2 catalyzes the enzymatic step following the synthesis of 1-hydroxy-N-methylcanadine. Subsequently, PsAT1 adds the acetyl group to result in 1-hydroxy-13-O-acetyl-N-methylcanadine, followed by CYP82X1 catalyzing the C-N bond cleavage and synthesizing 4'-O-desmethyl-3-O-acetylpapaveroxine. PsCXE1 and PsSDR1 function as previously suggested and synthesize narcotoline from 4'-O-desmethyl-3-O-acetylpapaveroxine. CYP82Y1 can function on (S)-canadine to produce 1-hydroxycanadine. N-methylophiocarpine can be synthesized from N-methylcanadine from the activity of CYP82X2. Narcotolinal is synthesized from canadine in the presence of TNMT, CYP82Y1 and CYP82X1. When CYP82X1 is introduced to the 1, 13-dihydroxy-N-methylcanadine producing strain, narcotolinogendial is produced.
**FIG. 2B** illustrates another biosynthetic scheme for conversion of canadine to noscapine and production of byproducts based on biochemical characterization in *Saccharomyces cerevisiae,* in accordance with embodiments of the invention.
**FIG. 3** illustrates EIC traces from liquid chromatography-mass spectrometry (LC-MS) analysis of compounds secreted into the culture medium by engineered yeast strains, in accordance with embodiments of the invention. In particular, **FIG. 3** illustrates LC-MS traces of noscapine, intermediates and derivatives production. **FIG. 3** depicts the liquid chromatography tandem mass spectrometry (LC-MS) analysis of compounds secreted into the culture medium by the engineered yeast strains. All the assays were performed in vivo from yeast grown in the presence of 250 µM canadine (S, R racemic mixture) for 72 h. The media was separated from the cell pellets through centrifugation and analyzed by LC-MS. Positive ion electrospray ionization (ESI) mass spectra were obtained with an Agilent 6320 Ion Trap (electrospray capillary voltage -3.5 kV; heated capillary temperature 350 °C; sheath gas: nitrogen) coupled to an Agilent 1200 Series HPLC equipped with an Agilent Zorbax SB-Aq column (3.0 × 50 mm 1.8 micron) and an Agilent Zorbax SB-Aq guard column (2.1 × 12.5 mm 5 micron). The LC separation method was gradient elution from H₂O:CH₃OH 80:20 to 40:60 over 7 min, gradient elution to 100% CH₃OH over 1 min, and finally isocratic elution for 4 min with a flow rate of 0.5 mL min⁻¹. Both solvents were 0.1% acetic acid.
**FIG. 4** illustrates positive ion electrospray ionization (ESI) mass spectra MS/MS fragmentation for compounds described in **FIG. 3****,** in accordance with embodiments of the invention. Positive ion electrospray ionization (ESI) mass spectra were obtained as described for **FIG. 3****.** And the MS/MS mass spectra for a given precursor ion was obtained with a fragmentation amplitude of 1.00 V for 40 ms and helium as the background gas.
**FIG. 5** illustrates synthesis of N-methylcanadine in vivo from yeast expressing TNMT from either *P*. *somniferum* or *Eschscholzia californica* and grown in the presence of canadine, in accordance with embodiments of the invention. Assays were performed in vivo from yeast expressing TNMT from either *P*. *somniferum* or *E. californica* and grown in the presence of 250 µM canadine (S, R racemic mixture) for 72 h at 30°C. Positive ion electrospray ionization (ESI) mass spectra were obtained as described for **FIG. 3****.** Extracted ion chromatograms for the product extracted ion chromatograms for the molecular ion were plotted and manually integrated. Error bars are S.D. of three biological replicates.
**FIG. 6** illustrates results of assays in vivo from yeast grown in the presence of canadine with expression of different versions of CYP82Y1 downstream of various promoters, with or without the presence of PsTNMT, in accordance with embodiments of the invention. Assays were performed in vivo from yeast grown in the presence of 250 µM canadine (S, R racemic mixture) for 72 h. Yeast express different versions of CYP82Y1 downstream of various promoters, with or without the presence of PsTNMT. Positive ion electrospray ionization (ESI) mass spectra were obtained as described for **FIG. 3****.** Extracted ion chromatograms for the product extracted ion chromatograms for the molecular ion were plotted and manually integrated. Error bars are S.D. of three biological replicates. A depicts the production of 1-hydroxy-N-methylcanadine by different versions of CYP82Y1 (1. N-terminus of CYP82Y1 tag is swapped with the N-terminus of other ER-binding proteins; 2. The N-terminus tag of other ER-binding protein is directly tagged on to CYP82Y1) downstream of various promoters (GAL1, GPD, TPI1, TEF1, PGK1, PYK1, CYC1, ADH1, HXT7 promoter) in the yeast strain with PsTNMT chromosomally integrated under different temperatures (25°C and 30°C). B depicts the production of either 1-hydroxy-N-methylcanadine or 1-hydroxycanadine in PsTNMT integrated S. *cerevisiae* expressing CYP82Y1 or CYP82Y1A (CYP82Y1 with the N-terminus tag swapped with that of MSH) downstream of HXT7 promoter or TPI11 promoter at 25°C.
**FIG. 7** illustrates results of assays in vivo from yeast grown in the presence of canadine with expression of CYP82X2 downstream of various promoters (GAL1, GPD, PGK1, HXT7), in the presence of PsTNMT and CYP82Y1A, in accordance with embodiments of the invention. Assays were performed in vivo from yeast grown in the presence of 250 µM canadine (S, R racemic mixture) for 72 h. Yeast express CYP82X2 downstream of various promoters (GAL1, GPD, PGK1, HXT7 promoter), in the presence of PsTNMT and CYP82Y1A. Positive ion electrospray ionization (ESI) mass spectra were obtained as described for FIG. 3. Extracted ion chromatograms for the product extracted ion chromatograms for the molecular ion were plotted and manually integrated. Error bars are S.D. of three biological replicates.
**FIG. 8** illustrates results of assays in vivo from yeast grown in the presence of canadine with expression of wild type (WT) downstream of various promoters (GAL1, GPD, PGK1, CYC1, ADH1, HXT7), in the presence of PsTNMT and CYP82Y1A, with and without CYP82X2 and PsAT1, in accordance with embodiments of the invention. Assays were performed in vivo from yeast grown in the presence of 250 µM canadine (S, R racemic mixture) for 72 h. Yeast express wild type CYP82X1 downstream of GAL1, HXT7, ADH1, CYC1, GPD, TEF1 and PGK1 promoter, in the presence of PsTNMT and CYP82Y1A, with or without CYP82X2 and PsAT1. Positive ion electrospray ionization (ESI) mass spectra were obtained as described for FIG. 3. Extracted ion chromatograms for the product extracted ion chromatograms for the molecular ion were plotted and manually integrated. Error bars are S.D. of three biological replicates.
**FIG. 9** illustrates a biosynthetic scheme for conversion of norlaudanosoline to noscapine, in accordance with embodiments of the invention.
**FIG. 10** illustrates LC-MS traces of 3-O-acetyl-papaveroxine, narcotinehemiacetal and noscapine. In particular, **FIG. 10** depicts the EIC-LCMS analysis of compounds secreted into the culture medium by the engineered yeast strains using methods as described herein.
**FIG. 11** illustrates biosynthesis of noscapine from norlaudanosoline, in accordance with embodiments of the invention. A depicts the MS/MS spectra of noscapine standard (black) and medium of the noscapine-producing strain CSYN16 (red). B depicts noscapine titer analyzed from engineered strains harboring different sets of expression cassettes. CSYN16: AtATR1, Ps6OMT, Ps4'OMT, PsCNMT, PsBBE, PsS9OMT, CjCAS, CYP82Y1, CYP82X2, PsAT1, PsSDR1, PsTNMT, PsMT2, CYP82X1, PsCXE1, and PsMT3 expressed from the chromosome. CSYN17_1: AtATR1, Ps6OMT, Ps4'OMT, PsCNMT, PsBBE, TfS9OMT, CjCAS, CYP82Y1, CYP82X2, PsAT1, PsSDR1, PsMT2, PsTNMT, CYP82X1, PsCXE1, and PsMT3 expressed from the chromosome; CYP82X2 expressed from a low-copy plasmid. CSYN17_2: AtATR1, Ps6OMT, Ps4'OMT, PsCNMT, PsBBE, TfS9OMT, CjCAS, CYP82Y1, CYP82X2, PsAT1, PsSDR1, PsMT2, PsTNMT, CYP82X1, PsCXE1, and PsMT3 expressed from the chromosome; PsS9OMT expressed from a low-copy plasmid. CSYN18: AtATR1, Ps6OMT, Ps4'OMT, PsCNMT, PsBBE, TfS9OMT, CjCAS, CYP82Y1, CYP82X2, PsAT1, PsSDR1, PsMT2, PsTNMT, CYP82X1, PsCXE1, and PsMT3 expressed from the chromosome; CYP82X2, PsS9OM expressed from a low-copy plasmid. C depicts the EIC of m/z+=288 (black) and 414 (red) of noscapine-producing yeast strain CSYN18. Bars represent mean values ± 1 s.d. of five biological replicates, and the error bars represent the standard deviation of the replicates.

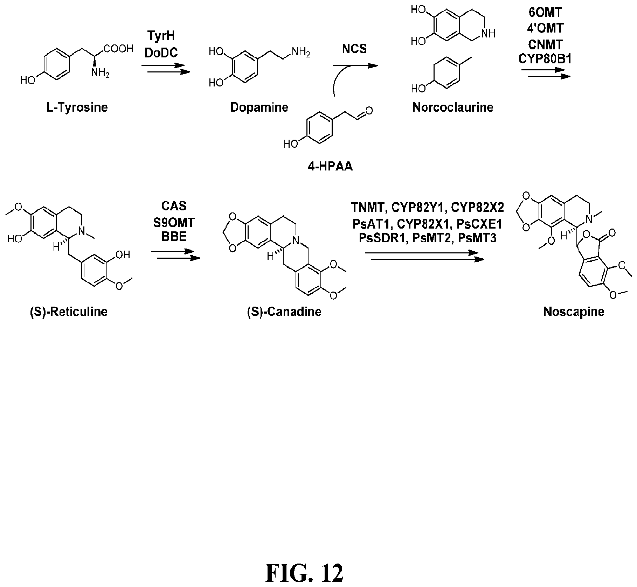

**FIG. 12** illustrates a biosynethic pathway of noscapine from tyrosine, in accordance with embodiments of the invention.
**FIG. 13** illustrates a demonstration of de novo production of noscapine in yeast, in accordance with embodiments of the invention.

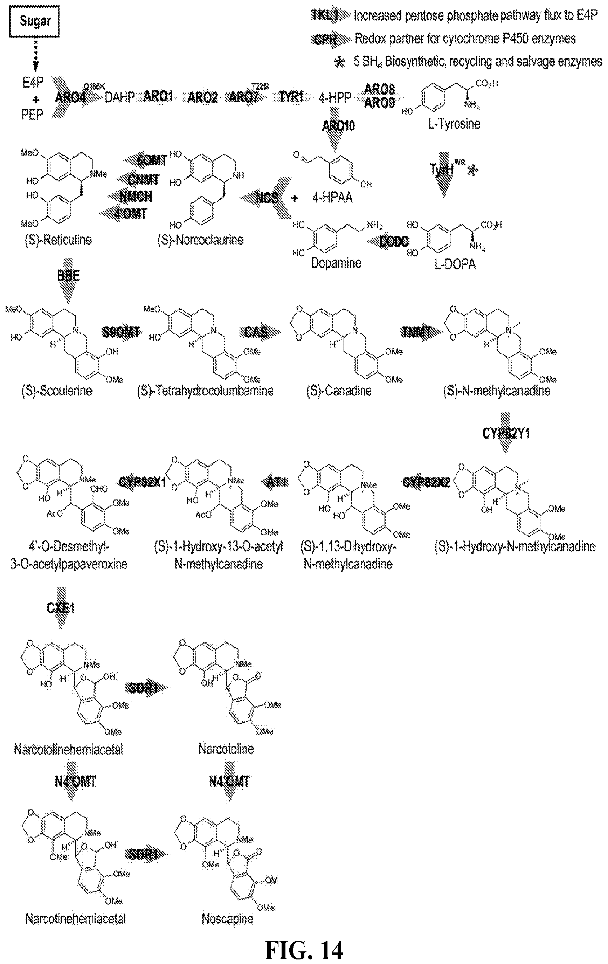

**FIG. 14** illustrates an exemplary biosynthetic schematic of de novo production of noscapinoids in yeast, in accordance with embodiments of the invention. N4'OMT represents the MT2, MT3 heterodimer.

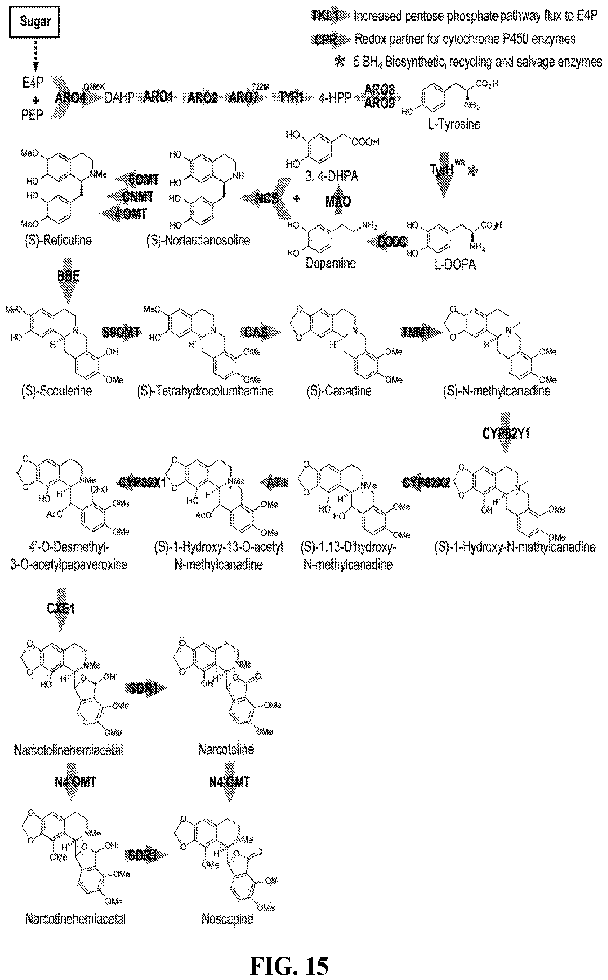

**FIG. 15** illustrates another exemplary biosynthetic schematic of de novo production of noscapinoids in yeast, in accordance with embodiments of the invention. N4'OMT represents the MT2, MT3 heterodimer.

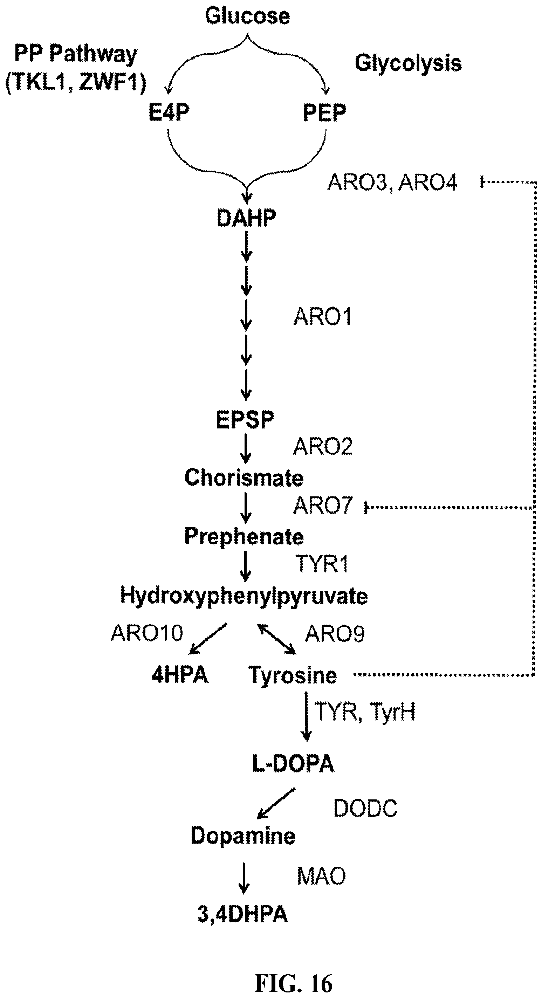

**FIG. 16** illustrates a biosynthetic scheme for conversion of glucose to 4-HPA, dopamine, and 3,4-DHPA, in accordance with embodiments of the invention.

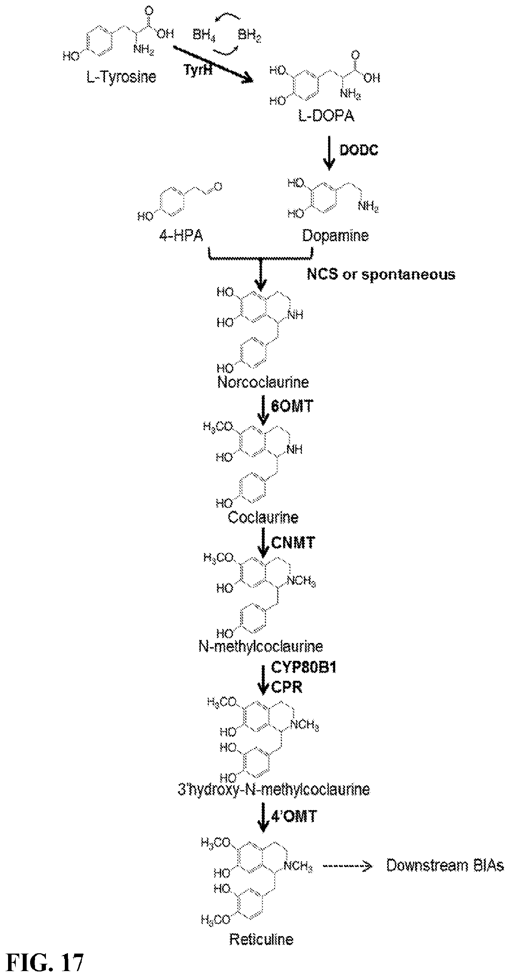

**FIG.** 17 illustrates a biosynthetic scheme for conversion of L-tyrosine to reticuline via norcoclaurine, in accordance with embodiments of the invention.

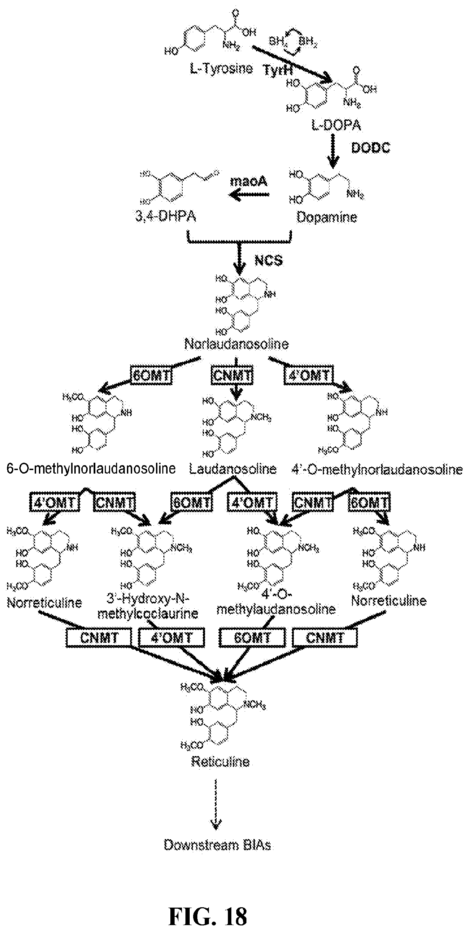

**FIG. 18** illustrates a biosynthetic scheme for conversion of L-tyrosine to reticuline via norlaudanosoline, in accordance with embodiments of the invention.

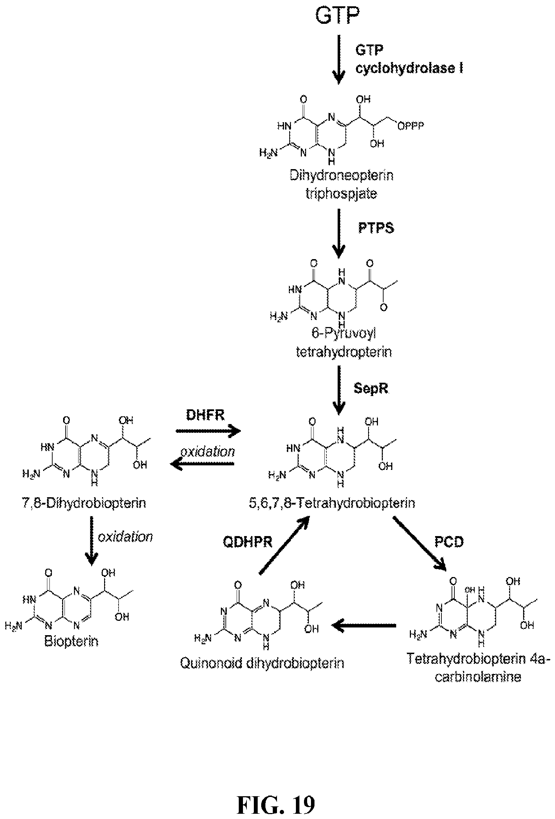

**FIG. 19** illustrates examples of synthesis, recycling, and salvage pathways of tetrahydrobiopterin, in accordance with embodiments of the invention.

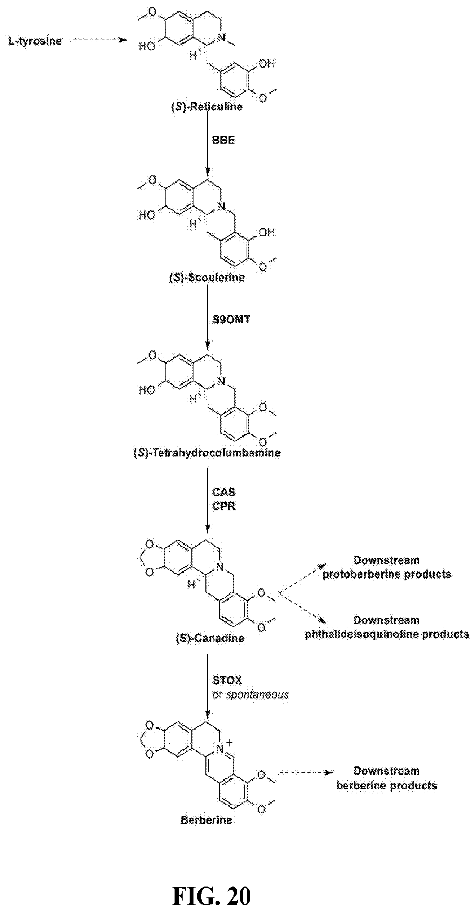

**FIG. 20** illustrates a biosynthetic scheme for conversion of L-tyrosine to protoberberine, phthalideisoquinoline, and berberine alkaloid products, in accordance with embodiments of the invention.

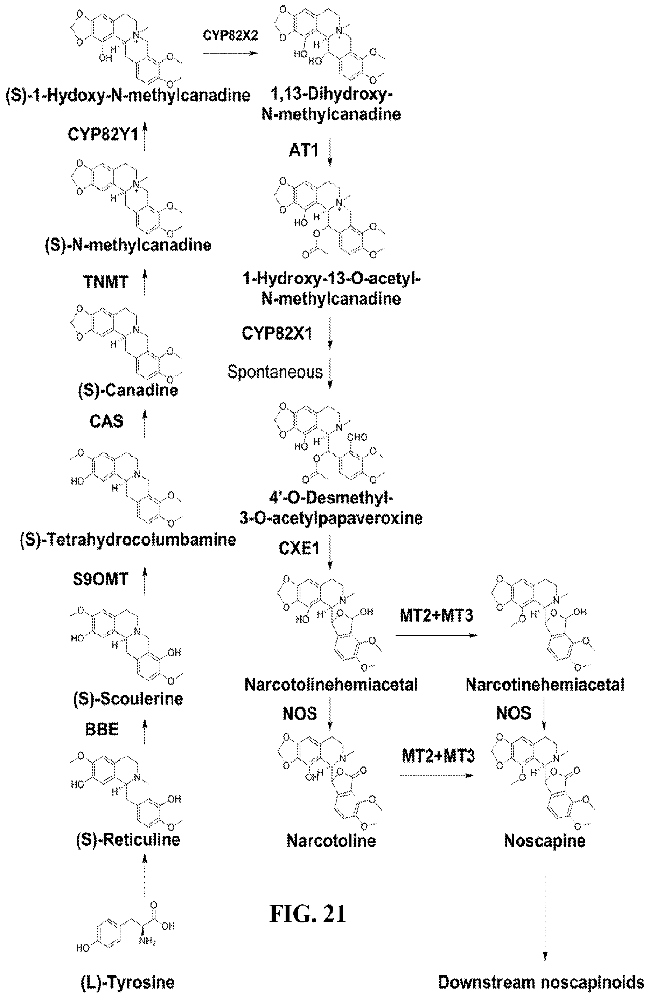

**FIG. 21** illustrates a biosynthetic scheme for conversion of L-tyrosine to noscapine, noscapinoid, and phthalideisoquinoline alkaloid products, in accordance with embodiments of the invention.

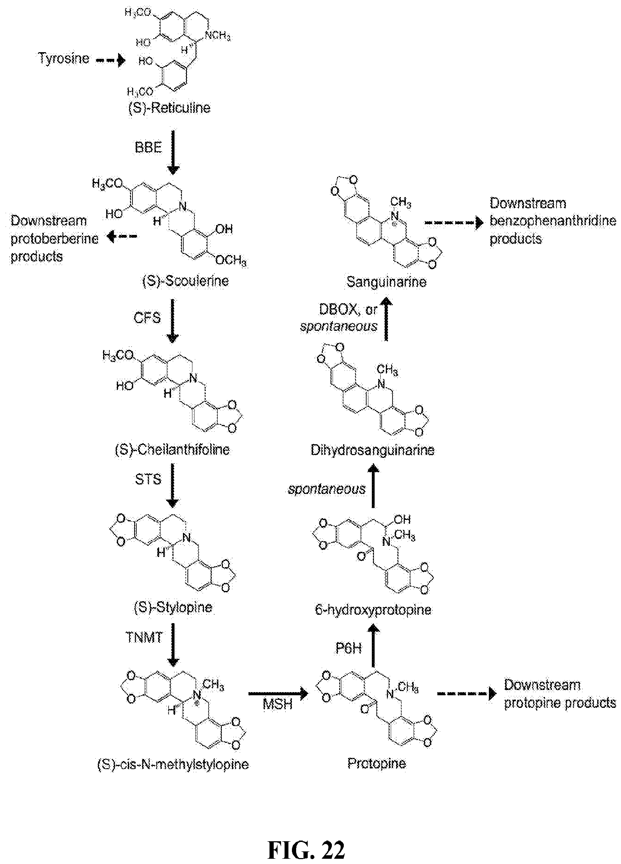

**FIG. 22** illustrates a biosynthetic scheme for conversion of L-tyrosine to sanguinarine and benzophenanthridine alkaloids, in accordance with embodiments of the invention.

**Table 1: Genes used as components of the engineered synthetic pathways in yeast**

| **Enzyme** | **Abbrev** | **Catalyzed Reactions** | **Source organisms** | **Similarity to naturally occurring gene** | **Modifications*** | **Genbank #** |
|---|---|---|---|---|---|---|
| Transketolase | TKL1 | fructose-6-phosphate + glyceraldehyde-3-phosph ate ↔ xylulose-5-phosphate + erythrose-4-phosphate (EC 2.2.1.1) | *Saccharomyces cerevisiae* | 100% | constitutive overexpression, synthetic regulation | NP_015399.1 |
| Glucose-6-phosph ate dehydrogenase | ZWF1 | glucose-6-phosphate → 6-phosphogluconolacto ne (EC 1.1.1.49) | *S*. *cerevisiae* | full deletion of coding region | full deletion of coding region | CAA96146.1 |
| 3-deoxy-d-arabino se-heptulosonate-7-phosphate synthase | ARO4, DHAP synthase | erythrose-4-phosphate + PEP → DHAP (EC 2.5.1.54) | *S*. *cerevisiae* | 100% | Feedback inhibition resistant mutation, K229L, Q166K | CAA85212.1 |
| Chorismate mutase | ARO7 | chorismate → prephenate (EC 5.4.99.5) | *S*. *cerevisiae* | 100% | Feedback inhibition resistant mutation, T226I | NP_015385.1 |
| Phenylpyruvate decarboxylase | ARO10 | hydroxyphenylpyruvate → 4HPA (EC 4.1.1.80) | *S*. *cerevisiae* | 100% | constitutive overexpression, synthetic regulation | NP_010668.3 |
| Aromatic aminotransferase | ARO9 | hydroxyphenylpyruvate + glutamate → tyrosine + alpha-ketogluterate (EC 2.6.1.57) | *S*. *cerevisiae* | 100% | constitutive overexpression, synthetic regulation | AEC14313.1 |
| Tyrosine hydroxylase | TyrH | tyrosine → L-DOPA (EC 1.14.16.2) | *Homo sapiens, Rattus norvegicus, Mus musculus* | 100% | constitutive expression, synthetic regulation | NM 012740, NM 000240 |
| L-DOPA decarboxylase | DODC | L-DOPA → dopamine (EC 4.1.1.28) | *Pseudomonas putida, Rattus norvegicus* | 100% | constitutive expression, synthetic regulation | AE015451.1, NP_001257782.1 |
| Norcoclaurine synthase | NCS | 4HPA + dopamine → S- norcoclaurine (EC 4.2.1.78) 3,4-DHPA + dopamine → S- norlaudanosoline | *Coptis japonica, Papaver somniferum, Papver bracteatum, Thalicitum flavum, Corydalis saxicola* | 80% | constitutive expression, synthetic regulation; N-terminal truncation | BAF45337.1, AB267399.2, ACI45396.1, AC O90258.1, ACO90247.1, AEB71889.1 |
| Norcoclaurine 6-0-methyltransferase | 6OMT | Norcoclaurine →□ coclaurine Norlaudanosoline →3'hydroxycoclaurine EC 2.1.1.128 | *P. somniferum* | 100% | constitutive expression, synthetic regulation | AY268894 |
| | | | *T. flavum* | 100% | | |
| | | | *C*. *japonica* | 100% | | AY610507 |
| | | | | | | D29811 |
| Coclaurine-N-methyltransferase | CNMT | Coclaurine →N-methylcoclaurine 3'hydroxycoclaurine →3'-hydroxy-N-methylcoclaurine EC 2.1.1.140 | *P. somniferum* | 100% | constitutive expression, synthetic regulation | AY217336 |
| | | | *T. flavum* | | | |
| | | | *C. japonica* | | | AY610508 |
| | | | | | | AB061863 |
| 4'-O-methyltransf erase | 4'OMT | 3'-hydroxy-N-methylcocl aurine →Reticuline EC 2.1.1.116 | *P. somniferum* | 100% | constitutive expression, synthetic regulation | |
| | | | *T. flavum* | 100% | | AY217333, |
| | | | *C. japonica* | 100% | | AY217334 |
| | | | | | | AY610510 |
| | | | | | | D29812 |
| Cytochrome P450 80B1 | CYP80B1 | N-methylcoclaurine → 3'-hydroxy-N-methylcocl aurine | *P. somniferum, Eschscholzia californica, T. flavum* | 77% | constitutive expression, synthetic regulation | AAF61400.1 |
| | | | | | | AAC39453.1 |
| | | | | | | AAU20767.1 |
| 6-pyruvoyl tetrahydrobiopteri n (PTP) synthase | PTPS | dihydroneopterin triphosphate → PTP (EC 4.2.3.12) | *R. norvegicus, H. sapiens, Mus musculus* | 80% | constitutive expression, synthetic regulation | AAH59140.1, BAA04224.1, AAH29013.1 |
| Sepiapterin reductase | SepR | PTP → BH4 (EC 1.1.1.153) | *R. norvegicus, H. sapiens, Mus musculus* | 72% | constitutive expression, synthetic regulation | NP_062054.1, NP_003115.1, NP_035597.2 |
| 4a-hydroxytetrahy drobiopterin (pterin-4lationinol amine) dehydratase | PCD | 4a-hydroxytetrahy drobiopterin → H2O + quinoid dihydropteridine (EC 4.2.1.96) | *R. norvegicus, H. sapiens, Mus musculus* | 79% | constitutive expression, synthetic regulation | NP_001007602.1 |
| | | | | | | AAB25581.1, NP_079549.1 |
| Quinoid dihydropteridine reductase | QDHPR | quinoid dihydropteridine → BH4 (EC 1.5.1.34) | *R. norvegicus, H. sapiens, Mus musculus* | 75% | constitutive expression, synthetic regulation | AAH72536.1, NP_000311.2, AAH02107.1 |
| Dihydrofolate reductase | DHFR | 7,8-Dihydrobiopterin → 5,6,7,8-Tetrahydrobiop terin (BH4) EC 1.5.1.3 | *R. norvegicus, H. sapiens* | 77% | constitutive expression, synthetic regulation | AF318150.1 |
| NADPH P450 reductase | CPR | | *E. californica* | 100% | constitutive expression, synthetic regulation | |
| | | | *P. somniferum* | | | |
| | | | *H. sapiens* | | | |
| | | | *S. cerevisiae* | | | |
| | | | *Arabidopsis thaliana* | | | |
| (S)-reticuline:oxy gen oxidoreductase (methylene-bridge-forming), also known as berberine bridge enzyme | BBE | (S)-reticuline + 02 → (S)-scoulerine + H2O2 EC 1.21.3.3 | *P. somniferum, Argemone mexicana, E. californica, Berberis stolonifera, Thalictrum flavum subsp. glaucum, C. japonica, Papaver spp, Eschscholzia spp, Berberis spp, Thalictrum spp, Coptis spp* | 99% | constitutive expression, synthetic regulation | AF025430, EU881889, EU881890, S65550 |
| | | | | | | AF005655, AF049347, AY610511, AB747097 |
| S-adenosyl-L-met hionine:(S)-scoulerine 9-*O-*methyltransferase | S9OMT | S-adenosyl-L-methioni ne + (S)-scoulerine → S-adenosyl-L-homocystei ne + (S)-tetrahydrocolumbamine EC 2.1.1.117 | *T. flavum subsp. glaucum, P. somniferum, C. japonica, Coptis chinensis, Thalictrum spp, Coptis spp, Papaver spp* | 100% | constitutive expression, synthetic regulation, Codon-optimized by Life Technologies for expression in *Saccharomyces cerevisiae* | AY610512, D29809, EU980450, JN185323 |
| | | | | 80% | | |
| NADPH:hemoprotei n oxidoreductase, also known as cytochrome P450 reductase | ATR1, CPR | NADPH + H+ + n oxidized hemoprotein → NADP+ + n reduced hemoprotein EC 1.6.2.4 | *A. thaliana, all plants, yeast, human* | 100% | constitutive expression, synthetic regulation at various promoter strengths | NM118585 , many others (Ref PMID 19931102) |
| (S)-tetrahydrocolum bamine, NADPH: oxygen oxidoreductase (methylenedioxy-br idge- forming), also known as (S)-canadine synthase | CAS | (S)-tetrahydrocolum bamine + NADPH + H+ + O2 → (S)-canadine + NADP+ + 2 H2O | *T. flavum subsp. glaucum, C. japonica, Thalictrum spp, Coptis spp* | 100% | constitutive expression, synthetic regulation | AY610513, AB026122, AB374407, AB374408 |
| | | EC 1.14.21.5 | | | | |
| Tetrahydroprotob erberine-N-methyltransferase | TNMT | Stylopine → cis-N-methylstylopi n e EC 2.1.1.122 Canadine → N-methylcanadine | *P. somniferum* | 100% | constitutive expression, synthetic regulation, Codon-optimized by Life Technologies for expression in *Saccharomyces cerevisiae* | DQ028579 |
| | | | *E. californica* | | | |
| | | | *P. bracteatum* | | | EU882977 |
| | | | *A. mexicana* | 81% | | EU882994 |
| | | | | | | HQ116698 |
| N-methylcanadine 14-hydroxylase | CYP82Y1 | N-methylcanadine → 1-hydroxy-N-methylcan adine | *P. somniferum, Papaver spp, Plantago arenaria, Rauwolfia heterophylla, Adlumia fungosa, Hydrastis Canadensis, Stylomecon heterophylla, Hypecoum leptocarpum, Dactylicapnos torulosa, Glaucium flavum, Berberis laurina, Beta vulgaris, Corydalis spp, Fumaria spp, Dactylicapnos spp* | 70(N-terminus engineering)-78 % | constitutive expression, synthetic regulation; Codon-optimized by Life Technologies for expression in *Saccharomyces cerevisiae*; N-terminus engineering | JQ65900651 |
| 1-hydroxy-N-met hylcanadine 13-hydroxylase | CYP82X2 | 1-hydroxy-N-methylcan adine → 1, 13-dihydroxy-N-methylc anadine | *P. somniferum,* | 70(N-terminus engineering)-77 % | constitutive expression, synthetic regulation; Codon-optimized by Life Technologies for expression in *Saccharomyces cerevisiae*; N-terminus engineering | JQ659004.1 |
| | | | *Papaver spp, P. arenaria, P. heterophylla, A. fungosa, H. Canadensis, S. heterophylla, D. torulosa, G. flavum, B. laurina, B. vulgaris, Corydalis spp, Fumaria spp, Dactylicapnos spp* | | | |
| 4'-O-Desmethyl-3 -O-acetylpapaver oxine synthase | CYP82X1 | 1-Hydroxy-13-O-acetyl-N-methylcanadine → 4' O-Desmethyl-3-O-acet ylpapaveroxine | *P. somniferum,* | 71(N-terminus engineering)-77 % | constitutive expression, synthetic regulation; Codon-optimized by Life Technologies for expression in *Saccharomyces cerevisiae*; N-terminus engineering | JQ659002.1 |
| | | | *Papaver spp, P. arenaria, R. heterophylla, A. fungosa, H. Canadensis, S. heterophylla, D. torulosa, G. flavum, B. laurina, B. vulgaris, Corydalis spp, Fumaria spp, Dactylicapnos spp* | | | |
| | | 1-hydroxy-N-methylcan adine →, 4'-O-desmethylmacrant aldehyde | | | | |
| Narcotoline 4'-O-methylase I | MT2 | Narcotoline → Noscapine | *P. somniferum,* | 80% | constitutive expression, synthetic regulation; Codon-optimized by Life Technologies for expression in *Saccharomyces cerevisiae* | JQ659000.1 |
| | | Narcotolinehemiacetal → Narcotinehemiacetal | *Papaver spp, Fumaria parviflora, P. arenaria, R. heterophylla* | | | |
| | | 4'-O-desmethyl-3-O-ace tylpapaveroxine → 3-O-acetylpapveroxine | | | | |
| 1, 13-dihydroxy-N-met hylcanadine 13-0 acetyl transferase | AT1 | 1, 13-dihydroxy-N-methylca nadine → 1-hydroxy-13-O-acetyl- -N-methycanadine | *P. somniferum, Papaver spp, P. arenaria, R. heterophylla, A. fungosa, H. Canadensis, S. heterophylla, H. leptocarpum, D. torulosa, G. flavum, B. laurina, B. vulgaris, Corydalis spp, Fumaria spp, Dactylicapnos spp* | 81% | constitutive expression, synthetic regulation; Codon-optimized by Life Technologies for expression in *Saccharomyces cerevisiae* | JQ659008.1 |
| Narcotinehemiac etal synthase | CXE1 | 4'-O-desmethyl-3-O-ace tylpapaveroxine → Narcotolinehemiacetal Papaveroxine → Narcotinehemiacetal | *P. somniferum, Papaver spp, P. arenaria, R. heterophylla, A. fungosa, H. Canadensis, S. heterophylla, H. leptocarpum, D. torulosa, G. flavum, B. laurina, B. vulgaris, Corydalis spp, Fumaria spp, Dactylicapnos spp* | 78% | constitutive expression, synthetic regulation; Codon-optimized by Life Technologies for expression in *Saccharomyces cerevisiae* | JQ659006.1 |
| Narcotinehemiac etal synthase | CXE2 | 4'-O-desmethyl-3-O-ace tylpapaveroxine → Narcotolinehemiacetal | *P. somniferum, Papaver spp, P. arenaria, R. heterophylla, A. fungosa, H. Canadensis, S. heterophylla, H. leptocarpum, D. torulosa, G. flavum, B. laurina, B. vulgaris, Corydalis spp, Fumaria spp, Dactylicapnos spp* | 78% | constitutive expression, synthetic regulation; Codon-optimized by Life Technologies for expression in *Saccharomyces cerevisiae* | KJ890443.1 |
| | | Papaveroxine → Narcotinehemiacetal | | | | |
| Noscapine synthase | SDR1 | Narcotolinehemiacetal → Narcotoline | *P. somniferum, Papaver spp, P. arenaria, P. heterophylla, A. fungosa, H. Canadensis, S. heterophylla, H. leptocarpum, D. torulosa, G. flavum, B. laurina, B. vulgaris, Corydalis spp, Fumaria spp, Dactylicapnos spp* | 79% | constitutive expression, synthetic regulation; Codon-optimized by Life Technologies for expression in *Saccharomyces cerevisiae* | JQ659007.1 |
| | | Narcotinehemiacetal → Noscapine | | | | |
| Narcotoline 4'-O-methylase II | MT3, | Narcotoline → Noscapine Narcotolinehemiacetal → Narcotinehemiacetal 4'-O-desmethyl-3-O-ace tylpapaveroxine → 3-O-acetylpapveroxine | *P. somniferum,* | 79% | constitutive expression, synthetic regulation; Codon-optimized by Life Technologies for expression in *Saccharomyces cerevisiae* | JQ659001.1 |
| | Ps6OMT | | *Papaver spp*, *F. parviflora, P. arenaria, R. heterophylla* | | | |

**Table 2. Comparison of impurities that may be present in concentrate of poppy straw and clarified yeast culture medium.**

| **Impurities:** | | **Concentrate of Poppy Straw** | **Clarified Yeast Culture Medium** |
|---|---|---|---|
| Inorganic | Sodium | ✔ | ✔ |
| | Magnesium | ✔ | ✔ |
| | Silicon | ✔ | × (not in culture medium) |
| | Phosphorus | ✔ | ✔ |
| | Sulfur | ✔ | ✔ |
| | Chloride | ✔ | ✔ |
| | Potassium | ✔ | ✔ |
| | Calcium | ✔ | ✔ |
| | Copper | ✔ | ✔ |
| | Zinc | ✔ | ✔ |
| | Molybdenum | ✔ | ✔ (sodium molybdate in medium) |
| | Iron | ✔ | ✔ |
| | Manganese | ✔ | ✔ |
| | Ammonium | ✔ | ✔ |
| | Boron | ✔ | ✔ |
| Organic | Polysaccharides (starch, cellulose, xylan) | ✔ | × (yeast fed simple sugars) |
| | Lignin (p-cournaryl, coniferyl, sinapyl alcohols) | ✔ | × |
| | Pigments (chlorophyll, anthocyanins, carotenoids) | ✔ | × |
| | Flavonoids | ✔ | × |
| | Phenanthreoids | ✔ | × |
| | Latex, gum, and wax | ✔ | × |
| | Rubisco | ✔ | × |
| | Meconic acid | ✔ | × |
| | Pseudomorphine | ✔ | × |
| | Narceine | ✔ | × |
| | Thebaol | ✔ | × |
| Other | Pesticides | ✔ | × |
| | Pollen | ✔ | × |

**Table 3. Distinct groups of molecules present in clarified yeast culture medium (CYCM).**

| **1.Benzylisoqulnoline** | **Proteberberine and Phthalldeisoquinoline** | **Morphinan** | **Isopavine** | **Aporphine** | **BisBIA** |
|---|---|---|---|---|---|
| Tetrahydropapaverine | Scoulerine | Salutaridiine | Pavine | Magnoflorine | Dauricine |
| Dihydropapaverine | Chelanthifoline | Salutaridinol | Caryachine | Corytuberine | Berbamunine |
| Papaverine | Stylopine | Salutaridine-7-O-acetate | Bisnorarge monine | Apomorphine | Ligensinine |
| | Cis-N-methylstylopine | Thebaine | Isonorarem onine | Boldine | Fangchinoline |
| | Protopine | Codeinone | | | Tetrandrine |
| | Dihydrosanguinarine | Oripavitie | | | Curine |
| | Sanguinarine | Morphinone | | | Cepharanthine |
| | Tetrahydrocolumbamine | Neopinone | | | Berbamine |
| | Canadine | Neopine | | | |
| | N-methylcanadine | Codeine | | | |
| | Noscapine | Morphine | | | |
| | Berberine | Neomorphine | | | |
| | | Hydrocodone | | | |
| | | Oxycodone | | | |
| | | 14-hydroxycodeinone | | | |
| | | 14-hydroxycodeine | | | |
| | | Dihydromorphine | | | |
| | | Dihydrocodeine | | | |

### Discussion of Enzyme List

The host cells may be engineered to include one or more modifications (such as two or more, three or more, four or more, five or more, or even more modifications) that provide for the production of BIAs of interest and/or enzymes of interest. **Table 1** provides a list of exemplary genes that may be acted upon by one or more modifications so as to provide for the production of BIAs of interest and/or enzymes of interest in an engineered host cell.

Modifications of genes as provided in **Table 1** may be used to produce BIAs of interest from engineered host cells that are supplied with a medium containing the minimal nutrients required for growth. This minimal medium may contain a carbon source, a nitrogen source, amino acids, vitamins, and salts. For example, modifications of genes as provided in **Table 1** may be used to produce BIAs of interest from engineered host cells that are fed sugar. Additionally, modifications of one or more genes as provided in **Table 1** may be used to augment the biosynthetic processes of host cells that may be engineered for drug production.

Additionally, the use of these modifications to provide for the production of BIAs of interest and/or enzymes of interest in engineered host cells is not readily apparent from the mere identification of enzymes that may be produced by the genes. In particular, synthetic pathways that have been reconstructed in host cells, such as yeast cells, as described herein comprise a variety of enzymes that do not act together in nature within a single organism. Additionally, some of the enzymes discussed herein do not act for BIA biosynthesis in their natural context. Further, some of the enzymes described herein are not evolved to function in particular host cells, such as yeast cells, and are not evolved to function together. In these cases, it would not be obvious that the enzymes would exhibit sufficient activity in the context of the synthetic BIA pathway in a host cell, such as yeast, to have sufficient flux through the pathway to produce downstream BIA end products.

For example, plant enzymes are often difficult to functionally express in heterologous microbial hosts, such as yeast. In many cases the enzymes may be misfolded, not correctly localized within the host cell, and/or incorrectly processed. The differences in protein translation and processing between yeast and plants can lead to these enzymes exhibiting substantially reduced to no detectable activities in the yeast host. These challenges arise commonly for endomembrane localized enzymes, such as cytochrome P450s, which are strongly represented in the BIA pathways. Even reduced enzyme activities may pose a substantial challenge to engineering yeast to produce complex BIAs, which requires sufficient activity at each step to ensure high-level accumulation of the desired BIA products.

Additionally, there are endogenous enzymes/pathways in some host cells, such as yeast, that may act on many of the early precursors in the BIA pathway (i.e., intermediates from tyrosine to norcoclaurine), and thus it may not be readily apparent that there would be sufficient flux through the heterologous pathway to achieve substantial BIA production given these competing endogenous pathways. For example, the Erlich pathway (Hazelwood, et al. 2008. Appl. Environ. Microbiol. 74: 2259-66; Larroy, et al. 2003. Chem. Biol. Interact. 143-144: 229-38; Larroy, et al. 2002. Eur. J. Biochem. 269: 5738-45) in yeast is the main endogenous pathway that would act to convert many of the intermediates in the early BIA pathway to undesired products and divert flux from the synthetic pathway.

Further, many of the enzymes as discussed herein, and as provided in **Table 1,** may function under very specific regulation strategies, including spatial regulation, in the native plant hosts, which may be lost upon transfer to the heterologous yeast host. In addition, plants present very different biochemical environments than yeast cells under which the enzymes are evolved to function, including pH, redox state, and substrate, cosubstrate, coenzyme, and cofactor availabilities. Given the differences in biochemical environments and regulatory strategies between the native hosts and the heterologous yeast hosts, it is not obvious that the enzymes would exhibit substantial activities when in the context of the yeast environment and further not obvious that they would work together to direct simple precursors such as sugar to complex BIA compounds. Maintaining the activities of the enzymes in the yeast host is particularly important as many of the pathways have many reaction steps (>10), such that if these steps are not efficient then one would not expect accumulation of desired downstream products.

In addition, in the native plant hosts, the associated metabolites in these pathways may be localized across different cell and tissue types. In several examples, there are cell types that may be specialized for biosynthesis and cell types that may be synthesized for metabolite accumulation. This type of cell specialization may be lost when expressing the pathways within a heterologous yeast host, and may play an important role in controlling the toxicity of these metabolites on the cells. Thus, it is not obvious that yeast could be successfully engineered to biosynthesize and accumulate these metabolites without being harmed by the toxicity of these compounds.

As one example, in the native plant hosts, the enzyme BBE is reported to have dynamic subcellular localization. In particular, the enzyme BBE initially starts in the ER and then is sorted to the vacuole (Bird and Facchini. 2001. Planta. 213: 888-97). It has been suggested that the ER-association of BBE in plants (Alcantara, et al. 2005. Plant Physiol. 138: 173-83) provides the optimal basic pH (pH ~8.8) for BBE activity (Ziegler and Facchini. 2008. Annu. Rev. Plant Biol. 59: 735-69). As another example, there is evidence that sanguinarine biosynthesis occurs in specialized vesicles within plant cells (Amann, et al. 1986. Planta. 167: 310-20), but only some of the intermediates accumulate in the vesicles. This may occur so as to sequester them from other enzyme activities and/or toxic effects.

As another example, the biosynthetic enzymes in the morphinan pathway branch are all localized to the phloem, which is part of the vascular tissue in plants. In the phloem, the pathway enzymes may be further divided between two cell types: the sieve elements common to all plants, and the laticifer which is a specialized cell type present only in certain plants which make specialized secondary metabolites. The upstream enzymes (i.e., from NCS through to SalAT) are predominantly in the sieve elements, and the downstream enzymes (i.e., T6ODM, COR, CODM) are mostly in the laticifer (Onoyovwe, et al. 2013. Plant Cell. 25: 4110-22). Additionally, it was discovered that the final steps in the noscapine biosynthetic pathway take place in the laticifer (Chen and Facchini. 2014. Plant J. 77: 173-84). This compartmentalization is thought to be highly important for regulating biosynthesis by isolating or trafficking intermediates, providing optimal pH, enhancing supply of cofactors, although the nature of the poppy laticifer microenvironment is still under investigation (Ziegler and Facchini. 2008. Annu. Rev. Plant Biol. 59: 735-69). Further, it is predicted that several of the enzymes may function as multi-enzyme complexes or metabolic channels common to plant secondary metabolism (Kempe, et al. 2009. Phytochemistry. 70: 579-89; Allen, et al. 2004. Nat. Biotechnol. 22: 1559-66). When biosynthetic enzymes are combined from different hosts and/or expressed recombinantly in a heterologous yeast cell it is not clear that these complexes or channels will form as they would in the native host. In an additional example, in *Coptis japonica,* berberine is biosynthesized in root tissues and then accumulated within the rhizome via the action of specialized ATP-binding cassette transport proteins (Shitan, et al. 2013. Phytochemistry. 91: 109-16). In opium poppy, morphinan alkaloids are accumulated within the latex (cytoplasm of laticifer cells) (Martin, et al. 1967. Biochemistry. 6: 2355-63).

Further, even without these considerations, it is also the case that the plant enzymes for several of the steps in the pathways described herein have not yet been characterized. For example, the conversion of tyrosine to the early benzylisoquinoline alkaloid scaffold norcoclaurine has not yet been characterized. Additionally, the conversion of narcotoline to noscapine has only recently been characterized as described herein. Thus, for several of the steps in the pathways described herein, alternative biosynthetic scheme were produced by bringing together enzyme activities that do not normally occur together in nature for the biosynthesis of BIAs or identifying new enzyme activities from genome sequence information to use in the reconstructed pathways.

For example, the two-step conversion of tyrosine to dopamine may be achieved by combining at least 5 mammalian enzymes and 1 bacterial enzyme, which do not naturally occur together and were not evolved to function in the context of this pathway or with plant enzymes. In these instances, it may not be obvious to utilize these enzymes for the biosynthesis of compounds they were not evolved for in nature and that they would function effectively in the context of a heterologous microbial host and this pathway. As another example, the enzyme responsible for the conversion of narcotoline to noscapine was unknown. Although the in planta investigation indicates PsMT2 involved in this conversion, PsMT2 itself cannot methylate narcotoline to afford noscapine. A novel enzyme complex as discussed herein performs this O-methylation reaction in yeast and in the context of the synthetic BIA pathway. Due to the high sequence similarity between Ps6OMT and PsMT3, it would have been difficult to discover the functional PsMT2/PsMT3 enzyme complex in plant. The clean enzyme background in yeast make the discovery of the PsMT2/PsMT3 heterodimer as the narcotoline 4'-O-methyltransferase possible as stated herein..

Examples of the genes that are the object of modifications so as to produce BIAs of interest and/or enzymes of interest are discussed below. Additionally, the genes are discussed in the context of a series of **FIGs.** that illustrate pathways that are used in generating BIAs of interest and/or enzymes of interest.

**[TLK1]** In some examples, the engineered host cell may modify the expression of the enzyme transketolase. Transketolase is encoded by the TKL1 gene. In examples, transketolase catalyzes the reaction of fructose-6-phosphate + glyceraldehyde-3-phosphate ↔ xylulose-5-phosphate + erythrose-4-phosphate, as referenced in **FIG. 16****.** An engineered host cell may be modified to include constitutive overexpression of the TKL1 gene in the engineered host cell. Additionally or alternatively, the engineered host cell may be modified to synthetically regulate the expression of the TKL1 gene in the engineered host cell. In examples, the engineered host cell may be modified to incorporate a copy, copies, or additional copies, of the TKL1 gene. Additionally or alternatively, the engineered host cell may be modified to incorporate the introduction of a strong promoter element for the overexpression of the TKL1 gene within the engineered host cell. The TKL1 gene may be derived from *Saccharomyces cerevisiae* or another species. In some examples, the TKL1 gene may be 100% similar to the naturally occurring gene.

**[ZWF1]** In some examples, the engineered host cell may modify the expression of the enzyme glucose-6-phosphate dehydrogenase. Glucose-6-phosphate dehydrogenase is encoded by the ZWF1 gene. In examples, glucose-6-phosphate dehydrogenase catalyzes the reaction of glucose-6-phosphate → 6-phosphogluconolactone, as referenced in **FIG. 16****.** An engineered host cell may be modified to delete the coding region of the ZWF1 gene in the engineered host cell. Alternatively, the engineered host cell may be modified to disable the functionality of the ZWF1 gene, such as by introducing an inactivating mutation.

**[ARO4]** In some examples, the engineered host cell may modify the expression of the enzyme 3-deoxy-D-arabino-heptulosonate-7-phosphate (DAHP) synthase. DAHP synthase is encoded by the ARO4 gene. In examples, DAHP synthase catalyzes the reaction of erythrose-4-phosphate + phosphoenolpyruvic acid → DAHP, as referenced in **FIG. 16****.** An engineered host cell may modify the ARO4 gene to incorporate one or more feedback inhibition alleviating mutations. In particular, a feedback inhibition alleviating mutation (e.g., ARO4^{FBR}) may be incorporated as a directed mutation to a native ARO4 gene at the original locus; as an additional copy introduced as a genetic integration at a separate locus; or as an additional copy on an episomal vector such as a 2-µm or centromeric plasmid. The identifier "FBR" in the mutation ARO4^{FBR} refers to feedback resistant mutants and mutations. The feedback inhibited copy of the DAHP synthase enzyme may be under a native yeast transcriptional regulation, such as when the engineered host cell is a yeast cell. Alternatively, the feedback inhibited copy of the DAHP synthase enzyme may be introduced to the engineered host cell with engineered constitutive or dynamic regulation of protein expression by placing it under the control of a synthetic promoter. In some cases, the ARO4 gene may be derived from *Saccharomyces cerevisiae.* In some cases, the ARO4 gene may be 100% similar to the naturally occurring gene. Examples of modifications to the ARO4 gene include a feedback inhibition resistant mutation, K229L, or Q166K.

**[ARO7]** In some examples, the engineered host cell may modify the expression of the enzyme chorismate mutase. Chorismate mutase is encoded by the ARO7 gene. In examples, chorismate mutase catalyzes the reaction of chorismate → prephenate, as referenced in **FIG. 16****.** An engineered host cell may modify the ARO7 gene to incorporate one or more feedback inhibition alleviating mutations. In particular, a feedback inhibition alleviating mutation (e.g., ARO7^{FBR}) may be incorporated as a directed mutation to a native ARO7 gene at the original locus; as an additional copy introduced as a genetic integration at a separate locus; or as an additional copy on an episomal vector such as a 2-µm or centromeric plasmid. The identifier "FBR" in the mutation ARO7^{FBR} refers to feedback resistant mutants and mutations. The feedback inhibited copy of the chorismate mutase enzyme may be under a native yeast transcriptional regulation, such as when the engineered host cell is a yeast cell. Alternatively, the feedback inhibited copy of the chorismate mutase enzyme may be introduced to the engineered host cell with engineered constitutive or dynamic regulation of protein expression by placing it under the control of a synthetic promoter. In some cases, the ARO7 gene may be derived from *Saccharomyces cerevisiae.* In some cases, the ARO7 gene may be 100% similar to the naturally occurring gene. Examples of modifications to the ARO7 gene include a feedback inhibition resistant mutation or T226I.

**[ARO10]** In some examples, the engineered host cell may modify the expression of the enzyme phenylpyruvate decarboxylase. Phenylpyruvate decarboxylase is encoded by the ARO10 gene. In examples, phenylpyruvate decarboxylase catalyzes the reaction of hydroxyphenylpyruvate → 4-hydroxyphenylacetate (4HPA), as referenced in **FIG. 16****.** An engineered host cell may be modified to include constitutive overexpression of the ARO10 gene in the engineered host cell. Additionally or alternatively, the engineered host cell may be modified to synthetically regulate the expression of the ARO10 gene in the engineered host cell. In examples, the engineered host cell may be modified to incorporate a copy, copies, or additional copies, of the ARO10 gene. Additionally or alternatively, the engineered host cell may be modified to incorporate the introduction of a strong promoter element for the overexpression of the ARO10 gene within the engineered host cell. The ARO10 gene may be derived from *Saccharomyces cerevisiae* or another species. In some examples, the ARO10 gene may be 100% similar to the naturally occurring gene.

**[ARO9]** In some examples, the engineered host cell may modify the expression of the enzyme aromatic aminotransferase. Aromatic aminotransferase is encoded by the ARO9 gene. In examples, aromatic aminotransferase catalyzes the reaction of hydroxyphenylpyruvate + glutamate → tyrosine + alpha-ketogluterate, as referenced in FIG. 16. An engineered host cell may be modified to include constitutive overexpression of the ARO9 gene in the engineered host cell. Additionally or alternatively, the engineered host cell may be modified to synthetically regulate the expression of the ARO9 gene in the engineered host cell. In examples, the engineered host cell may be modified to incorporate a copy, copies, or additional copies, of the ARO9 gene. Additionally or alternatively, the engineered host cell may be modified to incorporate the introduction of a strong promoter element for the overexpression of the ARO9 gene within the engineered host cell. The ARO9 gene may be derived from *Saccharomyces cerevisiae* or another species. In some examples, the ARO9 gene may be 100% similar to the naturally occurring gene.

**[TyrH]** In some examples, the engineered host cell may modify the expression of the enzyme tyrosine hydroxylase. Tyrosine hydroxylase is encoded by the TyrH gene. In examples, tyrosine hydroxylase catalyzes the reaction of tyrosine → L-DOPA, as referenced in **FIGs. 16** and **17****.** An engineered host cell may be modified to include constitutive expression of the TyrH gene in the engineered host cell. Additionally or alternatively, the engineered host cell may be modified to synthetically regulate the expression of the TyrH gene in the engineered host cell. In examples, the engineered host cell may be modified to incorporate a copy, copies, or additional copies, of the TyrH gene. Additionally or alternatively, the engineered host cell may be modified to incorporate the introduction of a strong promoter element for the overexpression of the TyrH gene within the engineered host cell. The TyrH gene may be derived from *Homo sapiens, Rattus norvegicus, Mus musculus,* or another species. In some examples, the TyrH gene may be 100% similar to the naturally occurring gene.

**[DODC]** In some examples, the engineered host cell may modify the expression of the enzyme L-DOPA decarboxylase. L-DOPA decarboxylase is encoded by the DODC gene. In examples, L-DOPA decarboxylase catalyzes the reaction of L-DOPA → dopamine, as referenced in **FIGs. 16** and **17****.** An engineered host cell may be modified to include constitutive expression of the DODC gene in the engineered host cell. Additionally or alternatively, the engineered host cell may be modified to synthetically regulate the expression of the DODC gene in the engineered host cell. In examples, the engineered host cell may be modified to incorporate a copy, copies, or additional copies, of the DODC gene. Additionally or alternatively, the engineered host cell may be modified to incorporate the introduction of a strong promoter element for the overexpression of the DODC gene within the engineered host cell. The DODC gene may be derived from *Pseudomonas putida, Rattus norvegicus,* or another species. In some examples, the DODC gene may be 100% similar to the naturally occurring gene.

[NCS] In some examples, the engineered host cell may modify the expression of the enzyme norcoclaurine synthase. Norcoclaurine synthase is encoded by the NCS gene. In examples, norcoclaurine synthase catalyzes the reaction of 4HPA + dopamine 4 (S)-norcoclaurine, as referenced in **FIG. 17****.** In particular, **FIG. 17** illustrates a biosynthetic scheme for conversion of L-tyrosine to reticuline via norcoclaurine, in accordance with embodiments of the invention. **FIG. 17** provides the use of the enzymes TyrH, tyrosine hydroxylase; DODC, DOPA decarboxylase; NCS, norcoclaurine synthase, as discussed herein; 6OMT, 6-O-methyltransferase; CNMT, coclaurine N-methyltransferase; CYP80B1, cytochrome P450 80B1; CPR, cytochrome P450 NADPH reductase; 4'OMT, 3'hydroxy-*N*-methylcoclaurine 4'-*O*-methyltransferase. L-DOPA, L-3,4-dihydroxyphenylalanine; and 4-HPA, 4-hydroxyphenylacetylaldehyde. Of the enzymes that are illustrated in FIG. 17, 4-HPA and L-tyrosine are naturally synthesized in yeast. All other metabolites shown are not naturally produced in yeast. Additionally, although TyrH is depicted as catalyzing the conversion of L-tyrosine to L-DOPA, other enzymes may also be used to perform this step as described in the specification. For example, tyrosinases may also be used to perform the conversion of L-tyrosine to L-DOPA. In addition, other enzymes such as cytochrome P450 oxidases may also be used to perform the conversion of L-tyrosine to L-DOPA. Such enzymes may exhibit oxidase activity on related BIA precursor compounds including L-DOPA and L-tyrosine.

Additionally, norcoclaurine synthase catalyzes the reaction of 3,4-DHPA + dopamine → (*S*)-norlaudanosoline, as referenced in **FIG. 18****.** In particular, **FIG. 18** illustrates a biosynthetic scheme for conversion of L-tyrosine to reticuline via norlaudanosoline, in accordance with embodiments of the invention. **FIG. 18** provides the use of the enzymes TyrH, tyrosine hydroxylase; DODC, DOPA decarboxylase; maoA, monoamine oxidase; NCS, norcoclaurine synthase; 6OMT, 6-*O*-methyltransferase; CNMT, coclaurine *N*-methyltransferase; 4'OMT, 3'hydroxy-*N*-methylcoclaurine 4'-*O*-methyltransferase. L-DOPA, L-3,4-dihydroxyphenylalanine; and 3,4-DHPA, 3,4-dihydroxyphenylacetaldehyde. Of the enzymes that are illustrated in **FIG. 18****,** L-tyrosine is naturally synthesized in yeast. Other metabolites that are shown in **FIG. 18** are not naturally produced in yeast.

An engineered host cell may be modified to include constitutive expression of the NCS gene in the engineered host cell. Additionally or alternatively, the engineered host cell may be modified to synthetically regulate the expression of the NCS gene in the engineered host cell. In examples, the engineered host cell may be modified to incorporate a copy, copies, or additional copies, of the NCS gene. Additionally or alternatively, the engineered host cell may be modified to incorporate the introduction of a strong promoter element for the overexpression of the NCS gene within the engineered host cell. Additionally, the norcoclaurine synthase may have an N-terminal truncation. In some cases, the NCS gene may be codon optimized for expression in *Saccharomyces cerevisiae.* The NCS gene may be derived from *Coptis japonica, Papaver somniferum, Papaver bracteatum, Thalicitum flavum, Corydalis saxicola,* or another species. In some examples, the NCS gene may be 80% similar to the naturally occurring gene.

**[6OMT]** In some examples, the engineered host cell may modify the expression of the enzyme norcoclaurine 6-*O*-methyltransferase. Norcoclaurine 6-*O*-methyltransferase is encoded by the 6OMT gene. In some examples, norcoclaurine 6-O-methyltransferase catalyzes the reaction of norcoclaurine → coclaurine, as referenced in **FIG. 17****.** In other examples, norcoclaurine 6-*O*-methyltransferase catalyzes the reaction of norlaudanosoline → 3'hydroxycoclaurine, as well as other reactions detailed herein, such as those provided in **FIG. 18****.** Additionally, the engineered host cell may be modified to include constitutive expression of the 6OMT gene in the engineered host cell. Additionally or alternatively, the engineered host cell may be modified to synthetically regulate the expression of the 6OMT gene in the engineered host cell. In examples, the engineered host cell may be modified to incorporate a copy, copies, or additional copies, of the 6OMT gene. Additionally or alternatively, the engineered host cell may be modified to incorporate the introduction of a strong promoter element for the overexpression of the 6OMT gene within the engineered host cell. The 6OMT gene may be derived from *P*. *somniferum, T. flavum, Coptis japonica,* or another species. In some examples, the 6OMT gene may be 100% similar to the naturally occurring gene.

**[CNMT]** In some examples, the engineered host cell may modify the expression of the enzyme coclaurine-*N*-methyltransferase. Coclaurine-*N*-methyltransferase is encoded by the CNMT gene. In some examples, coclaurine-*N*-methyltransferase catalyzes the reaction of coclaurine → *N*-methylcoclaurine, as referenced in **FIG. 17****.** In other examples, the coclaurine-*N*-methyltransferase enzyme may catalyze the reaction of 3'hydroxycoclaurine → 3'hydroxy-*N*-methylcoclaurine. In other examples, coclaurine-*N*-methyltransferase may catalyze other reactions detailed herein, such as those provided in **FIG. 18****.** Additionally, the engineered host cell may be modified to include constitutive expression of the CNMT gene in the engineered host cell. Additionally or alternatively, the engineered host cell may be modified to synthetically regulate the expression of the CNMT gene in the engineered host cell. In examples, the engineered host cell may be modified to incorporate a copy, copies, or additional copies, of the CNMT gene. Additionally or alternatively, the engineered host cell may be modified to incorporate the introduction of a strong promoter element for the overexpression of the CNMT gene within the engineered host cell. The CNMT gene may be derived from *P*. *somniferum, T. flavum, Coptis japonica,* or another species. In some examples, the CNMT gene may be 100% similar to the naturally occurring gene.

**[4'OMT]** In some examples, the engineered host cell may modify the expression of the enzyme 4'-*O*-methyltransferase. 4'-*O*-methyltransferase is encoded by the 4'OMT gene. In some examples, 4'-*O*-methyltransferase catalyzes the reaction of 3'-hydroxy-*N*-methylcoclaurine → reticuline, as referenced in **FIG. 17****.** In other examples, 4'-*O*-methyltransferase catalyzes other reactions detailed herein, such as those provided in **FIG. 18****.** Additionally, the engineered host cell may be modified to include constitutive expression of the 4'OMT gene in the engineered host cell. Additionally or alternatively, the engineered host cell may be modified to synthetically regulate the expression of the 4'OMT gene in the engineered host cell. In examples, the engineered host cell may be modified to incorporate a copy, copies, or additional copies, of the 4'OMT gene. Additionally or alternatively, the engineered host cell may be modified to incorporate the introduction of a strong promoter element for the overexpression of the 4'OMT gene within the engineered host cell. The 4'OMT gene may be derived from *P*. *somniferum, T. flavum, Coptisjaponica,* or another species. In some examples, the 4'OMT gene may be 100% similar to the naturally occurring gene.

**[CYP80B1]** In some examples, the engineered host cell may modify the expression of the enzyme cytochrome P450 80B1. Cytochrome P450 80B1 is encoded by the CYP80B1 gene. In examples, cytochrome P450 80B1 catalyzes the reaction of *N*-methylcoclaurine → 3'-hydroxy-*N*-methylcoclaurine, as referenced in **FIG. 17****.** An engineered host cell may be modified to include constitutive expression of the cytochrome P450 80B1 gene in the engineered host cell. Additionally or alternatively, the engineered host cell may be modified to synthetically regulate the expression of the cytochrome P450 80B1 gene in the engineered host cell. In examples, the engineered host cell may be modified to incorporate a copy, copies, or additional copies, of the cytochrome P450 80B1 gene. Additionally or alternatively, the engineered host cell may be modified to incorporate the introduction of a strong promoter element for the overexpression of the cytochrome P450 80B1 gene within the engineered host cell. In some cases, the CYP80B1 gene may be codon optimized for expression in *Saccharomyces cerevisiae.* The cytochrome P450 80B1 gene may be derived from *P*. *somniferum, E. californica, T. flavum,* or another species. In some examples, the P450 80B1 gene may be 77% similar to the naturally occurring gene.

**[PTPS]** In some examples, the engineered host cell may modify the expression of the enzyme 6-pyruvoyl tetrahydrobiopterin (PTP) synthase. Pyruvoyl tetrahydrobiopterin synthase is encoded by the PTPS gene. In some examples, 6-pyruvoyl tetrahydrobiopterin synthase catalyzes the reaction of dihydroneopterin triphosphate → PTP, as referenced in **FIG. 19****.** The engineered host cell may be modified to include constitutive expression of the PTPS gene in the engineered host cell. Additionally or alternatively, the engineered host cell may be modified to synthetically regulate the expression of the PTPS gene in the engineered host cell. In examples, the engineered host cell may be modified to incorporate a copy, copies, or additional copies, of the PTPS gene. Additionally or alternatively, the engineered host cell may be modified to incorporate the introduction of a strong promoter element for the overexpression of the PTPS gene within the engineered host cell. In some cases, the PTPS gene may be codon optimized for expression in *Saccharomyces cerevisiae.* The PTPS gene may be derived from *Rattus norvegicus, Homo sapiens, Mus musculus,* or another species. In some examples, the PTPS gene may be 80% similar to the naturally occurring gene.

**[SepR]** In some examples, the engineered host cell may modify the expression of the enzyme sepiapterin reductase. Sepiapterin reductase is encoded by the SepR gene. In some examples, sepiapterin reductase catalyzes the reaction of PTP → BH₄, as referenced in **FIG. 19****.** The engineered host cell may be modified to include constitutive expression of the SepR gene in the engineered host cell. Additionally or alternatively, the engineered host cell may be modified to synthetically regulate the expression of the SepR gene in the engineered host cell. In examples, the engineered host cell may be modified to incorporate a copy, copies, or additional copies, of the SepR gene. Additionally or alternatively, the engineered host cell may be modified to incorporate the introduction of a strong promoter element for the overexpression of the SepR gene within the engineered host cell. In some cases, the SepR gene may be codon optimized for expression in *Saccharomyces cerevisiae.* The SepR gene may be derived from *Rattus norvegicus, Homo sapiens, Mus musculus,* or another species. In some examples, the SepR gene may be 72% similar to the naturally occurring gene.

**[PCD]** In some examples, the engineered host cell may modify the expression of the enzyme 4a-hydroxytetrahydrobiopterin (pterin-4α-carbinolamine) dehydratase. 4a-hydroxytetrahydrobiopterin dehydratase is encoded by the PCD gene. In some examples, 4a-hydroxytetrahydrobiopterin dehydratase catalyzes the reaction of 4a-hydroxytetrahydrobiopterin → H₂O + quinonoid dihydropteridine, as referenced in **FIG. 19****.** The engineered host cell may be modified to include constitutive expression of the PCD gene in the engineered host cell. Additionally or alternatively, the engineered host cell may be modified to synthetically regulate the expression of the PCD gene in the engineered host cell. In examples, the engineered host cell may be modified to incorporate a copy, copies, or additional copies, of the PCD gene. Additionally or alternatively, the engineered host cell may be modified to incorporate the introduction of a strong promoter element for the overexpression of the PCD gene within the engineered host cell. In some cases, the PCD gene may be codon optimized for expression in *Saccharomyces cerevisiae.* The PCD gene may be derived from *Rattus norvegicus, Homo sapiens, Mus musculus,* or another species. In some examples, the PCD gene may be 79% similar to the naturally occurring gene.

**[QDHPR]** In some examples, the engineered host cell may modify the expression of the enzyme quinonoid dihydropteridine reductase. Quinonoid dihydropteridine reductase is encoded by the QDHPR gene. In some examples, quinonoid dihydropteridine reductase catalyzes the reaction of quinonoid dihydropteridine → BH₄, as referenced in **FIG. 19****.** The engineered host cell may be modified to include constitutive expression of the QDHPR gene in the engineered host cell. Additionally or alternatively, the engineered host cell may be modified to synthetically regulate the expression of the QDHPR gene in the engineered host cell. In examples, the engineered host cell may be modified to incorporate a copy, copies, or additional copies, of the QDHPR gene. Additionally or alternatively, the engineered host cell may be modified to incorporate the introduction of a strong promoter element for the overexpression of the QDHPR gene within the engineered host cell. In some cases, the QDHPR gene may be codon optimized for expression in *Saccharomyces cerevisiae.* The QDHPR gene may be derived from *Rattus norvegicus, Homo sapiens, Mus musculus,* or another species. In some examples, the QDHPR gene may be 75% similar to the naturally occurring gene.

**[DHFR]** In some examples, the engineered host cell may modify the expression of the enzyme dihydrofolate reductase. Dihydrofolate reductase is encoded by the DHFR gene. In some examples, dihydrofolate reductase catalyzes the reaction of 7,8-dihydrobiopterin (BH₂) → 5,6,7,8-tetrahydrobiopterin (BH₄), as referenced in **FIG. 19****.** This reaction may be useful in recovering BH₄ as a co-substrate for the converstion of tyrosine to L-DOPA, as illustrated in **FIG. 17****.** The engineered host cell may be modified to include constitutive expression of the DHFR gene in the engineered host cell. Additionally or alternatively, the engineered host cell may be modified to synthetically regulate the expression of the DHFR gene in the engineered host cell. In examples, the engineered host cell may be modified to incorporate a copy, copies, or additional copies, of the DHFR gene. Additionally or alternatively, the engineered host cell may be modified to incorporate the introduction of a strong promoter element for the overexpression of the DHFR gene within the engineered host cell. In some cases, the DHFR gene may be codon optimized for expression in *Saccharomyces cerevisiae.* The DHFR gene may be derived from *Rattus norvegicus, Homo sapiens,* or another species. In some examples, the DHFR gene may be 77% similar to the naturally occurring gene.

**[CPR]** In some examples, the engineered host cell may modify the expression of the enzyme cytochrome P450 reductase. The cytochrome P450 reductase catalyzes other reactions such as those described in FIGs. throughout the application. The engineered host cell may be modified to include constitutive expression of the CPR gene in the engineered host cell. Additionally or alternatively, the engineered host cell may be modified to synthetically regulate the expression of the CPR gene in the engineered host cell. In examples, the engineered host cell may be modified to incorporate a copy, copies, or additional copies, of the CPR gene. Additionally or alternatively, the engineered host cell may be modified to incorporate the introduction of a strong promoter element for the overexpression of the CPR gene within the engineered host cell. The CPR gene may be derived from *E. californica, P. somniferum, H. sapiens, S. cerevisiae, A. thaliana,* or another species. In some examples, the CPR gene may be 100% similar to the naturally occurring gene.

**[BBE]** In some examples, the engineered host cell may modify the expression of the enzyme berberine bridge enzyme. The berberine bridge enzyme is encoded by the BBE gene. In some examples, berberine bridge enzyme catalyzes the reaction of (*S*)-reticuline 4 (*S*)-scoulerine, as referenced in **FIG. 20. FIG. 20** illustrates a biosynthetic scheme for conversion of L-tyrosine to protoberberine alkaloids, in accordance with embodiments of the invention. In particular, **FIG. 20** provides the use of the enzymes BBE, berberine bridge enzyme; S9OMT, scoulerine 9-*O*-methyltransferase; CAS, canadine synthase; CPR, cytochrome P450 reductase; and STOX, tetrahydroprotoberberine oxidase. The engineered host cell may be modified to include constitutive expression of the BBE gene in the engineered host cell. Additionally or alternatively, the engineered host cell may be modified to synthetically regulate the expression of the BBE gene in the engineered host cell. In examples, the engineered host cell may be modified to incorporate a copy, copies, or additional copies, of the BBE gene. Additionally or alternatively, the engineered host cell may be modified to incorporate the introduction of a strong promoter element for the overexpression of the BBE gene within the engineered host cell. The BBE gene may be derived from *Papaver somniferum, Argemone mexicana, Eschscholzia californica, Berberis stolonifera, Thalictrum flavum subsp. glaucum, Coptis japonica,Papaver spp.,* or another species. In some examples, the BBE gene may be 99% similar to the naturally occurring gene.

**[S9OMT]** In some examples, the engineered host cell may modify the expression of the enzyme *S*-adenosyl-L-methionine:*(S)*-scoulerine 9-*O*-methyltransferase. *S*-adenosyl-L-methionine:*(S)*-scoulerine 9-*O*-methyltransferase is encoded by the S9OMT gene. In some examples, *S*-adenosyl-L-methionine:*(S)*-scoulerine 9-*O*-methyltransferase catalyzes the reaction of *S*-adenosyl-L-methionine + *(S)*-scoulerine 4 *S*-adenosyl-L-homocysteine + *(S)*-tetrahydrocolumbamine, as referenced in **FIG. 20****.** The engineered host cell may be modified to include constitutive expression of the S9OMT gene in the engineered host cell. Additionally or alternatively, the engineered host cell may be modified to synthetically regulate the expression of the S9OMT gene in the engineered host cell. In examples, the engineered host cell may be modified to incorporate a copy, copies, or additional copies, of the S9OMT gene. Additionally or alternatively, the engineered host cell may be modified to incorporate the introduction of a strong promoter element for the overexpression of the S9OMT gene within the engineered host cell. In some cases, the S9OMT gene may be codon optimized for expression in *Saccharomyces cerevisiae.* The S9OMT gene may be derived from *Thalictrum flavum subsp. glaucum, Coptis japonica, Coptis chinensis, Papaver somniferum, Thalictrum spp., Coptis spp., Papaver spp.,* or another species. In some examples, the S9OMT gene may be 100% similar to the naturally occurring gene. In examples, the S9OMT gene may be 80% similar to the naturally occurring gene.

**[CAS]** In some examples, the engineered host cell may modify the expression of the enzyme *(S)*-canadine synthase. *(S)*-canadine synthase is encoded by the CAS gene. In some examples, *(S)*-canadine synthase catalyzes the reaction of *(S)*-tetrahydrocolumbamine → *(S)*-canadine, as referenced in **FIG. 20****.** The engineered host cell may be modified to express the CAS gene in the engineered host cell. The engineered host cell may be modified to include constitutive expression of the CAS gene in the engineered host cell. Additionally or alternatively, the engineered host cell may be modified to synthetically regulate the expression of the CAS gene in the engineered host cell. In examples, the engineered host cell may be modified to incorporate a copy, copies, or additional copies, of the CAS gene. Additionally or alternatively, the engineered host cell may be modified to incorporate the introduction of a strong promoter element for the overexpression of the CAS gene within the engineered host cell. The CAS gene may be derived from *Thalictrum flavum subsp. glaucum, Coptis japonica, Thalictrum spp., Coptis spp.,* or another species. In some examples, the CAS gene may be 100% similar to the naturally occurring gene.

**[TNMT)** In some examples, the engineered host cell may modify the expression of the enzyme tetrahydroprotoberberine-*N*-methyltransferase. Tetrahydroprotoberberine-*N*-methyltransferase is encoded by the TNMT gene. In some examples, tetrahydroprotoberberine-*N*-methyltransferase catalyzes the reaction of canadine → *N*-methylcanadine, as referenced in **FIG. 21. FIG. 21** illustrates a biosynthetic scheme for conversion of L-tyrosine to noscapine, noscapinoid, and phthalideisoquinoline, in accordance with embodiments of the invention. In particular, **FIG. 21** provides the use of the enzymes BBE, berberine bridge enzyme; S9OMT, scoulerine 9-*O*-methyltransferase; CAS, canadine synthase; CPR, cytochrome P450 reductase; TNMT, tetrahydroprotoberberine cis-*N*-methyltransferase; CYP82Y1, *N*-methylcanadine 1-hydroxylase; CYP82X2, 1-hydroxy-*N*-methylcanadine 13-hydroxylase; AT1, 1,13-dihydroxy-*N*-methylcandine 13-*O*-acetyltransferase; CYP82X1, 4'-*O*-desmethyl-3-*O*-acetylpapaveroxine synthase; CXE1, narcotine hemiacetal synthase; NOS (or SDR1), noscapine synthase; MT2, narcotoline-4'-*O*-methyltrasnferase 1; MT3, narcotoline-4'-*O*-methyltransferase 2; and 6OMT, 6-*O*-methyltransferase.

In other examples, tetrahydroprotoberberine-*N*-methyltransferase catalyzes the reaction of stylopine → cis-*N*-methylstylopine, as referenced in **FIG. 22. FIG. 22** illustrates a biosynthetic scheme for conversion of L-tyrosine to sanguinarine and benzophenanthridine alkaloids, in accordance with embodiments of the invention. In particular, **FIG. 22** provides the use of the enzymes BBE, berberine bridge enzyme; CFS, cheilanthifoline synthase; STS, stylopine synthase; TNMT, tetrahydroberberine *N*-methyltransferase; MSH, cis-*N*-methylstylopine 14-hydroxylase; P6H, protopine 6-hydroxylase; and DBOX, dihydrobenzophenanthride oxidase. The engineered host cell may be modified to include constitutive expression of the TNMT gene in the engineered host cell. Additionally or alternatively, the engineered host cell may be modified to synthetically regulate the expression of the TNMT gene in the engineered host cell. In examples, the engineered host cell may be modified to incorporate a copy, copies, or additional copies, of the TNMT gene. Additionally or alternatively, the engineered host cell may be modified to incorporate the introduction of a strong promoter element for the overexpression of the TNMT gene within the engineered host cell. In some cases, the TNMT gene may be codon optimized for expression in *Saccharomyces cerevisiae.* The TNMT gene may be derived from *Papaver somniferum, Eschscholzia californica, Papaver bracteatum, Argemone mexicana,* or another species. In some examples, the TNMT gene may be 100% similar to the naturally occurring gene. In examples, the TNMT gene may be 81% similar to the naturally occurring gene.

**[CYP82Y1]** In some examples, the engineered host cell may modify the expression of the enzyme *N*-methylcanadine 1-hydroxylase. *N*-methylcanadine 1-hydroxylase is encoded by the CYP82Y1 gene. In some examples, *N*-methylcanadine 1-hydroxylase catalyzes the reaction of *N*-methylcanadine → 1-hydroxy-*N*-methylcanadine, as referenced in **FIG. 21****.** The engineered host cell may be modified to include constitutive expression of the CYP82Y1 gene in the engineered host cell. Additionally or alternatively, the engineered host cell may be modified to synthetically regulate the expression of the CYP82Y1 gene in the engineered host cell. In examples, the engineered host cell may be modified to incorporate a copy, copies, or additional copies, of the CYP82Y1 gene. Additionally or alternatively, the engineered host cell may be modified to incorporate the introduction of a strong promoter element for the overexpression of the CYP82Y1 gene within the engineered host cell. In some cases, the CYP82Y1 gene may be codon optimized for expression in *Saccharomyces cerevisiae.* In some examples the CYP82Y1 may be modified at the N-terminus. The CYP82Y1 gene may be derived from *Papaver somniferum, Papaver spp., Plantago arenaria, Rauwolfia heterophylla, Adlumia fungosa, Hydrastis canadensis, Stylomecon heterophylla, Hypecoum,* or another species. In some examples, the CYP82Y1 gene may be 70-78% similar to the naturally occurring gene.

**[CYP82X2]** In some examples, the engineered host cell may modify the expression of the enzyme 1-hydroxy-*N*-methylcanadine 13-hydroxylase. 1-hydroxy-*N*-methylcanadine 13-hydroxylase is encoded by the CYP82X2 gene. In some examples, 1-hydroxy-*N*-methylcanadine 13-hydroxylase catalyzes the reaction of 1-hydroxy-*N*-methylcanadine → 1-hydroxy-*N*-methylophiocarpine (i.e. 1,13-dihydroxy-*N*-methylcanadine), as referenced in **FIG. 21****.** The engineered host cell may be modified to include constitutive expression of the CYP82X2 gene in the engineered host cell. Additionally or alternatively, the engineered host cell may be modified to synthetically regulate the expression of the CYP82X2 gene in the engineered host cell. In examples, the engineered host cell may be modified to incorporate a copy, copies, or additional copies, of the CYP82X2 gene. Additionally or alternatively, the engineered host cell may be modified to incorporate the introduction of a strong promoter element for the overexpression of the CYP82X2 gene within the engineered host cell. In some cases, the CYP82X2 gene may be codon optimized for expression in *Saccharomyces cerevisiae.* In some examples the CYP82X2 may be modified at the N-terminus. The CYP82X2 gene may be derived from *P*. *somniferum, Papaver spp, Plantago arenaria, Rauwolfia heterophylla, Adlumia fungosa, Hydrastis Canadensis, Stylomecon heterophylla, Dactylicapnos torulosa, Glaucium flavum, Berberis laurina, B. Vulgaris, Corydalis spp, Fumaria spp, Dactylicapnos spp.,* or another species. In some examples, the CYP82X2 gene may be 70-77% similar to the naturally occurring gene. In other examples, the CYP82X2 gene may undergo N-terminus engineering. In examples, N-terminus engineering may include N-terminal truncation.

**[CYP82X1]** In some examples, the engineered host cell may modify the expression of the enzyme 4'-*O*-desmethyl-3-*O*-acetylpapaveroxine synthase. 4'-*O*-desmethyl-3-*O*-acetylpapaveroxine synthase is encoded by the CYP82X1 gene. In some examples, 4'-*O*-desmethyl-3-*O*-acetylpapaveroxine synthase catalyzes the reaction of 1-hydroxy-13-*O*-acetyl-*N*-methylcanadine → 4'-*O*-desmethyl-3-*O*-acetylpapaveroxine, as referenced in **FIG. 21****.** Additionally, CYP82X1 catalyzes the reaction of 1-hydroxy-*N*-methylcanadine → 4'-*O*-desmethylmacrantaldehyde. The engineered host cell may be modified to include constitutive expression of the CYP82X1 gene in the engineered host cell. Additionally or alternatively, the engineered host cell may be modified to synthetically regulate the expression of the CYP82X1 gene in the engineered host cell. In examples, the engineered host cell may be modified to incorporate a copy, copies, or additional copies, of the CYP82X1 gene. Additionally or alternatively, the engineered host cell may be modified to incorporate the introduction of a strong promoter element for the overexpression of the CYP82X1 gene within the engineered host cell. In some cases, the CYP82X1 gene may be codon optimized for expression in *Saccharomyces cerevisiae.* In some examples the CYP82X1 may be modified at the N-terminus. The CYP82X1 gene may be derived from *Papaver somniferum, Papaver spp., Plantago arenaria, Rauwolfia heterophylla, Adlumia fungosa, Hydrastis canadensis, Stylomecon heterophylla, Hypecoum,* or another species. In some examples, the CYP82X1 gene may be 71-77% similar to the naturally occurring gene. In other examples, the CYP82X1 gene may undergo N-terminus engineering. In examples, N-terminus engineering may include N-terminal truncation.

**[MT2 and MT3]** In some examples, the engineered host cell may modify the expression of the enzyme narcotoline 4'-*O*-methylase. Narcotoline 4'-*O*-methylase is a heterodimer formed by the *O*-methyltransferase monomer encoded by the MT2 and MT3 genes. In some examples, narcotoline 4'-*O*-methylase catalyzes the reaction of narcotoline → noscapine, as referenced in **FIG. 2****.** Additionally, narcotoline 4'-*O*-methylase catalyzes the reaction of narcotolinenehemiacetal → narcotinehemiacetal and 4'-*O*-desmethyl-3-*O*-acetylpapaveroxine → 3-*O*-acetylpapaveroxine. The engineered host cell may be modified to include constitutive expression of the MT2 and MT3 genes in the engineered host cell. Additionally or alternatively, the engineered host cell may be modified to synthetically regulate the expression of the MT2 and MT3 genes in the engineered host cell. In examples, the engineered host cell may be modified to incorporate a copy, copies, or additional copies, of the MT2 and MT3 genes. Additionally or alternatively, the engineered host cell may be modified to incorporate the introduction of a strong promoter element for the overexpression of the MT2 and MT3 genes within the engineered host cell. In some cases, the MT2 and MT3 genes may be codon optimized for expression in *Saccharomyces cerevisiae.* The MT2 and MT3 genes may be derived from *P*. *somniferum, Papaver spp, Fumaria parviflora, Plantago arenaria, Rauwolfia heterophylla,* or another species. In some examples, the MT2 and MT3 genes may be 80% and 79% similar, respectively, to the naturally occurring genes. In some examples, Ps6OMT may be substituted for MT3.

**[AT1]** In some examples, the engineered host cell may modify the expression of the enzyme 1, 13-dihydroxy-*N*-methylcanadine 13-*O* acetyl transferase. 1, 13-dihydroxy-N-methylcanadine 13-*O* acetyltransferase is encoded by the AT1 gene. In some examples, 1, 13-dihydroxy-N-methylcanadine 13-*O* acetyltransferase catalyzes the reaction of 1, 13-dihydroxy-N-methylcanadine → 1-hydroxy-13-*O*-acetyl-*N*-methylcanadine, as referenced in **FIG. 2. FIG. 2** illustrates a biosynthetic scheme for conversion of canadine to noscapine, in accordance with embodiments of the invention. The engineered host cell may be modified to include constitutive expression of the AT1 gene in the engineered host cell. Additionally or alternatively, the engineered host cell may be modified to synthetically regulate the expression of the AT1 gene in the engineered host cell. In examples, the engineered host cell may be modified to incorporate a copy, copies, or additional copies, of the AT1 gene. Additionally or alternatively, the engineered host cell may be modified to incorporate the introduction of a strong promoter element for the overexpression of the AT1 gene within the engineered host cell. In some cases, the AT1 gene may be codon optimized for expression in *Saccharomyces cerevisiae.* The AT1 gene may be derived from *P*. *somniferum, Papaver spp, Plantago arenaria, Rauwolfia heterophylla, Adlumia fungosa, Hydrastis Canadensis, Stylomecon heterophylla, Hypecoum leptocarpum, Dactylicapnos torulosa, Glaucium flavum, Berberis laurina, B. Vulgaris, Corydalis spp, Fumaria spp, Dactylicapnos spp,* or another species. In some examples, the AT1 gene may be 81% similar to the naturally occurring gene.

**[CXE1 or CXE2]** In some examples, the engineered host cell may modify the expression of the enzyme narcotinehemiacetal synthase. Narcotinehemiacetal synthase is encoded by the CXE1 gene. The enzyme encoded by the CXE2 gene can also function as a narcotinehemiacetal synthase. In some examples, narcotinehemiacetal synthase catalyzes the reaction of 4'-*O*-desmethyl-3-*O*-acetylpapaveroxine → narcotolinehemiacetal and 3-*O*-acetylpapaveroxine → narcotinehemiacetal, as referenced in **FIG. 2****.** The engineered host cell may be modified to include constitutive expression of the CXE1 or CXE2 gene in the engineered host cell. Additionally or alternatively, the engineered host cell may be modified to synthetically regulate the expression of the CXE1 or CXE2 gene in the engineered host cell. In examples, the engineered host cell may be modified to incorporate a copy, copies, or additional copies, of the CXE1 or CXE2 gene. Additionally or alternatively, the engineered host cell may be modified to incorporate the introduction of a strong promoter element for the overexpression of the CXE1 or CXE2 gene within the engineered host cell. In some cases, the CXE1 or CXE2 gene may be codon optimized for expression in *Saccharomyces cerevisiae.* The CXE1 or CXE2 gene may be derived from *P*. *somniferum, Papaver spp, Plantago arenaria, Rauwolfia heterophylla, Adlumia fungosa, Hydrastis Canadensis, Stylomecon heterophylla, Hypecoum leptocarpum, Dactylicapnos torulosa, Glaucium flavum, Berberis laurina, B. Vulgaris, Corydalis spp, Fumaria spp, Dactylicapnos spp,* or another species. In some examples, the CXE1 gene or CXE2 gene may be 78% similar to the naturally occurring gene.

**[SDR1]** In some examples, the engineered host cell may modify the expression of the enzyme noscapine synthase. Noscapine synthase is encoded by the SDR1 gene. In some examples, noscapine synthase catalyzes the reaction of narcotolinehemiacetal 4 narcotoline, as referenced in **FIG. 2****.** Additionally, noscapine synthase catalyzes the reaction of narcotinehemiacetal → noscapine. The engineered host cell may be modified to include constitutive expression of the SDR1 gene in the engineered host cell. Additionally or alternatively, the engineered host cell may be modified to synthetically regulate the expression of the SDR1 gene in the engineered host cell. In examples, the engineered host cell may be modified to incorporate a copy, copies, or additional copies, of the SDR1 gene. Additionally or alternatively, the engineered host cell may be modified to incorporate the introduction of a strong promoter element for the overexpression of the SDR1 gene within the engineered host cell. In some cases, the SDR1 gene may be codon optimized for expression in *Saccharomyces cerevisiae.* The SDR1 gene may be derived from *P*. *somniferum, Papaver spp, Plantago arenaria, Rauwolfia heterophylla, Adlumia fungosa, Hydrastis Canadensis, Stylomecon heterophylla, Hypecoum leptocarpum, Dactylicapnos torulosa, Glaucium flavum, Berberis laurina, B. Vulgaris, Corydalis spp, Fumaria spp, Dactylicapnos spp,* or another species. In some examples, the SDR1 gene may be 79% similar to the naturally occurring gene.

Examples of the aforementioned genes can be expressed from a number of different platforms in the host cell, including plasmid (2µ, ARS/CEN), YAC, or genome. In addition, examples of the aforementioned gene sequences can either be native or codon optimized for expression in the desired heterologous host (e.g., *Saccharomyces cerevisiae*)*.*

In examples, an engineered non-plant host cell that produces a noscapinoid or precursor thereof from canadine. In some examples, the noscapinoid or precursor thereof is selected from the group consisting of 1-hydroxy-N-methylcanadine, N-methylcanadine, noscapine, narcotoline, narcotinehemiacetal, 4'-O-desmethyl-3-O-acetylpapaveroxine, narcotolinehemiacetal, N-methylophiocarpine, 1,13-dihydroxyl-N-methylcanadine, 1-hydroxy-13-O-acetyl-N-methylcanadine, narcotolinogendial, narcotolinal and 1-hydroxycanadine. In additional examples, the cell may produce noscapine. In some examples, the cell may produce the noscapinoid or precursor thereof from canadine via a synthetic pathway that comprises one or more noscapine precursors selected from the group consisting of (S)-N-methylcanadine, 1-hydroxy-N-methylcanadine, 1,13-dihydroxy-N-methylcanadine, 1-hydroxy-13-O-acetyl-N-methylcanadine, 4'-O-desmethyl-3-O-acetylpapaveroxine, narcotolinehemiacetal, and narcotoline.

In some additional examples, the cell may comprise one or more heterologous coding sequences that encode one or more enzymes. In some embodiments, the one or more heterologous coding sequences may encode a tetrahydroprotoberberine N-methyltransferase (TNMT). In other examples, the one or more heterologous coding sequences encode a N-methylcanadine 1-hydroxylase (CYP82Y1). In further examples, the one or more heterologous coding sequences encode a 1-hydroxy-N-methylcanadine 13-hydroxylase (CYP82X2). Additionally, the one or more heterologous coding sequences may encode a 1, 13-dihydroxyl-N-methylcanadine 13-0 acetyl transferase (PsAT1). In some examples, the one or more heterologous coding sequences encode a 4'-O-desmethyl-3-acetylpapaveroxine synthase (CYP82X1). Additionally, in some examples, the one or more heterologous coding sequences may encode a narcotinehemiacetal synthase (PsCXE1).

Additionally, the one or more heterologous coding sequences may encode a noscapine synthase (PsSDR1). In other examples, the one or more heterologous coding sequences may encode one or more enzymes selected from PsMT3 and Ps6OMT. Further, the one or more heterologous coding sequences may encode the enzymes CYP82Y1, CYP82X1, and CYP82X2. In some embodiments, the cell may comprise heterologous coding sequences for a CPR enzyme. In some examples, the CPR enzyme is ATR1. In other examples, the one or more heterologous coding sequences are expressed from a low-copy construct. In further examples, the cell may lack one or more enzymes selected from PsMT3, Ps6OMT, and CYP82Y1.

In some cases, the cell may produce a noscapinoid and may comprise one or more heterologous coding sequences for one or more enzymes selected from a P450, a halogenase, a glycosylase, a methyltransferase, an acetyltransferase, a short-chain dehydrogenase, a carboxylesterase, and a prenyltransferase. In some examples, the one or more heterologous coding sequences may be derived from a source organism selected from *P. somniferum* and *E. californica.* The cell is a a yeast cell. In further examples, the yeast cell is an *S*. *cerevisiae* cell. Additionally, in some cases, the cell comprises one or more enzymes derived from two or more different source organisms as compared to the cell. In further cases, the cell comprises multiple heterologous coding sequences that each encode an enzyme and are each derived from a different source organism as compared to the cell. Additionally, the cell may comprise two or more heterologous coding sequences that each encode a TNMT enzyme. Further, the two or more heterologous coding sequences that each encode a TNMT may be derived from different source organisms. In some embodiments, the source organisms may be selected selected from *P*. *somniferum* and *E. californica.*

In some additional examples, the one or more heterologous coding sequences may encode one or more mutant enzymes. Additionally, one or more mutant enzymes may be a CYP82Y1 N-terminus mutant. In further examples, the cell may comprise one or more promoters for the one or more heterologous coding sequences. Additionally, in some cases the one or more promoters comprises a strong promoter. In examplese, the promoter may be selected from the group consisting of HXT7, ADH1, PGK1, TPI1, PYK1, TEF1, GAL1, CYC1, GPD. Additionally, in some examples, the one or more heterologous coding sequences encode CYP82Y1 or a CYP82Y1 mutant and the one or more promoters comprise HXT7. In some cases, the cell may produce 1-hydroxy-N-methylcanadine or 1-hydroxycanadine. Further, in some examples, the one or more heterologous coding sequences encode CYP82X2 or a CYP82X2 mutant and the one or more promoters comprise HXT7.

In some additional cases, the cell may produce 1, 13-dihydroxy-N-methylcanadine. Additionally, the one or more heterologous coding sequences may encode CYP82X2 or a CYP82X2 mutant and the one or more promoters comprise PGK1 and GPD. Further, the cell may produce N-methyl-ophiocarpine. The one or more heterologous coding sequences may encode CYP82X1 or a CYP82X1 mutant and the one or more promoters comprise HXT7. Additionally, the cell may produce 4'-O-desmethyl-2-O-acetylpapaveroxine, or the cell may produce narcotolinal and narcotolinogendial. In further examples, the cell may comprise one or more plant chaperones selected from binding immunoglobulin protein (BiP), DnaJ protein, glucose regulated protein (GRP) 94, binding protein (BiP), protein disulphide isomerase (PDI), cyclophilin, and calnexin.

In examples, the cell comprises multiple copies of the one or more heterologous coding sequences. In some examples, the one or more heterologous coding sequences encode one or more enzymes selected from CYP82Y1, CYP82X2, CYP82X1, PsCXE1, PsSDR1, and PsMT3. Additionally, in some examples, the multiple copies of the one or more heterologous coding sequences are derived from two or more different source organisms as compared to the cell. In further examples, one or more of the enzymes is spatially localized to a compartment in the yeast cell, wherein the compartment is selected from mitochondrion, endoplasmic reticulum (ER), golgi, vacuole, nucleus, plasma membrane, peroxisome, and periplasm. Additionally, in examples, the one or more enzymes is spatially localized to the outside of the compartment in the yeast cell. Further, in examples, the one or more enzymes is spatially localized to the inside of the compartment in the yeast cell.

In further examples, the cell may produce the noscapine alkaloid from norlaudanosoline. Additionally, the cell may comprise one or more heterologous coding sequences that encode one or more enzymes selected from TNMT, PsAT1, CYP82X1, PsCXE1, PsSDR1, PsMT3, CYP82Y1, CYP82X1, S9OMT, Ps6OMT, Ps4'OMT, PsCNMT, PsBBE, CPR1, and CYP719A. Further, the cell may express TNMT, PsAT1, CYP82X1, PsCXE1, PsSDR1, PsMT2 from a YAC. Additionally, the cell may express CYP82Y1 and CYP82X1 from a low-copy plasmid. In other examples, the cell may express S9OMT from a high-copy plasmid. In some cases, Ps6OMT, Ps4'OMT, PsCNMT, PsBBE, CPR1, and CYP719A are chromosomally integrated in the cell.

In some examples, a method of preparing a noscapinoid or a precursor thereof, comprising culturing a host cell according to any one of Claims 1 to 56 under conditions suitable for protein production to produce the noscapinoid or precursor thereof, is provided. In some cases, the method may further comprise recovering the noscapinoid or precursor thereof from the cell culture. In other examples, the method may futher comprise culturing the cell under conditions sufficient to produce a BIA from the noscapinoid; and recovering the BIA from the cell culture. In additional examples, the method may further comprise adding a starting compound to the cell culture. In some cases, the starting compound is canadine. In some cases, the starting compound is norlaudanosoline. Additionally, in some cases, the starting compound is tyrosine or a tyrosine analog. In further, examples, the method may further comprise adding a growth feedstock to the cell culture.

## Claims

1. A method for forming a product stream having a benzylisoquinoline alkaloid product that is downstream of canadine, the method comprising:
culturing an engineered yeast cell that produces a benzylisoquinoline alkaloid product that is a derivative of canadine along a metabolic pathway that converts canadine to a noscapinoid product, wherein the benzylisoquinoline alkaloid product is selected from the group consisting of noscapine and narcotinehemiacetal, and wherein the engineered yeast cell comprises at least one heterologous sequence encoding a heterodimeric enzyme of *Papaver somniferum O*-methyltransferase MT2 and *Papaver somniferum O-*methyltransferase MT3, or a heterodimeric enzyme of *Papaver somniferum O-*methyltransferase MT2 and *Papaver somniferum* norcoclaurine 6-O-methyltransferase 6OMT; and separating the benzylisoquinoline alkaloid product from cellular material to provide a product stream having the benzylisoquinoline alkaloid product, and wherein the cell increases production of noscapine or narcotinehemiacetal relative to a control cell.

2. The method of claim 1, wherein the engineered yeast cell comprises one or more heterologous coding sequences that encode one or more enzymes selected from the group consisting of 6-pyruvoyl tetrahydrobiopterin synthase, sepiapterin reductase, 4a-hydroxytetrahydrobiopterin (pterin-4a-carbinolamine) dehydratase, quinonoid dihydropteridine reductase, dihydrofolate reductase, tyrosine hydroxylase, norcoclaurine synthase, L-DOPA decarboxylase, cytochrome P450 80B1, cytochrome P450 reductase, norcoclaurine 6-O-methyltransferase, 4'-O-methyltransferase, coclaurine-N-methyltransferase, 3-deoxy-d-arabinose-heptulosonate-7-phosphate synthase, chorismate mutase, phenylpyruvate decarboxylase, transketolase, berberine bridge enzyme, (*S*)-scoulerine 9-O-methyltransferase, and (*S*)-canadine synthase.

3. The method of claim 1 or claim 2, wherein the product stream does not contain more than 5 ppm of a molecule selected from the group of lignin, flavonoids, phenanthreoids, latex, rubisco, meconic acid, pseudomorphine, narceine, thebaol, and pollen.

4. The method of any one of the preceding claims, wherein the method further comprises treating a solution containing the product to remove impurities by precipitation.

## Patentansprüche

1. Verfahren zur Bildung eines Produktstroms mit einem Benzylisochinolinalkaloid-Produkt, das stromabwärts von Canadin liegt, wobei das Verfahren Folgendes umfasst: Kultivieren einer konstruierten Hefezelle, die ein Benzylisochinolinalkaloidprodukt, bei dem es sich um ein Derivat von Canadin handelt, entlang einem Stoffwechselweg, bei dem Canadin in ein Noscapinoid-Produkt überführt wird, produziert, wobei das Benzylisochinolinalkaloid-Produkt aus der Gruppe bestehend aus Noscapin und Narcotinhemiacetal ausgewählt ist und wobei die konstruierte Hefezelle wenigstens eine heterologe Sequenz umfasst, die ein heterodimeres Enzym von *Papaver somniberum*-O-Methyltransferase MT2 und *Papaver somniberum*-O-Methyltransferase MT3 oder ein heterodimeres Enzym von *Papaver somniferum-*O-Methyltransferase MT2 und *Papaver somniferum-*Norcoclaurin-6-0-Methyltransferase 6OMT umfasst; und Trennen des Benzylisochinolinalkaloid-Produkts von Zellmaterial, so dass ein Produktstrom mit dem Benzylisochinolinalkaloid-Produkt bereitgestellt wird, und wobei in der Zelle die Produktion von Noscapin bzw. Narcotinhemiacetal relativ zu einer Kontrollzelle erhöht ist.

2. Verfahren nach Anspruch 1, wobei die konstruierte Hefezelle eine oder mehrere heterologe Codiersequenzen umfasst, die ein oder mehrere Enzyme codieren, die aus der Gruppe bestehend aus 6-Pyruvoyltetrahydrobiopterin-Synthase, Sepiapterin-Reduktase, 4a-Hydroxytetrahydrobiopterin(Pterin-4a-carbinolamin)-Dehydratase, Chinonoiddihydropteridin-Reduktase, Dihydrofolat-Reduktase, Tyrosin-Hydroxylase, Norcoclaurin-Synthase, L-DOPA-Decarboxylase, Cytochrom P450 80B1, Cytochrom-P450-Reduktase, Norcoclaurin-6-0-Methyltransferase, 4'-O-Methyltransferase, Coclaurin-N-Methyltransferase, 3-Desoxy-d-arabinoseheptulosonat-7-phosphat-Synthase, Chorismat-Mutase, Phenylpyruvat-Decarboxylase, Transketolase, Berberin-Brücken-Enzym, (S)-Scoulerin-9-O-Methyltransferase und (S)-Canadin-Synthase ausgewählt sind.

3. Verfahren nach Anspruch 1 oder Anspruch 2, wobei der Produktstrom nicht mehr als 5 ppm eines aus der Gruppe Lignin, Flavonoide, Phenanthreoide, Latex, Rubisco, Meconsäure, Pseudomorphin, Narcein, Thebaol und Pollen ausgewählten Moleküls enthält.

4. Verfahren nach einem der vorhergehenden Ansprüche, wobei das Verfahren ferner Behandeln einer das Produkt enthaltenden Lösung zur Entfernung von Verunreinigungen durch Präzipitation umfasst.

## Revendications

1. Procédé pour la formation d'un flux de produit ayant un produit d'alcaloïde de benzylisoquinoléine qui est en aval de la canadine, le procédé comprenant :
la culture d'une cellule de levure modifiée qui produit un produit d'alcaloïde de benzylisoquinoléine qui est un dérivé de canadine le long d'une voie métabolique qui convertit la canadine en un produit de noscapinoïde, le produit d'alcaloïde de benzylisoquinoléine étant choisi dans le groupe constitué par la noscapine et l'hémiacétal de narcotine, et la cellule de levure modifiée comprenant au moins une séquence hétérologue codant pour une enzyme hétérodimérique de l'*O-*méthyltransférase MT2 de *Papaver somniferum* et de l'*O-*méthyltransférase MT3 de *Papaver somniferum,* ou une enzyme hétérodimérique de l'*O*-méthyltransférase MT2 de *Papaver somniferum* et de la norcoclaurine 6-*O-*méthyltransférase 6OMT de *Papaver somniferum* ; et la séparation du produit d'alcaloïde de benzylisoquinoléine de la matière cellulaire pour fournir un flux de produit ayant le produit d'alcaloïde de benzylisoquinoléine, et la cellule augmentant la production de noscapine ou d'hémiacétal de narcotine par rapport à une cellule témoin.

2. Procédé selon la revendication 1, la cellule de levure modifiée comprenant une ou plusieurs séquences codantes hétérologues qui codent pour une ou plusieurs enzymes choisies dans le groupe constitué par la 6-pyruvoyle tétrahydrobioptérine synthase, sépiaptérine réductase, 4a-hydroxytétrahydrobioptérine (ptérine-4a-carbinolamine) déshydratase, quinonoïde dihydroptéridine réductase, dihydrofolate réductase, tyrosine hydroxylase, norcoclaurine synthase, L-DOPA décarboxylase, cytochrome P450 80B1, cytochrome P450 réductase, norcoclaurine 6-O-méthyltransférase, 4'-0-méthyltransférase, coclaurine-N-méthyltransférase, 3-désoxy-d-arabinose-heptulosonate-7-phosphate synthase, chorismate mutase, phénylpyruvate décarboxylase, transcétolase, enzyme du pont berbérine, (S)-scoulérine 9-0-méthyltransférase et (S)-canadine synthase.

3. Procédé selon la revendication 1 ou la revendication 2, le flux de produit ne contenant pas plus de 5 ppm d'une molécule choisie dans le groupe de la lignine, des flavonoïdes, des phénanthréoïdes, un latex, le rubisco, l'acide méconique, la pseudomorphine, la narcéine, le thébaol et du pollen.

4. Procédé selon l'une quelconque des revendications précédentes, le procédé comprenant en outre le traitement d'une solution contenant le produit pour éliminer des impuretés par précipitation.
